(19) Europäisches Patentamt
European Patent Office
Office européen des brevets

(11) Veröffentlichungsnummer : **0 010 243**
**B1**

(12) # EUROPÄISCHE PATENTSCHRIFT

(45) Veröffentlichungstag der Patentschrift : **30.09.81**

(51) Int. Cl.³ : **B 09 B 3/00**, C 02 F 11/00, C 08 K 11/00, B 01 J 31/02

(21) Anmeldenummer : **79103832.6**

(22) Anmeldetag : **08.10.79**

(54) **Verfahren zur Aufarbeitung von Biomassen; modifizierte Biomassen und deren Verwendung.**

(30) Priorität : **13.10.78 DE 2844641**

(43) Veröffentlichungstag der Anmeldung : **30.04.80 (Patentblatt 80/09)**

(45) Bekanntmachung des Hinweises auf die Patenterteilung : **30.09.81 Patentblatt 81/39**

(84) Benannte Vertragsstaaten : **BE CH DE FR GB IT NL**

(56) Entgegenhaltungen :
**DE - A - 2 000 133**
**DE - A - 2 324 134**
**DE - A - 2 523 483**
**FR - A - 2 171 108**
**FR - A - 2 223 078**
**FR - A - 2 265 758**
**FR - A - 2 398 700**
**US - A - 3 073 693**
**US - A - 3 226 318**
**US - A - 3 655 395**
**US - A - 3 929 630**
**US - A - 3 935 069**
**US - A - 4 021 368**
**US - A - 4 067 821**

(73) Patentinhaber : **BAYER AG**
**Zentralbereich Patente, Marken und Lizenzen**
**D-5090 Leverkusen 1, Bayerwerk (DE)**

(72) Erfinder : **Wagner, Kuno, Dr.**
**Am Kiesberg 8**
**D-5090 Leverkusen (DE)**
Erfinder : **Reischl, Artur, Dr.**
**von Böttinger-Strasse 19**
**D-5090 Leverkusen (DE)**
Erfinder : **Findeisen, Kurt, Dr.**
**In der Follmühle 10**
**D-5068 Odenthal (DE)**
Erfinder : **Mann, Theo, Dr.**
**Blumenstrasse 18**
**D-4018 Langenfeld (DE)**
Erfinder : **Mack, Kurt, Dr.**
**Claudiusweg 15**
**D-5600 Wuppertal 1 (DE)**
Erfinder : **Hamburger, Brigitte, Dr.**
**Mülheimer Strasse 117**
**D-5090 Leverkusen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 010 243 B1

Imprimerie Jouve, 17, rue du Louvre, 75001 Paris, France

Verfahren zur Aufarbeitung von Biomassen ; modifizierte Biomassen und deren Verwendung

Die vorliegende Anmeldung betrifft ein neues Verfahren zur Aufarbeitung von Biomassen mit Hilfe von Carbonyl- und/oder Thiocarbonyl-Verbindungen und zur Aminoplastbildung bzw. Phenoplastbildung befähigten Verbindungen. Die Anmeldung betrifft ferner die bei dem erfindungsgemäßen Verfahren anfallenden modifizierten Biomassen selbst und deren Verwendung.

Bei der Durchführung zahlreicher technischer und halbtechnischer Fermentationsprozesse mit Hilfe von Enzymen bzw. mit in Zellteilung befindlichen Biomassen und mikrobiellen Systemen der verschiedensten Art fallen bei der Herstellung von Futtermitteln, Nahrungsmitteln, Arzneimitteln, bei der Isolierung von Enzymen und Antibiotika etc., bei der Herstellung von alkoholischen Getränken, Futter- und Nährhefen der verschiedensten Art weltweit große Mengen an protein-, desoxy- und ribonukleinsäurehaltigen Biomassen an, die bisher nur zu einem Bruchteil einer nutzbringenden Verwendung zugeführt werden konnten. Außerdem führen die zahlreichen Erweiterungen vollbiologisch arbeitender Anlagen oder die Errichtung neuer Anlagen zur Reinigung industrieller und kommunaler Abwässer zu einer rapide anwachsenden Produktionssteigerung an Biomassen, wobei derartige Biomassen in Form kostspieliger Verfahren isoliert, kostenintensiv entwässert und in der Regel trotz ihres hohen Proteingehaltes infolge von möglicherweise vorhandenen Schadstoffen, wie hohen Gehalten an Metallionen, Pflanzenschutzmittel-Rückständen, pathogenen Keimen usw. zum Beispiel durch Verbrennung vernichtet werden müssen.

Die technisch bisher nicht in befriedigendem Maße durchführbare einfache Isolierung und chemische Modifizierung der verschiedensten Biomassen, ihre konfektionierung unter standardisierten Bedingungen bei gleichzeitiger vollständiger Zerstörung pathogener Keime, die lagerbeständige Konservierung aller Zellinhaltsstoffe unter totaler Enzymdesaktivierung, die Desaktivierung von enthaltenen Pflanzenschutzmittel-Rückständen oder Antibiotika-Rückständen und die nutzbringende Verwendung derartiger desaktivierter Biomassen im Sinne wirtschaftlicher Recycling-Prozesse ist von weltweiter Bedeutung. Dies geht unter anderem auch aus den nachstehenden Angaben hervor.

Ohne Berücksichtigung der bei den verschiedensten technischen Fermentationsprozessen anfallenden großen Mengen an Biomassen verarbeiten moderne Einzelanlagen der chemischen Großindustrie bei der vollbiologischen Reinigung industrieller und kommunaler Abwässer bis zu 100 000 $m^3$ Abwasser pro Tag, wobei Biomassen aus Mikroorganismen anfallen, deren Trockengewicht etwa zwischen 1 200 bis 1 500 Monatstonnen pro Einzelanlage liegt. Allein in der Bundesrepublik Deutschland fallen bereits Mengen von etwa 2 Mio jato an derartigen proteinhaltigen Biomassen an. Diese Menge steigt laufend infolge der Errichtung weiterer notwendiger vollbiologischer Reinigungsanlagen an. Die im gesamten europäischen Raum, USA und Canada anfallenden Biomassen stellen daher bereits jetzt riesige Monatstonnagen an Zellbestandteilen, z.B. interessanten Proteinen oder phosphorhaltigen Zellinhaltsstoffen dar. Sie werden grundsätzlich nicht nur nach teuren Verfahren isoliert und entwässert, sondern auch in der Regel wegen der möglichen Anwesenheit von pathogenen Bakterien, Protozonen, Pilzen, Pestiziden, Herbiziden oder wegen zu hoher Metallionen-Gehalte usw. nach der Entwässerung mittels kostspieliger Verfahren verbrannt bzw. in Deponien gelagert.

Die Vernichtung derartiger riesiger Mengen an Proteinen und anderen wertvollen Zellinhaltsstoffen ist gleichbedeutend mit der Vernichtung wertvoller Pflanzennährstoffe und damit indirekt auch wertvoller Tiernährstoffe.

Es hat daher nicht an Versuchen gefehlt, die anfallenden Biomassen aufzuarbeiten und nutzbringend zu verwenden. Die bisher erzielten Ergebnisse sind allerdings nicht voll befriedigend.

So ist bereits ein Verfahren zur Aufarbeitung von Klärschlamm bekannt geworden, welches darin besteht, daß man :

— die bei der biologischen Klärung anfallenden Klärschlämme zunächst filtriert,

— den Filterkuchen auf Teilchengrößen von etwa 3 mm und darunter zerkleinert,

— den Feuchtigkeitsgehalt der Teilchen auf etwa 30 bis 50 % herabsetzt und

— anschließend eine wäßrige Lösung eines separat alkalisch vorkondensierten N-Methylolharnstoffes mit den teilgetrockneten Klärschlammteilchen zur Reaktion bringt, wobei eine Temperatur zwischen etwa 30 °C und 80 °C und ein pH-Wert von 3 bis 5 eingehalten wird, um ein körniges Reaktionsprodukt zu erzeugen, das als Düngemittel oder Futterzusatzmittel für Tiere verwendbar ist (vgl. DT-OS 25 23 483).

Dieses Verfahren ist jedoch mit mehreren Nachteilen behaftet. Zum Beispiel ist das Verfahren nur beim Einsatz von teilgetrockneten Klärschlämmen durchführbar. Lebende mycelartige Biomassen mit einem Gehalt von 75-80 % an gebundenem Zellwasser und einem Gehalt von 100-300 % an extrazellulärem Wasser lassen sich nicht als Ausgangsmaterialien verwenden. Ebensowenig ist das Verfahren geeignet zur Aufarbeitung von beliebigen und lebenden Biomassen, das heißt sich im Zustande maximaler Turgeszenz und zellteilung befindlicher Biomassen, da die Zellwände von vielen Bakterien, Bazillen, Pilzen und den verschiedensten mikrobiellen Systemen, insbesondere von lebenden mikrobiellen Systemen, wie Pseudomonaden usw., aus biologisch oder vollbiologisch arbeitenden Kläranlagen unter den Umsetzungsbedingungen nicht zerstört oder hydrolysierend gesprengt und abgebaut werden. — Nachteilig ist auch, daß zur Herstellung der teilgetrockneten Klärschlämme aufwendige Filtrationen und mechanische Zerkleinerungen sowie mit hohen Kosten verbundene Trocknungen erforderlich sind.

Ferner sind die Verfahrensprodukte noch reich an resistenten sporenbildenden Mikroorganismen, wodurch sich erneut Bakterien oder Pilze bilden. Ein weiterer Nachteil besteht darin, daß nur geruchlose teilgetrocknete Klärschlämme in geruchslose Verfahrensprodukte überführt werden können. Sind in den eingesetzten Klärschlammteilchen übelriechende Substanzen enthalten, so werden diese durch die verfahrensgemäßen Kondensationen in überwiegendem Maße nicht zerstört.

Schließlich sei erwähnt, daß die nach dem in der DT-OS 25 23 483 beschriebenen Verfahren zugänglichen Produkte relativ hohe Mengen an unkomplexierten oder nicht ausreichend fest komplexierten Metallionen enthalten, welche in wasserlöslicher Form vorliegen, daher in relativ hohen Konzentrationen abgegeben werden und eine praktische Verwendung der Verfahrensprodukte beeinträchtigen.

Weiterhin ist bereits bekannt geworden, daß sich Biomassen-Abfälle mit einem Feststoffgehalt von bis zu 60 Gew.-% aufarbeiten lassen, indem man den Biomassen-Schlamm mit Formaldehyd vermischt (um Bakterien abzutöten), dann konzentrierte Salpetersäure zugibt, wobei sich eine homogene Mischung bildet und das verfügbare Stickstoffdioxid oxidierend auf den Abfallschlamm einwirkt, und man anschließend Harnstoff zu der homogenen Mischung zugibt, wonach innerhalb einer gewissen Zeit Polymerisation und dann Agglomerisation eintritt (vgl. US-PS 3 655 395). Dieses Verfahren besitzt jedoch die gleichen Nachteile wie das oben erwähnte vorbekannte Verfahren. Hervorzuheben ist, daß der einsetzbare Abfallschlamm einen möglichst hohen Feststoffgehalt aufweisen muß. Daher ist eine aufwendige Vortrocknung des Schlammes unerläßlich. Im übrigen wird eine relativ große Menge an Salpetersäure als Oxidationsmittel gebraucht.

Außerdem ist bekannt, daß man Kloaken-Schlamm in Stickstoff-Düngemittel überführen kann, indem man den Schlamm zunächst mit Torf und festem Harnstoff vermischt, dann eine wäßrige Harnstoff-Formaldehyd-Lösung sowie eine wäßrige Lösung einer starken Mineralsäure zugibt und anschließend mit wäßriger Ammoniak-Lösung neutralisiert (vgl. US-PS 3 073 693). Auch hierbei ist es erforderlich, den Klärschlamm in feste Form zu bringen, also weitgehend zu entwässern und vorzutrocknen. Da diese Operationen, — wie schon im Zusammenhang mit dem aus der DT-OS 2 523 483 bekannten Verfahren beschrieben —, sehr aufwendig sind, ist das Verfahren für einen praktischen Einsatz unrentabel.

Schließlich ist es bereits bekannt, Abfallschlamm zu verfestigen und für verschiedene Zwecke nutzbar zu machen, indem man den Abfallschlamm mit polymerisierbaren oder kondensierbaren Monomeren, wie Acrylsäure oder niedermolekulare Aldehyde und Benzolderivate, z.B. Phenole, vermischt und die Polymerisation bzw. Kondensation unter üblichen Bedingungen durchführt (vgl. US-PS 3 226 318). Dieses Verfahren ist ebenfalls mit den Nachteilen behaftet, die schon im Rahmen der Diskussion des in der DT-OS 2 523 483 offenbarten Verfahrens genannt wurden. So wird zum Beispiel ausdrücklich darauf hingewiesen, daß der Wassergehalt des Abfallschlammes möglichst niedrig liegen muß. Daher ist auch bei diesem Verfahren unbedingt eine arbeits- und kostenaufwendige Vortrocknung des Abfallschlammes nötig.

Gegenstand der vorliegenden Erfindung ist ein neues Verfahren zur Aufarbeitung von Biomassen verschiedenster Art durch Umsetzen der Biomassen in wäßrigem Medium mit Carbonylverbindungen, Thiocarbonylverbindungen und/oder Carbonylverbindungen, die mit niedermolekularen, nicht kondensierten N-Alkylolverbindungen im Dissoziationsgleichgewicht stehen, gegebenenfalls in Gegenwart eines Katalysators sowie gegebenenfalls in Gegenwart von Zusatzstoffen, dadurch gekennzeichnet, daß man anschließend die nicht umgesetzten Carbonylverbindungen, Thiocarbonylverbindungen und/oder Carbonylverbindungen, die mit niedermolekularen, nicht kondensierten N-Alkylolverbindungen im Gleichgewicht stehen, in wäßrigem Medium mit Aminoplastbildnern, die gegebenenfalls N-Alkylolgruppen enthalten, bzw. mit Phenoplastbildnern, gegebenenfalls in Gegenwart eines Katalysators und gegebenenfalls in Gegenwart von Kettenabbrechern sowie gegebenenfalls in Gegenwart von Zusatzstoffen kondensiert und

— die so entstehenden modifizierten Biomassen gegebenenfalls anschließend von noch enthaltenen unerwünschten Stoffen befreit und/oder einer Nachbehandlung unterwirft.

Gegenstand der Erfindung sind weiterhin modifizierte Biomassen, die nach dem erfindungsgemäßen Verfahren entstehen. Sie sind gekennzeichnet durch einen Gehalt an

— mit Carbonylverbindungen, Thiocarbonylverbindungen und/oder Carbonylverbindungen, die mit niedermolekularen, nicht kondensierten N-Alkylolverbindungen im Dissoziationsgleichgewicht stehen,

— und mit Aminoplastbildnern, die gegebenenfalls N-Alkylolgruppen enthalten bzw. mit Phenoplastbildnern

kondensierten Biomassen-Zellinhaltsstoffen bzw. anderen in Biomassen vorkommenden reaktionsfähigen Stoffen, sowie gegebenenfalls durch einen Gehalt an Zusatzstoffen.

Gegenstand der Erfindung ist außerdem die Verwendung der erfindungsgemäßen modifizierten Biomassen für verschiedene Zwecke. So eignen sich erfindungsgemäße Stoffe zur Fixierung von Metallionen beziehungsweise organischen oder anorganischen Säuren. Weiterhin können erfindungsgemäße Stoffe als reaktive Füllstoffe und Kombinationsfüllstoffe, Flammschutzmittel und Alterungsschutzmittel in Kunststoffen, wie natürlichen oder synthetischen Kautschuken, Polyamiden und Epoxidharzen verwendet werden. Darüber hinaus können erfindungsgemäße Stoffe als Katalysatoren oder als Trägerstoffe für Katalysatoren oder andere Stoffe fungieren. Zum Beispiel eignen sich bestimmte erfindungsgemäße Stoffe als Katalysatoren zur Formose-Zuckergemisch-Synthese auf der Grundlage

hochprozentiger Formalinlösungen. Schließlich können erfindungsgemäße Stoffe zur Desaktivierung von Pflanzenschutzmitteln, als Nährmedien für Bakterien sowie als Agrarchemikalien oder als Futterzusatzmittel eingesetzt werden.

Die glatte und reproduzierbare Durchführbarkeit des erfindungsgemäßen Verfahrens ist als überraschend zu bezeichnen, denn wie umfangreiche Versuche zeigten, ist eine allgemeine anwendbare rationelle Aufarbeitung von Biomassen unterschiedlichster Herkunft bisher weder

— durch Behandlung von Biomassen mit verschiedenartigsten hochreaktiven Reagenzien, die üblicherweise zur Fällung und Denaturierung von Proteinen verwendet werden,

— noch durch thermische Denaturierung und Ausflockung der Biomassen,

— oder durch Kondensation von Biomassen mit Carbonyl- oder Thiocarbonyl-Verbindungen,

— bzw. durch andere Flockungs- und Modifizierungsreaktionen

in befriedigender Weise gelungen.

So führen viele an Eiweißstoffen durchführbare Denaturierungen, Ausflockungen und Aussalzungen mit Reagenzien, wie gut hydratisierendem Ammoniumsulfat, Trichloressigsäure, Kupfersulfat, Zinkhydroxid, Äthanol oder Aceton, oder thermische Denaturierungen und Ausflockungen bei vielen Biomassen, z.B. Pseudomonaden oder Hefen etc., nicht zu pulvrigen, leicht filtrierbaren Produkten. Zudem gelangen die zugesetzten Stoffe in relativ hoher Konzentration in das Abwasser und schaffen dadurch neue Abwasserprobleme. Ebenso werden bei Kondensationen von Biomassen mit carbonylverbindungen, wie Formaldehyd, Glyoxal, Glyoxalsulfat, Trichloracetaldehyd, Furfurol oder Isobutyraldehyd oder Thiocarbonylverbindungen, wie Thioformaldehyd oder polymerem Thioacetaldehyd, die zwar starke Enzymdesaktivierungen und Sterilisationen von Biomassen bewirken, bei vielen Biomassen, zum Beispiel Pseudomonaden, die als Mikroorganismen meist in vollbiologisch arbeitenden Kläranlagen vorhanden sind, lediglich schwer filtrierbare, verklebende, schleimige Massen erhalten. Ferner sind Modifizierungsreaktionen und Flockungen von Biomassen mit Acylierungsmitteln, Alkylierungsmitteln, Schwefelchloriden, Phosphorchloriden, Phosgen und Sulfonsäuregruppen enthaltenden Fällungsmitteln nicht generell anwendhar. Es war daher nicht zu erwarten, daß sich Biomassen verschiedenster Art mit Hilfe der erfindungsgemäßen Umsetzungen in einfacher Weise aufarbeiten lassen könnten.

Das erfindungsgemäße Verfahren weist eine Reihe von Vorteilen auf. So ermöglicht es eine einfache und auch im technischen Maßstab durchführbare Aufarbeitung von Biomassen. Zudem ist das Verfahren nicht auf spezielle Biomassen besckränkt, sondern generell anwendbar. Eine Vorentwässerung der einzusetzenden Biomassen ist nicht erforderlich.

Die anfallenden Produkte verkleben in wäßriger Phase nicht, sind ohne Komplikationen filtrierbar und lassen sich in energiesparender Weise trocknen. Sie sind völlig lagerbeständig und frei von pathogenen Krankheitserregern. Bedingt durch totale Enzymdesaktivierung und vollständigen Zelltod der Biomassen in den erfindungsgemäßen Verahrensprodukten werden Zersetzungs-, Fäulnisvorgänge, Gärungen und unangenehme Geruchsbildungen von enzymatisch oder mikrobiologisch abbaubaren Zellinhaltsstoffen vollständig unterbunden. Die Verfahrensprodukte sind daher bei beliebig langen Lagerzeiten in trockenem Zustand und in abgefüllten Gebinden bezüglich unangenehmer Geruchsbildung und weiterem enzymatischem Abbau vollständig stabilisiert. Außerdem sind die Verfahrensprodukte für eine Vielzahl von Zwecken nutzbringend verwendbar. Im übrigen wird durch die vielseitige Anwendbarkeit des erfindungsgemäßen Verfahrens auf beliebige Biomassen ein wesentlicher Beitrag zur Lösung wichtiger Umweltschutzprobleme geleistet. Während bisher an sich hochwertige, proteinhaltige Stoffe, Nucleinsäuren und wertvolle Zellinhaltsstoffe enthaltende Produkte, wegen Beimengungen von Schadstoffen, Lagerinstabilität bzw. wegen Anwesenheit von zu hohen Metallgehalten, pathogenen Krankheitserregern etc. in der Regel zunächst nach kostspieligen Verfahren isoliert und getrocknet und dann verbrannt oder in Deponien für spätere Verbrennungen abgelagert werden mußten, ist es mit Hilfe des erfindungsgemäßen Verfahrens möglich, derartige Stoffe einer sinnvollen Verwendung zuzuführen. Das erfindungsgemäße Verfahren und die erfindungsgemäßen Produkte stellen daher eine wertvolle Bereicherung der Technik dar.

Wie bereits erwähnt, ist das erfindungsgemäße Verfahren zur Aufarbeitung beliebiger Biomassen geeignet. Dies ist möglich, obwohl die meisten Zellbestandteile beliebiger Zellarten, z.B. das Cytoplasma X, die Desoxyribonukleinsäure Y und Ribonukleinsäure Z (X + Y + Z) eines pflanzlichen oder tierischen Einzellers mit dem Cytoplasma $X_m$, der Desoxyribonukleinsäure $Y_m$ und Ribonukleinsäure $Z_m$ (= $X_m + Y_m + Z_m$) eines differenzierten Mehrzellers starke charakteristische Variationen im molekularen Aufbau von (X + Y + Z) gegenüber ($X_m + Y_m + Z_m$) aufweisen.

Unter Biomassen sind im vorliegenden Fall alle sich im Teilungszustand, Ruhezustand, partiellen oder vollständigen Zelltod, oder bereits in der enzymatischen Zersetzung oder in der Zersetzung durch Fremdkulturen befindlichen Biosysteme aus Mikroorganismen, wie Prokaryonten, Eukaryonten, Bakterien, Pilzen, Hefen, Algen, Protozoen und Einzellern usw., sowie auch Biosysteme höherdifferenzierter Zellen pflanzlicher oder tierischer Herkunft, zum Beispiel Zellbestandteile des Blutes, wie Erythrozyten, Granulozyten, Lymphozyten und Thrombozyten zu verstehen.

Als bevorzugt verwendbare Biomassen kommen bei der Durchführung des erfindungsgemäßen Verfahrens die folgenden Produkte in Betracht:

— Biomassen, die

a) bei Verfahren zur Gewinnung von Produkten des primären Stoffwechsels anfallen, also zum

Beispiel bei der biotechnischen Herstellung von Äthanol, Butanol, Aceton, Zitronensäure, Milchsäure, Weinsäure, einfachen aliphatischen Carbonsäuren, Aminosäuren usw.,

b) bei technischen Fermentationsprozessen zur Herstellung von Produkten des sekundären Stoffwechsels anfallen, also zum Beispiel bei der Gewinnung von Antibiotika, Vitaminen, Wuchsstoffen, Steroidhormonen, Alkaloiden usw.,

c) bei Verfahren zur Gewinnung von Zellbestandteilen, wie Enzymen, Nucleinsäuren oder Polysacchariden, anfallen und

d) bei Verfahren zur Gewinnung von Zellen als Biomasse, z.B. Hefen zum Backen oder zur alkoholischen Gärung, oder Hefen zur Proteingewinnung aus Methan, Erdöl und Methanol.

— Biomassen aus Mikroorganismen von biologisch arbeitenden Kläranlagen.

— Biomassen, die bei Verfahren der Biotransformation anfallen, also bei Verfahren, bei denen Mikroorganismen als Katalysatoren organisch chemischer Reaktionen, wie Oxidationen, Reduktionen, Decarboxylierungen, Phosphorylierungen, Aminierungen, Desaminierungen, Acetylierungen, Desacetylierungen usw. verwendet werden.

— Biomassen unterschiedlichster pflanzlicher Herkunft.

— Biomassen mikrobieller bzw. bakterieller Natur.

— Biomassen aus tierischen Abfallprodukten.

Besonders bevorzugte Biomassen sind :

— Die verschiedensten Hefearten (Pilzarten) aus technischen Prozessen, zum Beispiel aus Fermentationsprozessen der alkoholischen Gärung oder der Alkohol-Bier-Produktion, sowie untergärige und obergärige Hefen.

— Biomassen der Essig-, Milchsäure-, Zitronensäure-, Weinsäure- oder Weißkraut-Herstellung, ferner geruchlich nicht einwandfreie Biomassen dieser Verfahren und Bakterienkulturen, die infolge enzymatischer Prozesse vergären.

— Fehlpartien von Hefekulturen.

— Biomassen der Proteinherstellung auf der Grundlage verschiedener Kohlenwasserstoff-Quellen, wie Paraffinverschnitten, Methan oder Methanol. Speziell in Frage kommen hierbei Biomassen aus speziellen Hefezellen der Großanlagen zur Erzeugung von Eiweiß aus Petroleumfraktionen, die etwa 60 % Rohprotein enthalten, sich innerhalb einer Stunde teilen und etwa 40 % verdauliches Eiweiß enthalten, und Fehlpartien derartiger Biomassen. — Solche Hefebiomassen enthalten beispielsweise pro 220 g Rohprodukt an interessanten Aminosäuren, etwa 30 g Lysin, 7,5 g Methionin, 9 g Cystin, 12,5 g Tryptophan und etwa 9 g an Vitamin B und Vitamin D. — Speziell verwendbar sind auch Biomassen aus einzelligen Mikroorganismen, die bei der Eiweißgewinnung aus Erdgas (Methan) eingesetzt werden, die aus bakteriellen Mischkulturen bestehen, Eiweißgehalte von etwa 70 % aufweisen und etwa hochwertigem Fischmehl entsprechen. Ferner besonders geeignet sind Biomassen aus Pseudomonas-Bakterien, die im Fermenter bei etwa 37 °C gezüchtet werden und aus Methanol als Kohlenstoffquelle proteinreiche Biomassen erzeugen.

— Biomassen der Penicillin-Herstellung, z.B. Penicillium notatum und Penicillium chryzogenum.

— Biomassen aus der Endstufe der Tetracyclin-Herstellung (Streptomyceten).

— Biomassen aus fadenartigen Bakterien der Sisomicin-Herstellung (Micromonospora), sowie andere Streptomyces-Arten.

— Biomassen von biologisch arbeitenden Reinigungsanlagen von industriellen und kommunalen Abwässern. Derartige Biomassen bestehen zu einem großen Teil aus Pseudomonas-Arten und anderen Bakterien-, Algen- und Pilzarten, die optimal etwa bei einem P : N : C-Verhältnis von 1 : 5 : 100 arbeiten und als Omnivore (= Allesfresser) zu bezeichnen sind. Die aus Kläranlagen stammenden Biomassen, die auch als Belebtschlämme bezeichnet werden, sind bei dem erfindungsgemäßen Verfahren auch dann verwendbar, wenn sie Spuren an Ionen des Quecksilbers, Cadmiums, Zinks, Eisens, Chroms und/oder des Bleis enthalten.

— Algenarten, wie Blaualgen (Nostoc, Spirulina) Grünalgen (z.B. Chlorella, Scenedesmus und Coleastrum), Kieselalgen, Jochalgen (z.B. Spirogyra), Geißelalgen (z.B. Chlamydomonas), Braunalgen (Blasentang) und Rotalgen wie auch Protozoen.

— Blutplasma-Biomassen, extracelluläre und celluläre Bestandteile des Blutes, z.B. Erythrozyten, Granulozyten, Lymphozyten und Thrombozyten.

— Biomassen der Fischindustrie.

— Biomassen aus tierischen Abfällen, wie Leberzellen, Nervenzellen, Knochenmarkzellen etc.

— Biomassen aus dem Reich der Bakterien insbesondere solche der Ordnung Eubacteriales wie z.B. der Familien Pseudomonadaceae (z.B. Pseudomonas, Rhizobium, Acetobacter), der Enterobacteriaceae (z.B. Escherichia coli, Flavobacterium, Proteus, Serratia, Salmonella), der Bacillaceae (z.B. B. subtilis, B. megaterium, Clostridium), der Lactobacteriaceae (z.B. Lactobacillus), der Micrococcaceae (z.B. Leuconostoc), der Bacterioidaceae (z.B. Desulfovibrio), der Methanobacteriaceae (z.B. Methanobacter), aber auch gleitende Bakterien (wie Beggiatoa), Scheiden tragende Bakterien (z.B. Sphaerotilus), phototrope Bakterien (wie z.B. Chromatium) wie auch Bakterien der Ordnung Actinomycetales (wie Streptomyces, z.B. S. rimosus, Micromonospora, Actinoplanes, Streptosporangium), aus dem Reich der Pilze sowohl höhere wie niedere Pilze, Basidiomyceten (z.B. Agaricus), Ascomyceten (z.B. Penicillium, Aspergillus, Saccharomyces), Phycomyceten (z.B. Mucor) wie auch Fungi imperfecti (z.B. Fusarium), und

zahlreiche andere Biomassen mikrobieller Art wie sie in der Literatur beschrieben sind (vgl. Synthesis *4*, 120-134 und 147-157 (1969)). Diese Biomassen können aus Reinkulturen aber selbstverständlich auch aus Mischkulturen, d.h. aus bei Fermentationsprozessen infizierten und daher unbrauchbaren Kulturen bestehen und selbstverständlich auch in Mischung tote Zellen pflanzlicher Art oder Zellinhaltsstoffe wie Hemicellulosen, Zellwandbestandteile pflanzlicher Art wie Cellulose und andere Zuschläge wie homogenisierte Cellulose-abfälle und Lignin-Abfälle enthalten.

— Mischkulturen der verschiedenartigsten Bakterien, Pilze, Algen etc., ebenso infizierte Kulturen von Biomassen, die durch Infektion mit Pilzarten, andersartigen Bakterienarten etc. charakterisiert sind und eine komplexe Zusammensetzung aufweisen. Beispielhaft genannt seien Mischkulturen, die sich an in Zersetzung befindlichen Trebern, Nährmedien wie Gelatine, Melassen, Stärken, Polysacchariden an freier Luft und im feuchten Zustand sowie an proteinhaltigem, noch lebenden oder bereits in der Zersetzung befindlichen Algentang ausbilden. Ferner genannt seien Biomassen, die bei in Fäulnis übergehenden Pflanzenarten und Zellinhaltsstoffen (z.B. Zuckerrohr-Rückstands-Homogenisate, Zuckerrüben-Rückstands-Homogenisate, Melassen, Homogenisate von Breitblattgewächsen oder Grashomogenisate) bei der Lagerung in Wasser oder im feuchten Zustand aerob und anaerob durch Proteinabbau und enzymatischen Abbau entstehen und oft überlriechende Stoffe enthalten.

— Erdige Materialien, wie biologisch aktive Gartenerde, erdige Materialien normalen Bakterien-, Algen- und/oder Pilzgehaltes,

— Biomassen, die sich auf verdorbenen Nährstoffen bilden, ferner auf Ricinusölrückständen (z.B. auf warm gelagertem und feuchtem Ricinusschrot), Hefemassen, Baumwollsaatmehl, Sojamehl, Fischmehl, Tiermehl, Knochenmehl, Algenmehl, Ricinussamenmehl, Stärkemehl, Dextrinen, Peptonen, Lignin, feuchten Cellulosepulvern, feuchtem homogenisierten Nadelholz und homogenisiertem Blattmaterial, feucht gelagerten Pepton-Agar-Kombinationen, Fleisch-Extraktpulvern, unsachgemäß gelagerten Malzkeimen, Tabakrippen, Rückständen von nicht kristallisierenden Zuckern und Zuckerrübenschnitzeln, sowie auf feuchten Ligninsulfonaten aus Sulfitablaugen der Papierindustrie und auf unsachgemäß gelagertem Tang.

— Biomassen, in denen der Zelltod bereits weit fortgeschritten ist, also zum Beispiel solche Biomassen, die viel an Zellinhaltsstoffen (Polysaccharide, pflanzliche Pektinstoffe, gelatineartige Produkte) außerhalb der Zellmembran in wäßriger Lösung enthalten.

— Biomassen enthaltende Produkte der Kartoffelstärke-Industrie, zum Beispiel mit verschiedenen bakteriellen und hefeartigen Mikroorganismen befallene lösliche Proteinanteile in Mutterlaugen der Stärkeaufbereitung. Ebenso können Produkte der Cellulose-Industrie verwendet werden, z.B. neutralisierte und von Bakterien, Pilzen, Algen oder Hefen befallene Sulfitablaugen der Zellstoffindustrie.

— Faul- und Bioschlämme der verschiedensten Art sowie Biomassen mit hohen Anteilen an Escherichia coli und/oder verschiedensten pflanzlichen Schwebstoffen.

— Biomassen anaerober Faulung (= Intensiv-faulung), Müll-Klärschlamm-Kompostierungsprodukte, zum Beispiel aus Verfahren der thermophilen Faulung (= aerobthermophile Verfahren), ferner Produkte der aeroben Klärschlamm-Kompostierung nach dem System der Schnellrotte, weiterhin mikrobiell befallene Faserschlämme, Schlämme der Nahrungs- und Genußmittel-Industrie, Schlämme aus Molkereien und Schlachthöfen sowie bereits getrocknete und in Deponien befindliche Bioschlämme.

Bei dem erfindungsgemäßen Verfahren können auch Gemische verschiedenster Biomassen eingesetzt werden. Ferner ist das erfindungsgemäße Verfahren auch dann anwendbar, wenn die Biomassen verschiedenartigste Verunreinigungen enthalten. Beispielsweise genannt seien in diesem Zusammenhang Biomassen, in denen Schwermetall-Salze, Pflanzenschutzmittel, Antibiotika oder andere organische oder anorganische Chemikalien vorhanden sind.

Als reaktive Komponenten der Biomassen können fungieren :

— Alle Proteine beliebiger Zellarten, von Einzellern bis zu höchst differenzierten Mehrzellern.

— Alle Lipoproteine der verschiedensten Zellarten der belebten Natur.

— Verschiedenste Desoxyribonucleinsäuren sowie die verschiedensten Ribonucleinsäuren.

— Glykoproteide als Bestandteile der verschiedenartigsten Enzyme.

— Nucleoproteide.

— Phosphorproteine.

— Phosphatide, insbesondere Inositphosphatid, Colamin-kephalin und Serin-kephalin.

— Lipoide oder Plasmalogene, soweit sie als Base Colamin in Form eines Phosphorsäureesters gebunden enthalten.

— Alle Zucker und polysaccharidartigen Zellreserve- und Zellinhaltsstoffe, Hemicellulosen, Stärken, Pektine und Lignine.

— Bestandteile der Zellwände von verschiedenartigsten Bacterienarten. Derartige Stoffe sind beispielsweise Polymere von Aminozuckern (Acetylglykosamin + N-Acetylmuraminsäure), die im N-Acetylmuraminsäure-Anteil über Polypeptide vernetzt sind.

— Zellwandbestandteile von verschiedenartigsten Pilzen und Algen. Beispielsweise genannt seien Cellulosen, Hemicellulosen und andere Polysaccharide und Chitinanteile mit Acetyl-glukosamin- bzw. Acetyl-galaktosamin-Anteilen.

— Enzyme, wie Glukoseoxidase, Katalase, Glukose-Isomerase, Invertase, Lactase, Naringinase, Lipasen, Asparaginasen, α-Amylasen und Glykoamylasen, Cellulasen, Lysozyme, Proteasen usw.

Biomassen der Penicillin-Herstellung (Penicillium chrysogenum) können als lebende Pilzmassen, als tote Zellaggregate oder als bereits in Gärung und Zersetzung oder partielle Fäulnis übergegangene Systeme beim erfindungsgemäßen Verfahren eingesetzt werden. Die zum Beispiel über Filterpressen abfiltrierten Biosysteme der Penicillin-Herstellung können selbst bei einem gewissen adsorbierten Penicillingehalt erfindungsgemäß kondensiert werden, wobei an der Biomasse noch adsorbiertes Penicillin durch Kondensation in seiner antibiotischen Wirksamkeit vollständig desaktiviert wird.

Biomassen der Sisomizin-Herstellung, die aus Micromonospora (= fädige Bakterien) bestehen, und in denen der Wirkstoff zunächst intracellulär gebildet und dann durch Zellsprengung beim Ansäurern in die wäßrige Phase überführt wird, fallen als unlösliche, klebrige Produkte an und können als solche bei der erfindungsgemäßen Umsetzung verwendet werden ; ebenso geeignet sind Biomassen aus Streptomyces-Arten und Coli-Bakterien.

Biomassen, die aus Mikroorganismenmassen von Fermentationsprozessen der Arzneimittel-, Enzym-, Lebensmittel- und Genußmittel-Industrie bestehen, nehmen gegenüber den Mikroorganismenmassen aus biologischen Kläranlagen, — den sogenannten Anwasser- oder Überschußschlämmen (« Belebtschlämmen ») —, dadurch eine Sonderstellung ein, daß sie (die erstgenannten Biomassen) eine sehr gleichmäßige Zusammensetzung besitzen und völlig frei von Schwermetall-Salzen sind. Sie kommen daher auch bevorzugt als Reaktionskomponenten bei dem erfindungsgemäßen Verfahren in Frage, sofern die Verfahrensprodukte auf dem Agrar- bzw. Futtermittel-Sektor verwendet werden. Besonders bevorzugt sind innerhalb dieses Komplexes industriell genutzte Mikroorganismen, deren Stoffwechsel zur Gewinnung hochwertiger organischer und pharmazeutischer Produkte aus natürlichen Rohstoffen gelenkt werden kann.

Als Carbonylverbindungen, die bei der Durchführung des erfindungsgemäßen Verfahrens als Reaktionskomponenten eingesetzt werden können, kommen alle üblichen Carbonylverbindungen mit ausreichend reaktiven Carbonylgruppen in Betracht. Hierzu gehören vorzugsweise Aldehyde und ketone.

Besonders bevorzugte Aldehyde sind gesättigte, aliphatische, gegebenenfalls durch Halogen oder Hydroxy substituierte Mono-Aldehyde, wie Formaldehyd, Acetaldehyd, Butyraldehyd, Isobutyraldehyd, Pivalinaldehyd, Chloral, Hydroxyacetaldehyd, Hydroxypivalinaldehyd, Glycerinaldehyd, Hydroxyaldehyde, die in Formose-Zuckergemischen enthalten sind, und Hydroxyaldehyde, die aus anderen Aldehyde durch Aldol-Kondensationen entstehen. Besonders bevorzugte Aldehyde sind weiterhin ungesättigte aliphatische Aldehyde, wie Acrolein und Crotonaldehyd, ferner cycloaliphatische Aldehyde, wie Cyclohexanaldehyd, außerdem aliphatische Dialdehyde, wie Glyoxal, Methylglyoxal, Glyoxalsulfat und Glutardialdehyd, darüberhinaus aromatische Aldehyde, wie Benzaldehyd, 4-Methylbenzaldehyd, Salicylaldehyd und Terephthaldialdehyd, sowie ferner von Heterocyclen abgeleitete Aldehyde, wie Furfurol und Hydroxymethyl-furfurol.

Bevorzugt verwendbar sind auch « verkappte Aldehyde », also solche Verbindungen, die unter den Reaktionsbedingungen Aldehyde freisetzten oder wie Aldehyde reagieren. Speziell genannt seien in diesem Zusammenhang Paraformaldehyd, Trioxan, Chloralhydrat, Hexamethylentetramin und Halbacetale von Aldehyden, insbesondere von Formaldehyd, mit mono-, di- oder polyfunktionellen Alkoholen, wie Methanol, Äthanol, Butanol, Äthylenglykol und Diäthylenglykol.

Besonders bevorzugte Ketone sind Hydroxyaceton, Dihydroxyaceton, Methyläthylketon, Methylisobutylketon, Cyclohexanon, Acetophenon und Chinone, wie Benzochinon.

Verwendet werden können auch Mischungen aus Aldehyden und/oder Ketonen, wobei Mischungen aus Formaldehyd und anderen Aldehyden oder Ketonen besonders bevorzugt sind Aus derartigen Mischungen von Formaldehyd mit zahlreichen Aldehyden oder Ketonen, die α-ständige Wasserstoffatome besitzen, können durch Aldol-Kondensationen in situ Hydroxyaldehyde bzw. Hydroxyketone entstehen, wie dies für Formaldehyd und Isobutyraldehyd durch das nachstehende Formelschema veranschaulicht ist.

a) 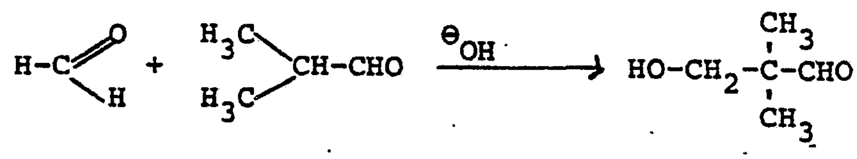

Speziell erwähnt seien hierbei außerdem diejenigen Hydroxyaldehyde, deren Bildung durch die nachstehenden Reaktionsgleichungen wiedergegeben ist.

b) 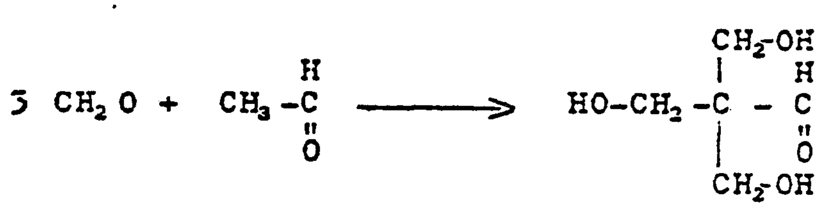

c) 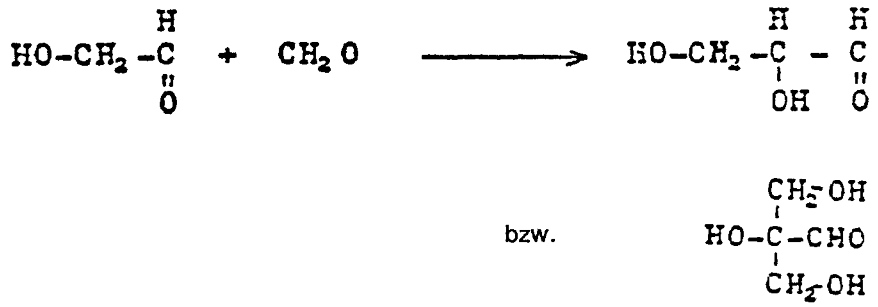


d) 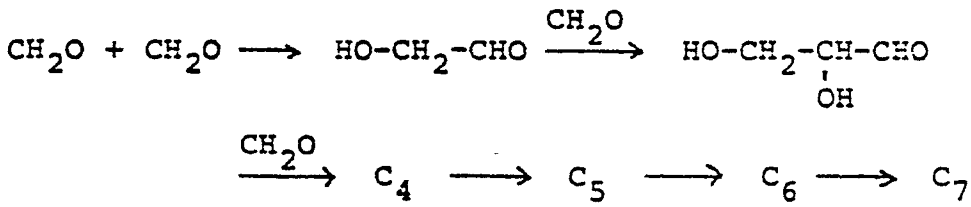


Polyhydroxyaldehyde

e) 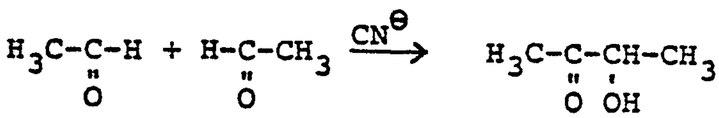


Die Umsetzung gemäß Gleichung (d) entspricht dabei der Butlerow-Löw-Formose-Reaktion.

Bei der Umsetzung gemäß Gleichung (e) handelt es sich um eine Acyloinkondensation, die ablaufen kann, wenn Cyanid-Ionen im Reaktionsgemisch in katalytischer Menge enthalten sind.

Analog können Ketone mit α-ständigen Wasserstoffatomen mit Formaldehyd reagieren. Die so entstehenden Hydroxyaldehyde und Polyhydroxyketone gehen im Maße ihrer Bildung, insbesondere im schwach bis stark alkalischen Bereich, zum Beispiel mit Harnstoff und vielen Aminoplast-bildern leicht Additionsreaktionen zu N-Alkylol-Verbindungen ein, die wiederum Kondensationspartner für die vorgenannten Biomassen darstellen.

Als Thiocarbonylverbindungen , die bei der Durchführung des erfindungsgemäßen Verfahrens als Reaktionskomponenten eingesetzt werden können, kommen alle üblichen Thiocarbonylverbindungen mit ausreichend reaktiven Thiocarbonylgruppen in Betracht. Hierzu gehören vorzugsweise Thioaldehyde und Thioketone. Besonders bevorzugte Thioaldehyde und Thioketone sind solche, die sich von denjenigen Aldehyden und Ketonen ableiten, die bereits als besonders bevorzugt genannt wurden.

Bevorzugt verwendbar sind auch « verkappte Thioaldehyde », also solche Verbindungen, die unter den Reaktionsbedingungen Thioaldehyde freisetzen. Speziell genannt sei der trimere Thioformaldehyd, — das Trithian —, der bei erhöhter Temperatur in Gegenwart von Säuren in Thioformaldehyd zerfällt.

Als Carbonylverbindungen, die mit niedermolekularen, nicht kondensierten N-Alkylolverbindungen im Dissoziationsgleichgewicht stehen, kommen vorzugsweise einfache Aldehyde, insbesondere Formaldehyd in Betracht, der mit den entsprechenden N-Methylolverbindungen im Gleichgewicht steht. Zu solchen N-Methylolverbindungen gehören insbesondere N-Methylol-harnstoff, N,N'-Dimethylol-harnstoff, methyloliertes Dicyandiamid, methyloliertes Oxamid, N-Methylol-thioharnstoff, N,N'-Dimethylolthioharnstoff und methylolierte Melamine, wie Hexamethylolmelamin und Trishydroxymethyl-melamin der Konstitution

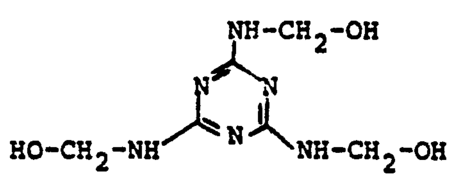

Weiterhin genannt sei Monomethylol-äthylenharnstoff der Konstitution

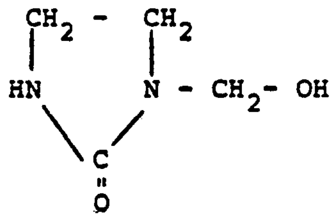

Monomethylol-äthylenthioharnstoff der Konstitution

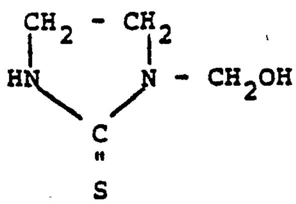

und Tetramethylolacetylen-diharnstoff der Konstitution

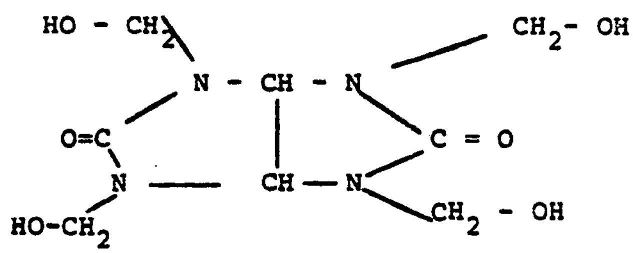

In Betracht kommen ferner Alkylol-Verbindungen, die sich von einfachen Aldehyden ableiten, bevorzugt von solchen mit bis zu 5 Kohlenstoffatomen.

Insbesondere kommen bei der Durchführung der erfindungsgemäßen Verfahrens als Carbonylverbindungen die folgenden Stoffe in Frage: Formaldehyd, Acetaldehyd, Isobutyraldehyd, Crotonaldehyd, Glyoxal, Furfurol, Hydroxymethylfurfurol, Salicylaldehyd sowie deren Halbacetale, außerdem Polymere des Formaldehyds, wie Paraformaldehyd und Trioxan, ferner Hexamethylentetramin und auch Thioaldehyde, wie Thioformaldehyd. Als nicht kondensierte (niedermolekulare) N-Alkylolverbindungen kommen bei der Durchführung des erfindungsgemäßen Verfahrens insbesondere in Betracht: N-Methylolharnstoff, Dimethylolharnstoff, Trimethylolmelamin, Hexamethylolmelamin, Monomethylol-äthylenharnstoff, Monomethylol-äthylenthioharnstoff und Tetramethylolacetylen-diharnstoff.

Bei dem erfindungsgemäßen Verfahren werden als Ausgangsstoffe weiterhin Aminoplastbildner eingesetzt. Unter Aminoplastbildnern sind hierbei alle diejenigen Stickstoff-Verbindungen zu verstehen, die in der Lage sind, mit reaktionsfähigen Carbonyl-Verbindungen N-Oligo- und N-Polykondensationsprodukte zu bilden.

Hierzu gehören Stickstoffverbindungen, wie Harnstoffe, z.B. der Harnstoff selbst, Acetylenharnstoff, Dimethylacetylenharnstoff und N-Methylenharnstoff, ferner Thioharnstoffe, wie der unsubstituierte Thioharnstoff, weiterhin Diharnstoffe, wie Hexamethylendiharnstoff, Tetramethylendiharnstoff und Äthylendiharnstoff, außerdem Polyharnstoffe wie sie durch Umsetzung von aliphatischen, cycloaliphatischen bzw. araliphatischen Di- oder Triisocyanaten oder auch Biuret-polyisocyanaten mit Ammoniak oder primären Aminen erhalten werden, darüber hinaus Polycarbonsäureamide, wie Oxalsäurediamid, Bernsteinsäurediamid und Adipinsäurediamid, ferner Mono-, Di- und Polyurethane, wie beispielsweise die Umsetzungsprodukte von aliphatischen, cycloaliphatischen, araliphatischen und aromatischen Mono- oder Bischlorameisensäureestern mit Ammoniak oder primären Aminen, außerdem Biurete, Melamine, wie das Melamin selbst, Amidine, wie Dicyandiamidin, Guanidine, wie Aminoguanidin, weiterhin Guanazole, Guanamine, Cyanamid, Dicyandiamid, primäre Monoamine, sekundäre Monoamine, Arylamine, Ammoniak, Diamine, Triamine, Hydrazine und Carbonsäurehydrazide, wie Hydrazodicarbonamid, Carbacinsäureester und Hydrazodicarbonsäureester, und außerdem ähnliche zur Aminoplastbildung befähigte Stickstoffverbindungen, vorzugsweise die den vorgenannten stickstoffverbindungen entsprechenden N-Alkylolgruppen, vorzugsweise N-Methylolgruppen aufweisenden Derivate und die entsprechenden $C_1$-$C_4$ Alkyläther dieser N-Alkylol-Derivate.

Ferner kommen als Aminoplastbildner vorzugsweise auch höhermolekulare α,ω-Di-harnstoffe in Betracht, deren N-Methylol-Derivate und N-Methylolalkyläther, ferner α,ω-Bis-alkoxymethylurethane, welche zwischen den α-,ω-ständigen funktionellen Gruppen Polyäther-, Polythioäther-, Polyacetal-, Polyester-, Polyesteramid- oder Polycarbonatreste vom Durchschnittsmolekulargewicht 400 bis 10 000 und gegebenenfalls zusätzliche Urethan- oder substituierte Harnstoffgruppen aufweisen. Besonders bevorzugt sind hierbei als höhermolekulare zur Aminoplastbildung befähigte Stickstoffverbindungen wasserlösliche oder in Wasser dispergierbare Verbindungen, z.B. Verbindungen, welche zwischen den α,ω-ständigen funktionellen Urethan- oder Harnstoffgruppen Polyäthylenoxidreste oder Reste von Mischpolymerisaten des Äthylenoxids mit Propylenoxid bzw. Tetrahydrofuran oder von wasserlöslichen Polyacetalen, hergestellt aus Di-, Tri- oder Tetraäthylenglykol und Formaldehyd, aufweisen.

Diese als Ausgangsverbindungen benötigten Aminoplastbildner sind bekannt oder lassen sich nach im Prinzip bekannten Methoden herstellen (vgl. Houben-Weyl « Methoden der organischen Chemie », Band XIV, Teil 2 (1963), Seiten 319-402, Georg Thieme-Verlag, Stuttgart).

Bei der Durchführung des erfindungsgemäßen Verfahrens kommen als Aminoplastbildner vorzugsweise auch « modifizierte Aminoplastbildner » in Betracht. Hierunter sind Aminoplastbildner zu verstehen, die zusätzliche leicht einbaubare Stoffe enthalten. Genannt seien z.B. Verbindungen, die rasch und leicht durch Mischkondensation einbaufähig sind. Hierzu gehören vorzugsweise Polyurethane und polyharnstoffe mit $NH_2$-Endgruppen, Polyamide aus Poly-(β-alanin) mit Molgewichten bis 2 000, N-Methylolmethyläther von Polycaprolactam, Polythiolactame, Polypeptide aus N-Carboxy-α-aminocarbonsäuren, niedermolekulare Polyamide aus aliphatischen Dicarbonsäuren und Diaminen, Polyamide aus cycloaliphatischen Komponenten und aromatischen Komponenten, Polyamide mit O- bzw. S- oder N-als Heteroatome, Polyesteramide, Mischkondensate, die neben Amidgruppen noch Ester-, Urethan- oder Harnstoffgruppen enthalten, äthoxylierte und propoxylierte Mono- und Polyamide, Polyhydrazide und Polyaminotriazole, Polysulfonamide, Formaldehyd-Mischkondensate mit Harnstoff, Melamin und Dicyandiamid, niedermolekulare Anilin-Formaldehyd-Kondensate, Sulfonsäureamide, Mono- und Dinitrile, Acrylnitril, Urotropin, Hexahydrotriazine aus primären Aminen und Formaldehyd, Schiff'sche Basen und Ketimine oder polyketimine, wie z.B. solche aus einem Mol Hexamethylendiamin und 2 Mol Cyclohexanon, Polyadditionsprodukte und Polykondensationsprodukte des Melamins und anderer Aminoheterocyclen mit Aldehyden und Alkoholen, Polyadditions- und Polykondensationsprodukte von Nitrilen mit Aldehyden, Umsetzungsprodukte von phosphoriger Säure und Dialkylphosphiten mit Carbonylverbindungen und Aminen bzw. Polyaminen. Ferner kommen als zur Aminoplastbildung befähigte Verbindungen in diesem Zusammenhang auch diejenigen Verbindungen in Frage, die in der DE-OS 2 324 134 auf den Seiten 7 bis 12 angeführt sind.

Zu den modifizierten Aminoplastbildnern, die bei dem erfindungsgemäßen Verfahren eingesetzt werden können, gehören weiterhin N-Alkylolverbindungen und insbesondere N-Methylolverbindungen, die zum Teil veräthert sind mit Verbindungen wie

— polyfunktionellen Hydroxylverbindungen, z.B. Polyalkoholen, wobei Äthylenglykol, Glyzerin, Formose-Zuckergemische, Glukose, Oligo- und Polysaccharide sowie Stärke beispielhaft genannt seien ;

— Polyäthern mehr oder wenigen hoher OH-Funktionalität, wie sie in der polyurethanchemie verwendet werden, also z.B. Polyäther aus Propylenoxid, die anteilweise Äthylenoxidsegmente, sei es in Mischblöcken, in statistischer Verteilung oder bevorzugt als endständige Segmente enthalten und die primäre Hydroxylgruppen als Endgruppen besitzen, wobei diese Polyäther bis zu 70 Gew.-% und mehr an Polyäthylenoxid-Segmenten enthalten können und bevorzugt 13-30 Gew.-%, — bezogen auf eingebaute Propylenoxid-Segmente —, an Polyäthylenoxid-Segmenten enthalten können ;

— höherschmelzende, reine Polyäthylenoxide vom Durchschnittsmolekulargewicht 500-60 000, wobei insbesondere in Frage kommen Additionsprodukte des Propylenoxids an Trimethylolpropan bzw. Glyzerin, die in zweiter Stufe mit Äthylenoxid derartig umgesetzt werden, daß auf etwa 83-87 Gew.-Teile an gebundenem Propylenoxid etwa 17-13 Gew.-Teile an Äthylenoxid entfallen ;

— Polyhydroxylverbindungen mit einem Durchschnittsmolekulargewicht von 250-14 000, vorzugsweise 400-6 000, welche gegebenenfalls im Gemisch mit niedermolekularen Polyhydroxylverbindungen des Molekulargewichtsbereiches 62-250 vorliegen ;

— höhermolekularen Polyhydroxylverbindungen, wie z.B. mindestens zwei endständige Hydroxylgruppen aufweisende Polyäther, in denen bevorzugt mindestens 10 % der Hydroxylgruppen primäre Hydroxylgruppen darstellen ;

— Polyhydroxylpolyestern, wie sie in großer Variationsbreite beim Diisocyanat-polyadditionsverfahren eingesetzt werden.

Der Anteil von Alkoholen bzw. Polyalkoholen in diesen Produkten kann je nach Komponente bis zu 60 Gew.-%, bezogen auf die Summe der Prozente an Stickstoff-Verbindungen und Alkoholen, betragen.

Bei der Durchführung des erfindungsgemäßen Verfahrens kommen als Aminoplastbildner insbesondere unter anderem die folgenden Stoffe in Frage : Harnstoff, Thioharnstoff, Diharnstoffe, wie z.B. Hexamethylendiharnstoff, Tetramethylendiharnstoff, Äthylenharnstoff, Acetylenharnstoff, Dimethylacetylenharnstoff, Oxalsäurediamid, Bernsteinsäurediamid, Adipinsäurediamid, Mono- oder Bishydrazide, wie Hydrazodicarbonamid, Carbazinsäure-methyl- und äthylester, Hydrazodicarbonsäureester, Mono- und insbesondere Diurethane, wie beispielsweise die Umsetzungsprodukte von aliphatischen, cycloaliphatischen, araliphatischen und aromatischen Mono- oder Bis-chlorameisensäureestern mit Ammoniak und primären Aminen, ferner Anilin, Melamin, Dicyandiamid, Cyanamid, Aminoguanidin, Dicyandiamidin, Guanamine, Guanazole, ferner Polyharnstoffe und Polybiurete, wie sie durch Umsetzung von aliphatischen, cycloaliphatischen, araliphatischen Di-oder Triisocyanaten, wie auch Biuretpolyisocyanaten mit einem Überschuß an Ammoniak oder primären Aminen erhalten werden.

Im übrigen kommen bei der Durchführung des erfindungsgemäßen Verfahrens als Aminoplastbildner auch nahezu fehlerstellenfreie Azulminsäuren, fehlerstellenhaltige sogenannte modifizierte Azulminsäuren, durch Kondensation mit Carbonylverbindungen stabilisierte Azulminsäuren, ferner durch Kondensation mit Carbonylverbindungen und Aminoplastbildnern bzw. deren niedermolekularen Kondensationsprodukten stabilisierte Azulminsäuren, sowie Metallsalzkomplexe der vorgenannten Azulminsäuren in Frage. Diese Stoffe werden bei dem erfindungsgemäßen Verfahren bevorzugt zusammen mit anderen Aminoplastbildnern, insbesondere Harnstoff, eingesetzt.

Unter nahezu fehlerstellenfreien Azulminsäuren sind braunschwarze bis schwarze, in allen inerten Solventien unlösliche Blausäure-Polymerisate zu verstehen, denen nach einem Vorschlag von Th. Völker im wesentlichen die folgende Formel zukommt (vgl. Angew. Chem *72,* (1960) Seiten 379-384) :

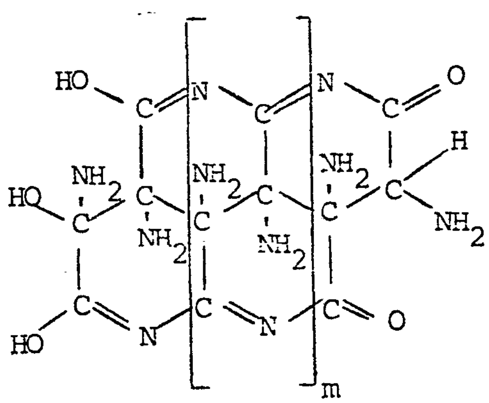

Aus den Sauerstoffgehalten dieser Azulminsäuren wurde ein Polymerisationsgrad (HCN) von X = 15 – 24 berechnet, so daß sich für *m* (Formel I) Werte von 1 bis 4 ergeben. Die maximal erzielten Molekulargewichte der Polymere liegen wenig oberhalb von 700.

Derartige nahezu fehlerstellenfreie Azulminsäuren sind bekannt (vgl. Houben-Weyl, Band 8 (1952), Seite 261, DT-PS 662 338 und DT-PS 949 600).

Unter fehlerstellenhaltigen Azulminsäuren (modifizierte Azulminsäuren) sind solche Azulminsäuren zu verstehen, die einen Gehalt von 0,5 bis 55 Gewichtsprozent an ionischen Gruppen der Formel

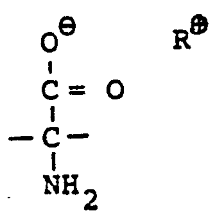

$(F_1)$

in welcher

R für Wasserstoff, Ammonium, ein Äquivalent einer protonisierten oder quaternierten organischen Stickstoffbase, eines Sulfonium-Kations oder für ein Äquivalent eines Metallkations steht,

und einen Gehalt von 0,5 bis 15 Gewichtsprozent an durch Decarboxylierungsreaktionen standenen Gruppen der Formel

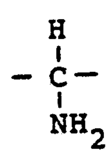

$(F_2)$

aufweisen.

Vorzugsweise verwendbar bei dem erfindungsgemäßen Verfahren sind solche modifizierten Azulminsäuren, in denen R für Wasserstoff, Ammonium, oder ein Äquivalent eines Kations von einem

Metall aus der I. bis V. Hauptgruppe bzw. aus der I. bis VIII. Nebengruppe steht, wobei als Kationen Lithium, Natrium, Kalium, Beryllium, Magnesium, Calcium, Strontium, Barium, Aluminium, Thallium, Zinn, Blei, Wismut, Kupfer, Silber, Gold, Zink, Cadmium, Quecksilber, Titan, Zirkon, Chrom, Mangan, Eisen, Cobalt, Nickel, Platin und Palladium, Rhodium und Ruthenium beispielhaft genannt seien.

Bevorzugt verwendbar sind ferner solche modifizierten Azulminsäuren, in denen R für ein Äquivalent eines protonisierten Alkylamins mit 1 bis 6 Kohlenstoffatomen, eines protonisierten Dialkylamins mit 1 bis 6 Kohlenstoffatomen pro Alkylgruppe, eines protonisierten Trialkylamins mit 1 bis 6 Kohlenstoffatomen pro Alkylgruppe, eines protonisierten Hydroxyalkylamins mit 1 bis 6 Kohlenstoffatomen, eines protonisierten Di-(hydroxy-alkyl)-amins mit 1 bis 6 Kohlenstoffatomen pro Hydroxyalkylgruppe, eines protonisierten Tri-(hydroxyalkyl)-amins mit 1 bis 6 Kohlenstoffatomen pro Hydroxyalkylgruppe, eines protonisiserten Cycloalkylamins mit 3 bis 8 Kohlenstoffatomen, eines protonisierten Alkylendiamins mit 2 bis 6 Kohlenstoffatomen, eines protonisierten Guanidins, Melamins oder Dicyandiamids oder einer protonisierten, gesättigten oder ungesättigten heterocyclischen Stickstoffbase mit 5 bis 7 Ringgliedern und 1 bis 3 Stickstoffatomen im heterocyclischen Ring, sowie für diejenigen Kationen steht, die durch Quaternisierung, wie z.B. Permethylierung, der vorgenannten basischen Stickstoff-Verbindungen entstehen. Besonders bevorzugte Stickstoffbasen sind in diesem Zusammenhang Methylamin, Dimethylamin, Trimethylamin, Äthylamin, Diäthylamin, Triäthylamin, tert.-Butylamin, Äthanolamin, Diäthanolamin, Triäthanolamin, Cyclopropylamin, Cyclopentylamin, Cyclohexylamin, Äthylendiamin, Pyrrolidin, Piperidin, Morpholin, Imidazol, Pyrazol, 1,2,4-Triazol, 1,2,3-Triazol, 2-Äthylimidazol und Aminotriazol.

Weiterhin bevorzugt sind solche modifizierten Azulminsäuren, in denen R für Trialkylsulfonium-Kationen, insbesondere für das Triäthylsulfonium-Kation steht.

In den modifizierten Azulminsäuren vorhandene ($F_1$)- und ($F_2$)-Gruppen haben ihren Ursprung in Nitrilgruppen, die in der Azulminsäure vorhanden sind und als Haltepunkte der cyclisierenden Nitrilpolymerisation aufgefaßt werden können.

In idealisierter Darstellung kann der Übergang von einer Nitrilgruppe der Azulminsäure in eine entsprechende Carboxylgruppe formelmäßig wie folgt veranschaulicht werden :

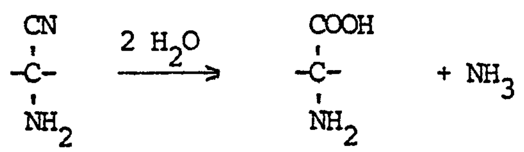


bzw.

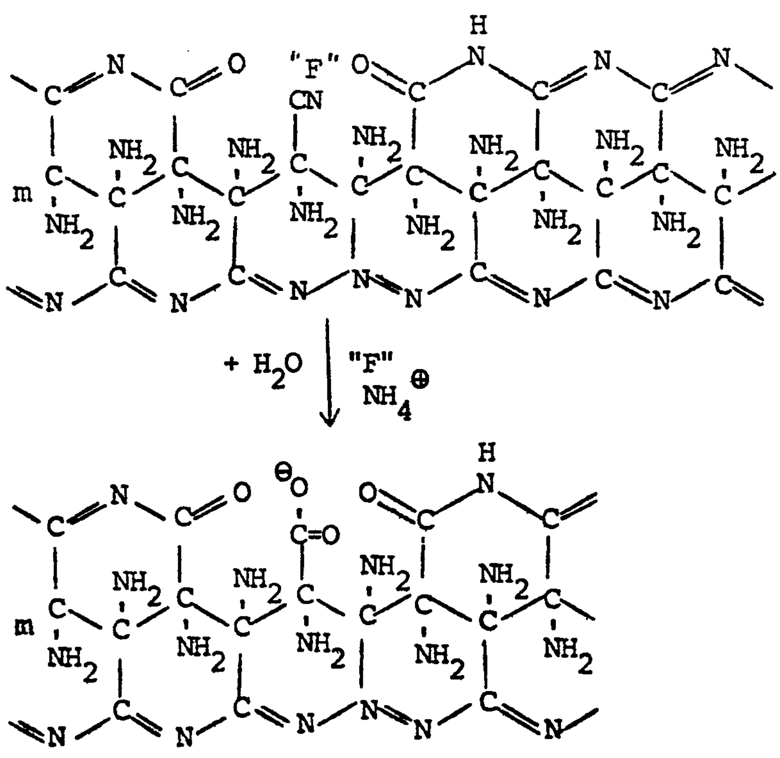


(II)

Selbstverständlich ist auch die Bildung von Amid- oder Imidgruppen aus Nitrilgruppen gegeben. So läßt sich z.B. die Bildung von Amidgruppen durch das nachstehende Formelschema wiedergeben.

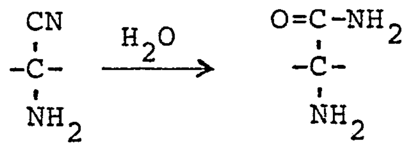


Die Erzeugung von ($F_1$)- und ($F_2$)-Gruppen erfolgt nicht nur an Nitril-Gruppen, die in dem eingesetzten Polymerisat bereits vorhanden sind, sondern auch an solchen Nitrilgruppen, die durch katalytische Entcyclisierungen entstehen. Darüber hinaus sind verschiedene andere Hydrolyse-Reaktionen für die Bildung von Fehlerstellen verantwortliche. Z.B. kann eine

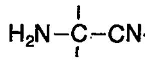-Gruppe,

die im Molekularverband der Azulminsäure als α-Aminonitril aufzufassen ist, durch Cyanwasserstoff-Abspaltung und anschließende topochemische Hydrolysereaktion gemäß nachstehendem Formelschema in eine Carbonylgruppe überführt werden :

a)

b)

c)

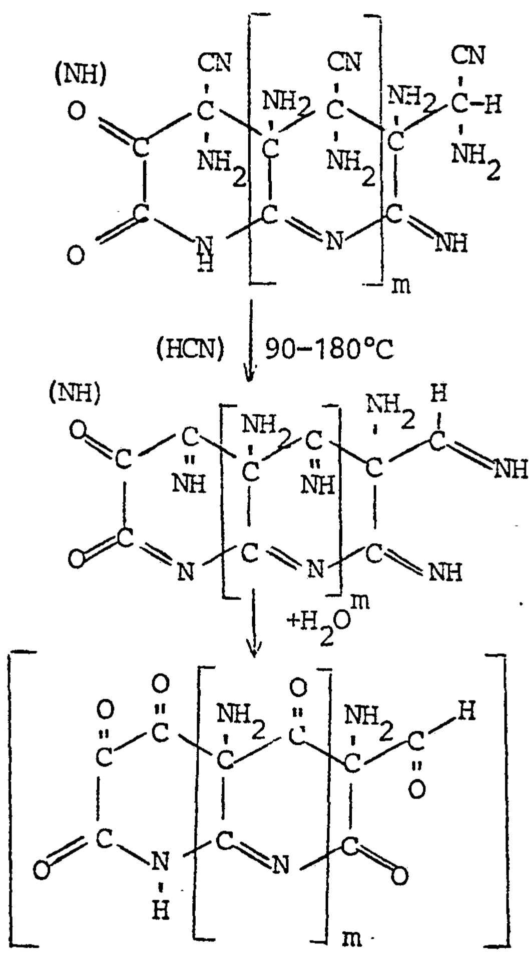


Im folgenden werden die ionischen Gruppen der Formel

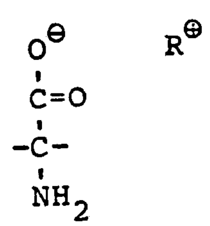

als *$F_1$-Fehlerstellen* und die Gruppen der Formel

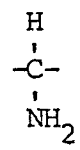

als *$F_2$-Fehlerstellen* bezeichnet.

Die $F_2$-Fehlerstellen entstehen aus den $F_1$-Fehlerstellen, in denen R für Wasserstoff oder ein anderes geeignetes Ion steht, gemäß nachstehendem Formelschema :

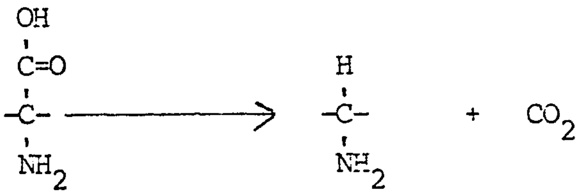

bzw. im Molekularverband der Azulminsäure :

Fehlerstellen durch. Decarboxylierungsreaktion

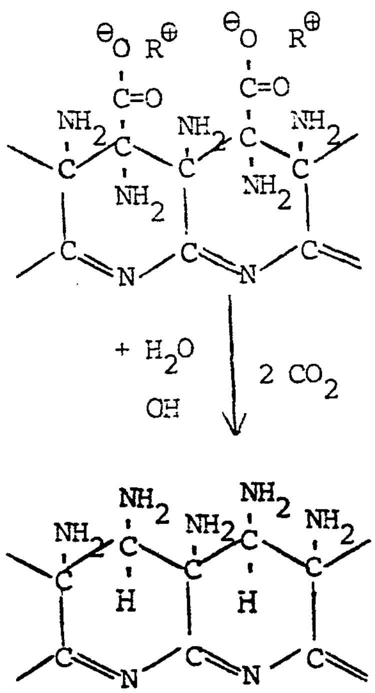


Da die Entstehung der $F_1$-Fehlerstellen mit der Freisetzung von Ammoniak und die Entstehung der $F_2$-Fehlerstellen mit der Freisetzung von Kohlendioxid gekoppelt ist, stellt die entbundene Menge an Ammoniak und Kohlendioxid ein quantitatives Maß für die Menge der erzeugten Fehlerstellen dar. Der Quotient aus der entbundenen Molmenge an Ammoniak und der entbundenen Molmenge an Kohlendioxid gibt Aufschluß über das Verhältnis von $F_1$- zu $F_2$-Fehlerstellen.

Der Fehlerstellengehalt in modifizierten Azulminsäuren ausgedrückt in Gewichtsprozent kann jeweils in der Weise bestimmt werden, daß man das Äquivalentgewicht der betreffenden Fehlerstelle (= ionische oder nichtionische Gruppierung $F_1$ oder $F_2$) in Relation setzt zu der entsprechenden nicht in eine ionische oder nicht-ionische Gruppierung überführten Gewichtsgröße (100 g). So errechnet sich beispielsweise die Fehlerstellenkonzentration für eine $F_1$-Fehlerstelle, in der R für Wasserstoff steht, aus der jeweils entstandenen molaren Menge an Ammoniak und der Tatsache, daß die zugehörige ionische Gruppierung der Formel

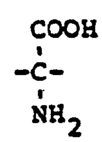

ein Äquivalentgewicht von 73 aufweist.

In analoger Weise errechnet sich der $F_2$-Fehlerstellengehalt aus der jeweils entbundenen molaren Menge an Kohlendioxid und der Tatsache, daß die betreffende Gruppierung der Formel

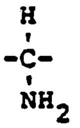

ein Äquivalentgewicht von 29 aufweist.

Die genannten fehlerstellenhaltigen Azulminsäuren sind bisher noch nicht bekannt. Sie lassen sich jedoch in einfacher Weise nach mehreren Verfahren herstellen. So erhält man derartige modifizierte Azulminsäuren dadurch, daß man

— nahezu fehlerstellenfreie bekannte Azulminsäuren in wässrigen Medium bei Temperaturen zwischen 0 °C und 200 °C, vorzugsweise zwischen 20 °C und 120 °C

a) mit organischen oder anorganischen Säuren behandelt, oder

b) mit Basen oder basischen Salzen behandelt, oder

c) mit Wasser im Neutralbereich behandelt, oder

d) mit pflanzlichen Aschen, katalytisch wirksamen Naturstoffen und/oder Düngemitteln behandelt, oder

e) gegebenenfalls in Anwesenheit von Oxidationsmitteln sowie gegebenenfalls in Anwesenheit von organischen Säuren mit Metallsalzen behandelt, oder

f) mit Metallsalz-Komplexen stabilisierter Azulminsäuren behandelt, oder

g) mit Oxidationsmitteln behandelt,

oder daß man

— Blausäure mit Hilfe basischer Katalysatoren, wie Natriumcyanat, unter hydrolysierenden Bedingungen in wässrigen Medium bei Temperaturen zwischen 0 °C und 100 °C, vorzugsweise zwischen 20 °C und 95 °C, gegebenenfalls in Anwesenheit von Zusatzstoffen polymerisiert,

oder daß man

— modifizierte Azulminsäuren im wässrigen Medium bei Temperaturen zwischen 50 °C und 120 °C, vorzugsweise zwischen 60 °C und 100 °C, mit starken Basen, wie z.B. Natriumhydroxid oder Kaliumhydroxid, umsetzt und gegebenenfalls anschließend das Kation durch Behandlung mit Metallsalzen austauscht,

oder daß man

— modifizierte Azulminsäuren in wässrigem Medium bei Temperaturen zwischen 0 °C und 200 °C, vorzugsweise zwischen 20 °C und 120 °C, mit organischen oder anorganischen Säuren behandelt.

Die Isolierung der Reaktionsprodukte erfolgt bei allen diesen Verfahren nach üblichen Methoden. Im allgemeinen verfährt man in der Weise, daß man nach beendeter Umsetzung das feste Reaktionsprodukt abfiltriert, wäscht und trocknet.

Unter « durch Kondensation mit Carbonylverbindungen stabilisierten Azulminsäuren » sind solche Azulminsäuren zu verstehen, die einen Gehalt von 0,5 bis 55 Gewichtsprozent an ionischen Gruppen der Formel

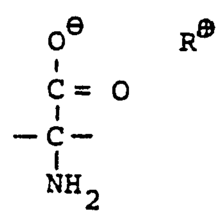

$(F_1)$

in welcher

R die oben angegebene Bedeutung hat, und einen Gehalt von 0,5 bis 15 Gewichtsprozent an durch Decarboxylierungsreaktionen entstandenen Gruppen der Formel

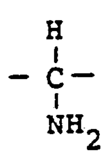

$(F_2)$

aufweisen und durch Umsetzung mit Carbonylverbindungen gegenüber Blausäureabspaltung stabilisiert sind.

Vorzugsweise verwendbar bei dem erfindungsgemäßen Verfahren sind solche stabilisierten Azulminsäuren

— in denen R diejenigen Bedeutungen hat, die schon im Zusammenhang mit der Beschreibung der modifizierten Azulminsäuren vorzugsweise für R aufgeführt wurden, und

— bei denen als Carbonylkomponenten zur Stabilisierung einfache Aldehyde, insbesondere Formaldehyd, benutzt wurden.

Die genannten durch Kondensation mit Carbonylverbindungen stabilisierten Azulminsäuren sind bisher noch nicht bekannt. Sie lassen sich jedoch in einfacher Weise nach mehreren Verfahren herstellen. So erhält man derartige durch Kondensation mit Carbonylverbindungen stabilisierten Azulminsäuren dadurch, daß man

— modifizierte Azulminsäuren mit einem Gehalt von 0,5 bis 55 Gewichtsprozent an ionischen Gruppen der Formel

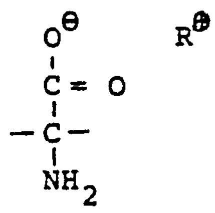

$(F_1)$

in welcher

R die oben angegebene Bedeutung hat,

und mit einem Gehalt von 0,5 bis 15 Gewichtsprozent an Gruppen der Formel

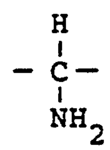

$(F_2)$

im wässrigen Medium bei Temperaturen zwischen 10 °C und 250 °C, vorzugsweise zwischen 50 °C und 150 °C, mit Carbonylverbindungen kondensiert,

oder daß man

— Säureadditions-Salze bzw. Komplex-Verbindungen von modifizierten Azulminsäuren mit einem Gehalt von 0,5 bis 55 Gewichtsprozent an ionischen Gruppen der Formel

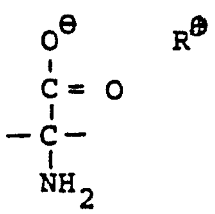

$(F_1)$

in welcher

R die oben angegebene Bedeutung hat,

und mit einem Gehalt von 0,5 bis 15 Gewichtsprozent an Gruppen der Formel

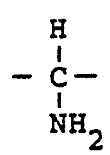

$(F_2)$

in wässrigem Medium bei Temperaturen zwischen 10 °C und 250 °C, vorzugsweise zwischen 50 °C und 150 °C mit Carbonylverbindungen kondensiert,

oder daß man

— nahezu fehlerstellenfreie Azulminsäuren in wässrigem Medium bei Temperaturen zwischen 10 °C und 250 °C, vorzugsweise zwischen 50 °C und 150 °C, mit Carbonylverbindungen kondensiert,

oder daß man

— Blausäure mit Hilfe basischer Katalysatoren, wie Natriumcyanat, unter hydrolysierenden Bedingungen in wässrigem Medium bei Temperaturen zwischen 0 °C und 100 °C, vorzugsweise zwischen 20 °C und 95 °C, gegebenenfalls in Gegenwart von Zusatzstoffen polymerisiert und anschließend ohne

vorherige Isolierung der Reaktionsprodukte gegebenenfalls in Gegenwart von Zusatzstoffen bei Temperaturen bis zu 250 °C, vorzugsweise bis zu 150 °C, in wässrigem Medium mit Carbonylverbindungen kondensiert,

oder daß man

— modifizierte Azulminsäuren mit einem Gehalt von 0,5 bis 55 Gewichtsprozent an ionischen Gruppen der Formel

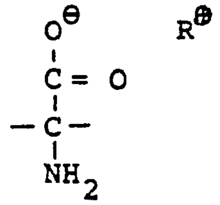

$(F_1)$

in welcher

R die oben angegebene Bedeutung hat,

und mit einem Gehalt von 0,5 bis 15 Gewichtsprozent an Gruppen der Formel

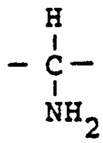

$(F_2)$

in wäßrigem Medium mit starken Basen wie Natriumhydroxid oder Kaliumhydroxid umsetzt, gegebenenfalls das Kation durch Behandlung mit Metallsalzen austauscht und anschließend in wässrigem Medium bei Temperaturen zwischen 10 °C und 250 °C, vorzugsweise zwischen 50 °C und 150 °C, mit Carbonylverbindungen kondensiert,

oder daß man

— modifizierte Azulminsäuren in wäßrigem Medium mit organischen oder anorganischen Säuren behandelt und anschließend gegebenenfalls in Gegenwart von Zusatzstoffen in wäßrigem Medium bei Temperaturen zwischen 10 °C und 250 °C, vorzugsweise zwischen 50 °C und 150 °C, mit Carbonylverbindungen kondensiert,

oder daß man

— nahezu fehlerstellenfreie Azulminsäuren in Anwesenheit von hydrolytisch abbaubaren Naturstoffen sowie in Gegenwart von Säure in wäßrigem Medium bei Temperaturen zwischen 10 °C und 250 °C, vorzugsweise zwischen 50 °C und 150 °C, mit Carbonylverbindungen kondensiert.

Als Carbonylverbindungen können bei diesen Verfahren Aldehyde und Ketone mit reaktiven Carbonylgruppen verwendet werden. Vorzugsweise verwendbar sind alle diejenigen Carbonylverbindungen, die auch bei der Durchführung des erfindungsgemäßen Verfahrens in Frage kommen. Besonders bevorzugt als Carbonylkomponente ist Formaldehyd.

Die Isolierung der Reaktionsprodukte erfolgt bei allen diesen Verfahren nach üblichen Methoden. Im allgemeinen verfährt man in der Weise, daß man nach beendeter Umsetzung das feste Reaktionsprodukt abfiltriert, wäscht und trocknet.

Von den durch Kondensation mit Carbonylverbindungen stabilisierten Azulminsäuren sind bei dem erfindungsgemäßen Verfahren bevorzugt partiell mit Carbonylverbindungen, insbesondere Formaldehyd, stabilisierte Azulminsäuren verwendbar, worunter solche fehlerstellenhaltigen Azulminsäuren zu verstehen sind, in denen nur ein Teil der zur Verfügung stehenden reaktiven Gruppen mit Carbonylverbindungen, insbesondere Formaldehyd, umgesetzt wurde.

Unter « durch Kondensation mit Carbonylverbindungen und Aminoplastbildnern bzw. deren niedermolekularen Kondensationsprodukten stabilisierten Azulminsäuren » sind solche Azulminsäuren zu verstehen, die einen Gehalt von 0,5 bis 55 Gewichtsprozent an ionischen Gruppen der Formel

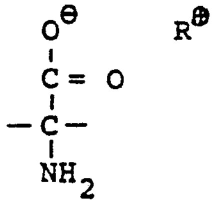

$(F_1)$

in welcher

R die oben angegebene Bedeutung hat,

und einen Gehalt von 0,5 bis 15 Gewichtsprozent an durch Decarboxylierungsreaktionen entstandenen

Gruppen der Formel

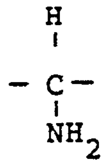

(F2)

aufweisen und durch Umsetzung mit Carbonylverbindungen und Aminoplastbildnern bzw. deren niedermolekularen Kondensationsprodukten gegenüber Blausäureabspaltung stabilisiert sind.

Vorzugsweise verwendbar bei dem erfindungsgemäßen Verfahren sind solche stabilisierten Azulminsäuren,

— in denen R diejenigen Bedeutungen hat, die schon im Zusammenhang mit der Beschreibung der modifizierten Azulminsäuren vorzugsweise für R aufgeführt wurden und

— bei denen als Carbonylkomponenten zur Stabilisierung einfache Aldehyde, wie Form-aldehyd, und einfache Aminoplastbildner, insbesondere Harnstoff, bzw. niedermolekulare Additionsprodukte aus solchen Carbonylverbindungen und Aminoplastbildnern, z.B. Monomethylolharnstoff oder Dimethylolharnstoff, bzw. ihre oligomeren Kondensationsprodukte benutzt wurden.

Die genannten durch Kondensation mit Carbonylverbindungen und Aminoplastbildnern bzw. deren niedermolekularen Additions- bzw. Kondensationsprodukten stabilisierten Azulminsäuren sind bisher noch nicht bekannt. Sie lassen sich jedoch in einfacher Weise nach mehreren Verfahren herstellen. So erhält man derartige durch Kondensation mit Carbonylverbindungen und Aminoplastbildnern bzw. deren niedermolekularen Additions- bzw. Kondensationsprodukten stabilisierten Azulminsäuren dadurch, daß man

— modifizierte Azulminsäuren mit einem Gehalt von 0,5 bis 55 Gewichtsprozent an ionischen Gruppen der Formel

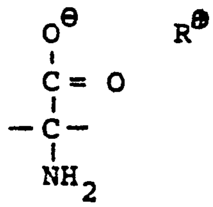

(F1)

in welcher

R die oben angegebenen Bedeutung hat,

und mit einem Gehalt von 0,5 bis 15 Gewichtsprozent an Gruppen der Formel

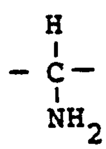

(F2)

in wäßrigem Medium bei Temperaturen zwischen 0 °C und 200 °C, vorzugsweise zwischen 10 °C und 150 °C, gegebenenfalls in Gegenwart von Katalysatoren sowie gegebenenfalls in Gegenwart von Kettenabbrechern mit Aminoplastbildnern und Carbonylverbindungen bzw. deren frisch zubereiteten Additions- bzw. Kondensationsprodukten umsetzt,

oder daß man

— Säureadditions-Salze bzw. Komplex-Verbindungen von modifizierten Azulminsäuren mit einem Gehalt von 0,5 bis 55 Gewichtsprozent an ionischen Gruppen der Formel

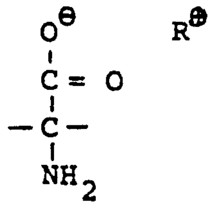

(F1)

in welcher

R die oben angegebene Bedeutung hat,

und mit einem Gehalt von 0,5 bis 15 Gewichtsprozent an Gruppen der Formel

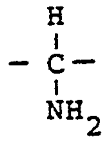

($F_2$)

in wäßrigem Medium bei Temperaturen zwischen 0 °C und 200 °C, vorzugsweise zwischen 10 °C und 150 °C gegebenenfalls in Gegenwart von Kettenabbrechern sowie gegebenenfalls in Gegenwart von Katalysatoren mit Aminoplastbildnern und Carbonylverbindungenen bzw. deren frisch zubereiteten Additions- bzw. Kondensationsprodukten umsetzt,
oder daß man

— nahezu fehlerstellenfreie Azulminsäuren in wäßrigem Medium bei Temperaturen zwischen 0 °C und 200 °C, vorzugsweise zwischen 10 °C und 150 °C gegebenenfalls in Gegenwart von Katalysatoren mit Aminoplastbildnern und Carbonylverbindungen bzw. deren frisch zubereiteten Additions- bzw. Kondensationsprodukten umsetzt,
oder daß man

— Blausäure mit Hilfe basischer Katalysatoren, wie Natriumcyanat, unter hydrolysierenden Bedingungen in wäßrigem Medium gegebenenfalls in Gegenwart von Zusatzstoffen bei Temperaturen zwischen 0 °C und 100 °C, vorzugsweise zwischen 20 °C und 95 °C, polymerisiert und anschließend ohne vorherige Isolierung der Reaktionsprodukte gegebenenfalls in Gegenwart von Zusatzstoffen und gegebenenfalls in Gegenwart von Katalysatoren sowie gegebenenfalls in Gegenwart von Kettenabbrechern mit Aminoplastbildnern und Carbonylverbindungen bzw. deren frisch zubereiteten Additions- bzw. Kondensationsprodukten bei Temperaturen bis zu 200 °C, vorzugsweise bis zu 150 °C, in wäßrigem Medium kondensiert,
oder daß man

— Blausäure mit Hilfe basischer Katalysatoren, wie Natriumcyanat, in Gegenwart von Aminoplastbildnern unter hydrolysierenden Bedingungen in wäßrigem Medium gegebenenfalls in Gegenwart von Zusatzstoffen bei Temperaturen zwischen 0 °C und 100 °C, vorzugsweise zwischen 20 °C und 95 °C, polymerisiert und anschließend ohne vorherige Isolierung der Reaktionsprodukte gegebenenfalls in Gegenwart von Zusatzstoffen und gegebenenfalls in Gegenwart von Katalysatoren sowie gegebenenfalls in Gegenwart von Kettenabbrechern mit Carbonylverbindungen und Aminoplastbildnern bzw. deren frisch zubereiteten Additions- bzw. Kondensationsprodukten in wäßrigem Medium bei Temperaturen bis zu 200 °C, vorzugsweise bis zu 150 °C, kondensiert,
oder daß man

— modifizierte Azulminsäuren mit einem Gehalt von 0,5 bis 55 Gewichtsprozent an ionischen Gruppen der Formel

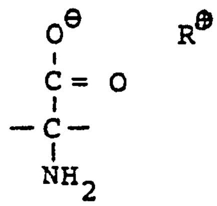

($F_1$)

in welcher

R die oben angegebene Bedeutung hat,

und mit einem Gehalt von 0,5 bis 15 Gewichtsprozent an Gruppen der Formel

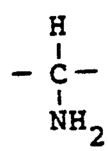

($F_2$)

in wäßrigem Medium mit starken Basen, wie Natriumhydroxid oder Kaliumhydroxid, bei Temperaturen zwischen 50 °C und 120 °C, vorzugsweise zwischen 60 °C und 110 °C, umsetzt, gegebenenfalls das Kation durch Behandlung mit Metallsalzen austauscht und anschließend in wäßrigem Medium bei Temperaturen zwischen 0 °C und 200 °C, vorzugsweise zwischen 10 °C und 150 °C, gegebenenfalls in Gegenwart von Katalysatoren sowie gegebenenfalls in Gegenwart von Kettenabbrechern mit Aminoplastbildnern und Carbonylverbindungen bzw. deren frisch zubereiteten Additions- bzw. Kondensationsprodukten umsetzt,
oder daß man

— modifizierte Azulminsäuren in wäßrigem Medium bei Temperaturen zwischen 0 °C und 200 °C, vorzugsweise zwischen 20 °C und 120 °C, mit organischen oder anorganischen Säuren behandelt und

anschließend in wäßrigem Medium bei Temperaturen zwischen 0 °C und 200 °C, vorzugsweise zwischen 10 °C und 150 °C, gegebenenfalls in Gegenwart von Katalysatoren sowie gegebenenfalls in Gegenwart von Kettenabbrechern mit Aminoplastbildnern und Carbonylverbindungen bzw. deren frisch zubereiteten Additions- bzw. Kondensationsprodukten umsetzt,

oder daß man

— nahezu fehlerstellenfreie Azulminsäuren in Anwesenheit von hydrolytisch abbaubaren Naturstoffen in Gegenwart von Säure in wäßrigem Medium, gegebenenfalls in Gegenwart von Katalysatoren sowie gegebenenfalls in Gegenwart von Kettenabbrechern mit Aminoplastbildnern und Carbonylverbindungen bzw. deren frisch zubereiteten Additions- bzw. Kondensationsprodukten bei Temperaturen zwischen 0 °C und 200 °C, vorzugsweise zwischen 10 °C und 150 °C, umsetzt,

oder daß man

— partiell oder vollständig mit Carbonylverbindungen stabilisierte Azulminsäuren in wäßrigem Medium bei Temperaturen zwischen 0 °C und 200 °C, vorzugsweise zwischen 10 °C und 150 °C, gegebenenfalls in Gegenwart von Katalysatoren sowie gegebenenfalls in Gegenwart von Kettenabbrechern mit Aminoplastbildnern und Carbonylverbindungen bzw. deren frisch zubereiteten Additions- bzw. Kondensationsprodukten umsetzt,

oder daß man

— thermisch bei Temperaturen bis zu 550 °C vorbehandelte Blausäure-Polymerisate in wäßrigem Medium gegebenenfalls in Gegenwart von Zusatzstoffen und gegebenenfalls in Gegenwart von Katalysatoren sowie gegebenenfalls in Gegenwart von Kettenabbrechern mit Aminoplastbildnern und Carbonylverbindungen bzw. deren frisch zubereiteten Additions- bzw. Kondensationsprodukten bei Temperaturen zwischen 0 °C und 200 °C, vorzugsweise zwischen 10 °C und 150 °C, umsetzt,

oder daß man

— an der Oberfläche chemisch modifizierte Azulminsäuren in wäßrigem Medium bei Temperaturen zwischen 0 °C und 200 °C, vorzugsweise zwischen 10 °C und 150 °C, gegebenenfalls in Gegenwart von Katalysatoren sowie gegebenenfalls in Gegenwart von Kettenabbrechern mit Aminoplastbildnern und Carbonylverbindungen bzw. deren frisch bereiteten Additions- bzw. Kondensationsprodukten umsetzt.

Als Carbonylverbindungen können bei diesen Verfahren Aldehyde und Ketone mit reaktiven Carbonylgruppen verwendet werden. Vorzugsweise verwendbar sind alle diejenigen Carbonylverbindungen, die auch bei der Durchführung des erfindungsgemäßen Verfahrens in Frage kommen. Besonders bevorzugt als Carbonylkomponente ist Formaldehyd.

Als Aminoplastbildner können bei diesen Verfahren alle üblichen Aminoplastbildner verwendet werden. Vorzugsweise verwendbar sind alle diejenigen Aminoplastbildner, die auch bei der Durchführung des erfindungsgemäßen Verfahrens in Frage kommen. Besonders bevorzugt als Aminoplastbildner ist Harnstoff.

Als niedermolekulare Additions- bzw. Kondensationsprodukte aus Aminoplastbildnern und Carbonylverbindungen können bei diesen Verfahren vorzugsweise N-Alkylolverbindungen, insbesondere N-Methylolverbindungen, verwendet werden, wobei Monomethylol-harnstoff und Dimethylolharnstoff beispielhaft genannt seien.

Als Katalysatoren können bei diesen Verfahren alle üblichen Kondensationskatalysatoren verwendet werden. Hierzu gehören vorzugsweise alle diejenigen Säuren, Basen und Salze, die auch bei dem erfindungsgemäßen Verfahren vorzugsweise als Kondensationskatalysatoren in Frage kommen (vgl. Seite 60).

Als Kettenabbrecher können bei diesen Verfahren alle übliche, für Kettenabbruch-Reaktionen geeigneten monofunktionellen Verbindungen eingesetzt werden. Vorzugsweise in Betracht kommen hierbei alle diejenigen Kettenabbrecher, die auch bei dem erfindungsgemäßen Verfahren vorzugsweise als Kettenabbrecher in Frage kommen (vgl. Seite 61).

Die Isolierung der Reaktionsprodukte erfolgt bei allen diesen Verfahren nach üblichen Methoden. Im allgemeinen verfährt man in der Weise, daß man nach beendetet Umsetzung das feste Reaktionsprodukt abfiltriert, wäscht und trocknet.

Von den durch Kondensation mit Aminoplastbildnern und Carbonylverbindungen bzw. deren niedermolekularen frisch zubereiteten Additions- bzw. Kondensationsprodukten stabilisierten Azulminsäuren sind bei dem erfindungsgemäßen Verfahren bevorzugt partiell stabilisierte Azulminsäuren verwendbar, worunter solche fehlerstellenhaltigen Azulminsäuren zu verstehen sind, in denen nur ein Teil der zu Verfügung stehenden reaktiven Gruppen mit Aminoplastbildnern, insbesondere Harnstoff, und Carbonylverbindungen, insbesondere Formaldehyd, bzw. mit deren niedermolekularen Additions- bzw. Kondensationsprodukten umgesetzt wurde.

Die Metallsalz-Komplexe der vorgenannten modifizierten und stabilisierten Azulminsäuren sind bisher noch nicht bekannt. Sie lassen sich jedoch herstellen, indem man die vorgenannten modifizierten oder stabilisierten Azulminsäuren in wässrigen Medium bei Temperaturen zwischen 20 °C und 120 °C, vorzugsweise bei 50 °C bis 110 °C, mit den zu komplexierenden Stoffen verrührt. Die Aufarbeitung erfolgt nach üblichen Methoden. Im Allgemeinen isoliert man die Reaktionsprodukte durch Filtration.

Als Phenoplastbildner können bei dem erfindungsgemäßen Verfahren alle üblichen zur Phenoplastbildung befähigten Verbindungen verwendet werden. Hierzu gehören vorzugsweise Phenole und davon abgeleitete Verbindungen. Beispielhaft genannt seien Phenole, Kresole, Bisphenol A, Nitrophenol,

Brenzkatechin, Hydrochinon und Naphtholsulfonsäure.

Als Katalysatoren können bei dem erfindungsgemäßen Verfahren alle üblichen Kondensationskatalysatoren verwendet werden. Hierzu gehören Säuren, wie Salzsäure, Schwefelsäure, Salpetersäure, Phosphorsäure, Phosphorige Säure, andere vom Phosphor abgeleitete Säuren, Ameisensäure, Essigsäure, Thioessigsäure, Maleinsäure und Oxalsäure, ferner Basen wie Natriumhydroxid, Kaliumhydroxid, Calciumhydroxid, Bariumhydroxid, Bleihydroxid, Zinkoxid, Magnesiumoxid und andere Metalloxide und deren Hydrate, weiterhin Salze, wie Phosphate, z.B. primäres oder sekundäres Kaliumhydrogenphosphat, Ammoniumsulfat, Kupfer-, Zink-, Zinn-(II)-, Cadmium- und Magnesium-Salze verschiedenster organischer Säuren, außerdem zahlreiche organische Säureanhydride sowie säureabspaltenden Verbindungen wie Ammoniumchlorid, Trimethylammoniumformiat, Chloralhydrat, darüber hinaus Aminsalze der Ameisensäure und anderer organischer Carbonsäuren, Maleinsäurehalbester, tertiäre Aminsalze und tertiäre Amine, Dibenzoylperoxid, Kohlensäure, N-Carbaminsäuren, Glykolchlorhydrin, Glycerinchlorhydrin und Epichlorhydrin.

Bevorzugt verwendbare Katalysatoren sind Säuren wie Phosphorsäure, Phosphorige Säure, Salpetersäure, Salzsäure, Schwefelsäure, Ameisensäure, Oxalsäure und Maleinsäure, ferner Basen wie Natriumhydroxid, Kaliumhydroxid, Calciumhydroxid, Bariumhydroxid, Bleihydroxid, Benzyldimethylamin und Triäthylamin.

Verwendet man Phosphorsäure oder Schwefelsäure als Kondensationskatalysatoren, so können die genannten Säuren oft mit Vorteil durch Fällung mit Calcium- oder für den Fall der Phosphorsäure mit Eisen- oder Aluminiumionen auf den Verfahrensprodukten quantitativ niedergeschlagen werden, so daß Auswaschoperationen der Verfahrensprodukte und Abwasserbelastungen entfallen.

Als Kettenabbrecher können bei dem erfindungsgemäßen Verfahren alle üblichen, für Kettenabbruch-Reaktionen geeigneten monofunktionellen Verbindungen eingesetzt werden. Vorzugsweise in Betracht kommende monofunktionelle Kettenabbrecher sind Lactame, wie ε-Caprolactam, Valerolactam, Butyrolactam und die entsprechenden Thiolactame, ferner Formamid und Acetamid, weiterhin Alkohole, wie Methanol, Äthanol, Propanol, Butanol, Allylalkohol, Isopropanol, Oleylalkohol und Benzylalkohol, welche die wachsenden Aminoplast-Segmente durch Verätherungsreaktionen stoppen. — Vorzugsweise verwendbare Kettenabbrecher sind auch solche Verbindungen, wie sie in der Deutschen Offenlegungsschrift 2 324 134 auf den Seiten 13 und 14 beschrieben werden. In einer besonderen Ausführungsform des erfindungsgemäßen Verfahrens können als Kettenabbrecher auch N-Methylolcaprolactam, N-Methylolvalerolactam, N-Methylolbutyrolactam und N-Methylolazalactame, wie z.B. basische Methylolverbindungen von Azalactamen der Konstitution

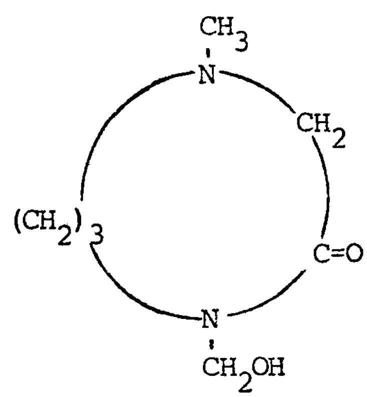

fungieren. Die letzgenannten Stoffe sind bisher noch nicht bekannt. Sie lassen sich jedoch durch Methylolierung mit Formaldehyd nach üblichen Methoden aus den entsprechenden Azalactamen herstellen. Die benötigen Azalactame sind bekannt (vgl. DE-OS 2 035 800).

Als Zusatzstoffe können bei der Durchführung des erfindungsgemäßen Verfahrens organische Naturstoffe und daraus gewonnene Produkte, anorganische Naturstoffe und daraus gewonnene Produkte, synthetische organische Produkte, synthetische anorganische Produkte und/oder Mischprodukte aus organischen und anorganischen Produkten verwendet werden.

Vorzugsweise in Frage kommende organische Naturstoffe und daraus gewonnene Produkte sind hierbei Holzpulver, Ligninpulver, Ligninsulfonsäuren, ammonifizierte Ligninsulfonsäuren, Humus, Huminsäuren, ammonifizierte Huminsäuren, Torf, Proteine und deren Abbauprodukte, z.B. Hydrolyseprodukte von Hefen, Algenmaterial (Alginate), Polypeptiden, wie Wolle und Gelatine, Fischmehl und Knochenmehl, ferner Aminosäuren, Oligopolypeptide, Pektine, Monosaccharide, wie Glucose und Fructose, Disaccharide, wie Saccharose, Oligosaccharide, Polysaccharide, wie Stärke und Cellulose, weiterhin Hemicellulosen, homogenisierte Materialien pflanzlichen und tierischen Ursprungs,

Aktivkohlen sowie Aschen, die durch Partialoxidation, vollständige Oxidation oder Verbrennung organischer, durch Photosynthese gebildeter Stoffe oder üblicher Brennstoffe erhältlich sind, wobei Tannenasche, Ginsterasche, Asche von serbischen Fichten, Eichenasche, Birkenasche, Buchenasche, Weidenasche und Tabakblätter-Asche speziell genannt seien.

Als anorganische Naturstoffe und daraus gewonnene Produkte kommen vorzugsweise in Betracht Silikate, wie Aluminiumsilikate, Calciumsilikate, Magnesiumsilikate und Alkalisilikate, ferner Seesand und andere natürlich vorkommende Siliziumdioxide, Kieselsäuren, insbesondere disperse Kieselsäuren, Kieselgele, weiterhin Tonmineralien, Glimmer, Carbonate wie Calciumcarbonat, Phosphorit und Phosphate wie Calciumphosphat und Ammoniummagnesiumphosphat, Sulfate wie Calciumsulfat und Bariumsulfat, außerdem Oxide wie Zirkondioxid, Nickeloxid, Palladiumoxid, Bariumoxid, disperse Antimonoxide und Aluminiumoxide, wie Bauxit, Aluminiumoxidhydrat, darüber hinaus Flugaschen und Ruß-Sorten verschiedenster Art.

Als synthetische organische Produkte kommen vorzugsweise in Frage Aminoplastkondensate, insbesondere solche aus Harnstoff, Dicyandiamid, Melamin oder Oxamid und Aldehyden, wie Formaldehyd, Acetaldehyd, Isobutyraldehyd, Hydroxypivalinaldehyd, Crotonaldehyd, Hydroxyacetaldehyd, Furfurol, Hydroxymethylfurfurol, Glyoxal un Glucose, wobei speziell genannt seien Kondensationsprodukte aus Harnstoff und Formaldehyd, Harnstoff und Glyoxal, Harnstoff und Acetaldehyd, Harnstoff und Isobutyraldehyd, Harnstoff und Crotonaldehyd, Harnstoff und Hydroxypivalinaldehyd sowie das 2-Oxo-4-methyl-6-ureido-hexahydropyrimidin, welches ein bekanntes Kondensationsprodukt aus 1 Mol Crotonaldehyd und 2 Mol Harnstoff ist, das aus intermediär anfallendem Crotonyliden-diharnstoff unter Absättigung der Doppelbindung entsteht und dem die Konstitution

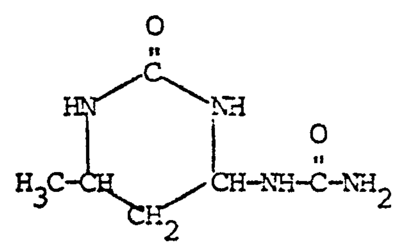

zukommt. Ferner genannt seien Polyalkylidenharnstoffe, wie Polymethylenharnstoffe, außerdem Polymethylenthioharnstoffe, hochvernetzte Aminoplastkondensate, Harnstoff-Hydrazodicarbonamid-Formaldehyd-Kondensate, Dicyandiamid-Kondensate, Oxamid-Kondensate und hochmolekulare Polyammoniumpolyphosphate der Konstitution

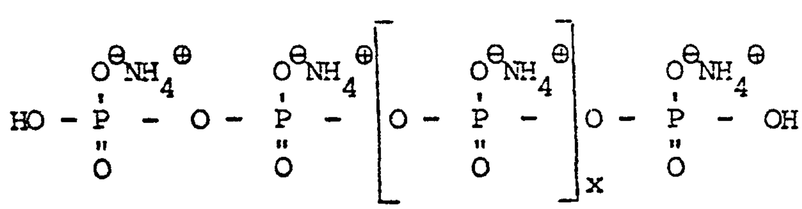

x = 10 – 200.

Weitherhin kommen als synthetische organische Produkte vorzugsweise in Betracht Kunststoffe, wie Polyamidpulver, Polyurethanpulver und Polycarbodiimide, ferner polymere Chinone, Additions- zw. Kondensationsprodukte aus Chinonen, insbesondere Benzochinon, mit Aminen oder Ammoniak, außerdem mit Aldehyden, insbesondere Formaldehyd, vernetzte Gelatine, synthetische Bodenverbesserungsmittel, wie z.B. das als Hygromull bekannte Produkt (= Harnstoff-Formaldehyd-Harzflocken), darüber hinaus synthetische Zucker, wie z.B. aus Formaldehyd hergestellte Formose-Zuckergemische, ferner schwerlösliche Rohrzucker-Komplexe, wie der Saccharose-Calciumoxid-Komplex der Zusammensetzung 1 Mol Saccharose. 3 Mol Calciumoxid, und schließlich organische Ammoniumsalze, wie Ammoniumcarbaminat, und andere organische Stickstoffverbindungen wie Hexamethylentetramin und Hexahydrotriazine.

Als synthetische anorganische Produkte, die vorzugsweise in Frage kommen, seien genannt Düngemittel, wie Superphosphat, Thomasschlacke, Rhenaniaphosphat, Phosphorit, Kalkstickstoff, Kalkammonsalpeter, Leunasalpeter, Kaliumphosphate, Kaliumnitrat und Ammoniumnitrat, ferner Pigmente, wie Eisenoxide und Titandioxide, außerdem Metalloxide und Metallhydroxide, wie Calciumoxid, Calciumhydroxid, Bleihydroxid, Wismuthydroxid, Manganhydroxid und Magnesiumhydroxid, wobei in situ hergestellte Hydroxide besonders bevorzugt sind, außerdem Schwefel, schwerlösliche Metall-Sulfate, -Carbonate, -Phosphate und Silikate, Heteropolysäuren des Wolframs, Vanadins und Molydäns, weiterhin synthetische Kieselsäuren, insbesondere in situ hergestellte Kieselsäure und deren Salze, außerdem Wasserglas, Salze wie Cobaltmolybat, Ammoniumcarbonat und Calciumcarbonat, und darüber hinaus Katalysatoren, insbesondere Schwermetallkatalysatoren, der verschiedensten Art.

Als Mischprodukte aus anorganischen und organischen Produkten kommen vorzugsweise neutrale, basische oder saure Böden, natürliche Bodenverbesserungsmittel und biologische aktive Gartenerde in Betracht.

Im übrigen können bei dem erfindungsgemäßen Verfahren vor der Durchführung der Kondensation der Biomassen mit Carbonylverbindungen und Aminoplastbildnern auch Phenoplastbildner zugesetzt werden. Unter Phenoplastbildnern sind in diesem Zusammenhang Phenole und davon abgeleitete Verbindungen zu verstehen. Beispielhaft genannt seien Phenole, Kresole, Bisphenol A, Nitrophenol, Benzkatechin, Hydrochinon und Naphtholsulfonsäure. — Bei dieser Art der Durchführung des erfindungsgemäßen Verfahrens werden Aminoplast-Phenoplast-Segmente in die Kondensationsprodukte eingeführt. Derartige Mischkondensate erhöhen oft die Geschwindigkeit der Flockungsreaktion und können Vorteile bei der Verbesserung der Filtrierfähigkeit und der Verminderung der Klebrigkeit der Verfahrensprodukte erbringen.

Außerdem kann es bei der Durchführung des erfindungsgemäßen Verfahrens von Vorteil sein, den Biomassen vor der Durchführung der Kondensationen Hydroxyalkanphosphonsäureester bzw. Hydroxyalkanphosphonsäuren, insbesondere Hydroxymethylphosphonsäureester oder Hydroxymethylphosphonsäure, zuzugeben, da diese Stoffe über ihre Hydroxymethylgruppe mit Aminoplastbildnern Mischkondensationen eingehen und gleichzeitig katalytisch wirksam sind.

Auch die Zugabe von 10-20 Gew.-% an Mono- und Polynitrilen zu den Biomassen, z.B. von Acrylnitril und insbesondere Hydroxyacetonitril, kann vor der Durchführung der Kondensationen von Vorteil sein, da z.B. Hydroxyacetonitril in Gegenwart von Formaldehyd und Aminoplastbildnern, wie Harnstoff, Mischkondensationen eingeht.

Bei der Durchführung des erfindungsgemäßen Verfahrens kann auch eine Nachbehandlung der Verfahrensprodukte erfolgen, indem man sie gegebenenfalls in Gegenwart von Verdünnungsmitteln, wie z.B. wasserfreien organischen Lösungsmitteln bei Temperaturen zwischen 0 °C und 150 °C, vorzugsweise zwischen 10 °C und 120 °C mit den verschiedensten Reagenzien behandelt bzw. den verschiedensten Reaktionen unterwirft. Hierbei treten in geringem Umfang an der Oberfläche der Verfahrensprodukte chemische Reaktionen auf, so daß man an der Oberfläche chemisch modifizierte Produkte erhält.

Zur chemischen Modifizierung der Oberfläche der nach dem erfindungsgemäßen Verfahren herstellbaren Mischkondensate kommen vorzugsweise in Betracht

— die Behandlung mit Harnstoffschmelzen (= Isocyansäurelieferent);

— die Behandlung mit mit Acylierungsmitteln, wie Ameisensäure, Essigsäureanhydrid, Buttersäureanhydrid, gemischten Säureanhydriden aus Essigsäure und Ölsäure, vorzugsweise in Gegenwart von Natrium- oder Kaliumacetat;

— die Behandlung mit cyclischen Säure-Anhydriden, wie Maleinsäureanhydrid, Phthalsäureanhydrid oder Hexahydrophthalsäureanhydrid;

— die Behandlung mit Schmelzen von Dicarbonsäuren, wie Adipinsäure, Phthalsäure, Hexahydrophthalsäure oder Trimelithsäure;

— die Behandlung mit anorganischen Säurechloriden, wie Chlorcyan, Phosgen, Thionylchlorid, Schwefelchloriden, Phosphoroxychlorid, Phosphorpentachlorid, Siliziumtetrachlorid, Antimontrichlorid oder Titantetrachlorid;

— die Behandlung mit organischen Säurechloriden, wie Acetylchlorid, Benzoylchlorid, Chlorameisensäureestern der Formel

$$R'\text{-}O\text{-}\underset{\underset{O}{\|}}{C}\text{-}Cl$$

in welcher

R' für Alkyl mit 1 bis 8 Kohlenstoffatomen steht, bifunktionellen Chlorameisensäureestern der Formel

$$Cl\text{-}\underset{\underset{O}{\|}}{C}\text{-}O\text{-}R''\text{-}O\text{-}\underset{\underset{O}{\|}}{C}\text{-}Cl$$

in welcher

R'' für Alkylen mit 2 bis 8 Kohlenstoffatomen steht, Benzolsulfonsäurechloriden, Phosphorsäureesterchloriden, Chlormethansulfochlorid oder Cyanursäurechlorid;

— die Behandlung mit Alkylierungsmitteln, wie Dimethylsulfat, Methyljodid oder Methylbromid,

— durch Behandlung mit Dichloräthan, Glykolchlorhydrin, Chloressigsäureäthylester, Dichloressigsäureäthylester Chloracetaldehyd-di-äthylacetal, Allylchlorid, Benzylchlorid, Trichlormethylisocyaniddichlorid oder anderen Isocyaniddichloriden bzw. Alkylierungsreagentien ;

— die Behandlung mit ε-Caprolactam, ε-Caprolacton, Hydroxypivalinsäurelacton, cyclischen 6-gliedrigen oder 8-gliedrigen Siloxanen, Azalactamen wie sie aus der DT-OS 2 035 800 bekannt sind, Glykolcarbonat, Äthylenoxid, Propylenoxid, Butylenoxid, Styroloxid, Epichlorhydrin, Butyrolacton, Valerolacton, Oxazolidinen, Oxazolinen, Imidazolidinen, Isatosäureanhydrid oder Leuchsschen Anhydriden aus Aminosäuren und Phosgen ;

— die Behandlung mit Acrylnitril oder anderen Vinylmonomeren, wie Acrylsäure, Methacrylsäure bzw. deren Methyl-, Äthyl-, β-hydroxyäthyl- oder Propylestern ;

— die Behandlung mit Alkoholen oder bifunktionellen Alkoholen, wie Äthylenglykol, Hexandiol oder Octandiol, unter den Bedingungen der Pinner-Reaktion (vorzugsweise in wasserfreier HCl und Alkoholen) ;

— die Behandlung mit Mono- oder Biscyanaten bzw. Mono- oder Biscyanamiden ;

— die Behandlung mit Hydroxy-alkanphosphorsäureestern bzw. den zugrunde liegenden Säuren, insbesondere mit Hydroxymethyl-phosphonsäureestern bzw. der freien Hydroxymethyl-phosphonsäure ;

— die Behandlung mit Chlormethylalkoxysilanen, z.B. solchen der Formeln

$$Cl-CH_2-Si(-OC_2H_5)_3, \quad Cl-CH_2-\underset{CH_3}{Si}(-OC_2H_5)_2,$$

$$Cl-CH_2-\overset{CH_3}{\underset{CH_3}{Si}}(-OC_2H_5)$$ bzw.

$$Cl-CH_3-\overset{CH_3}{\underset{CH_3}{Si}}-O-\overset{CH_3}{\underset{CH_3}{Si}}-CH_2-Cl$$ und $$Cl-CH_2-CH_2-CH_2-Si-(OC_2H_5)_3;$$

— Die Behandlung mit den verschiedensten Mono- oder Polynitrilen, bevorzugt Hydroxymethylnitril, unter den Bedingungen der durch Hydroxylgruppen katalysierten Thorpe-Reaktion.

Als Nachbehandlungsreagenzien seien außerdem erwähnt : Natriumhydroxid, Kaliumhydroxid, Calciumhydroxid, Natriumsulfid, Rongalit, Ammoniumpolysulfide, Diäthylphosphit und Dimethylphosphit.

Bei diesen Nachbehandlungsreaktionen können auch die verschiedensten Mischpolymerisationen oder Polymerisationen von Vinylmonomeren durchgeführt werden, wobei die Biomassen-Mischkondensate durch die entstehend Polymeren umhüllt bzw. mikroverkapselt werden. Hierbei können selbstverständlich die « Hüllmaterialien » auch in einem großen Überschuß eingesetzt werden.

Bei der Durchführung des erfindungsgemäßen Verfahrens arbeitet man bevorzugt in wäßrigem Medium oder in wäßrigalkoholischem Medium. Dabei können auch zusätzliche inerte organische Lösungsmittel mitverwendet werden ; letztere dienen zur azeotropen Entfernung des Wassers nach beendeter Umsetzung. Vorzugsweise kommt jedoch Wasser ohne zusätzliche organische Lösungsmittel als Reaktionsmedium in Betracht.

Die Reaktionstemperaturen können im Falle des erfindungsgemäßen Verfahrens innerhalb eines größeren Bereiches variiert werden. Im allgemeinen arbeitet man bei Temperaturen zwischen 0 °C und 200 °C, vorzugsweise zwischen 10 °C und 150 °C. Es ist jedoch auch möglich, die erfindungsgemäße Mischkondensation im Verlaufe des Trocknungsprozesses, zum Beispiel bei der Sprühtrocknung, bei Temperaturen bis zu 250 °C zu Ende zu führen. In vielen Fällen kann das erfindungsgemäße Verfahren auch bevorzugt bei Raumtemperatur durchgeführt werden. Eventuell noch verbleibende pathogene Krankheitskeime können dabei in der Trocknungsphase durch Sterilisierung abgetötet werden.

Die Umsetzung nach dem erfindungsgemäßen Verfahren wird im allgemeinen unter Normaldruck ausgeführt. Es ist jedoch auch möglich, unter erhöhtem oder vermindertem Druck zu arbeiten. Zum Beispiel kann das erfindungsgemäße Verfahren unter erhöhtem Druck zum Beispiel bei Temperaturen zwischen 120 °C und 160 °C durchgeführt werden wobei neben Sterilisation der Produkte auch ein gezielter Abbau von Proteinen, Ribonucleinsäuren, Desoxyribonucleinsäuren, Nucleoproteiden und/oder anderer Zellinhaltsstoffe erfolgen kann.

Bei der Durchführung des erfindungsgemäßen Verfahrens setzt man auf 1 kg Biomasse mit einem

Feststoffgehalt von 1 bis 16 Gewichtsprozent, etwa 0,1 bis 6 Mol, vorzugsweise 0,2 bis 5 Mol, an Carbonylverbindungen, Thiocarbonylverbindungen und/oder an niedermolekularen, nicht kondensierten N-Alkylolverbindungen, die mit Carbonylverbindungen im Dissoziationsgleichgewicht stehen, sowie 0,1 bis 6 Mol, vorzugsweise 0,2 bis 5 Mol, an Aminoplastbildnern bzw. Phenoplastbildnern ein, ferner setzt man gegebenenfalls Kettenabbrecher, gegebenenfalls Katalysatoren und außerdem gegebenenfalls eine solche Menge an Zusatzstoffen ein, daß deren Anteil im Endprodukt zwischen 1 und 95 Gewichtsprozent vorzugsweise zwischen 5 und 90 Gewichtsprozent liegt.

Katalysatoren werden im allgemeinen in Mengen von 0,05 bis 10 Gew.-%, vorzugsweise von 0,1 bis 5 Gew.-%, bezogen auf die Gesamtmenge aller an der Polykondensation beteiligten Reaktionskomponenten, eingesetzt. In manchen Fällen können jedoch auch wesentlich höhere Katalysator-Konzentrationen zur Anwendung gelangen. So können zum Beispiel insbesondere dann, wenn die Kondensation unter Mitverwendung von Azulminsäuren durchgeführt wird, 0,4 bis 0,6 Mol an saurem Katalysator, vorzugsweise an Phosphorsäure oder Salpersäure, pro 100 g an Biomassen und Azulminsäuren eingesetzt werden. Hierbei entstehen Produkte, in denen die Katalysator-Säuren an basischen Gruppen der Mischkondensate fixiert sind.

Kettenabbrecher können in Mengen von 0,5 bis 60 Gew.-%, bezogen auf die Gesamtmenge der zur Aminoplastbildung befähigten Ausgangsverbindungen eingesetzt werden. Dienen N-Methylollactame bzw. N-Methylolazalactame als Kettenabbrecher, so liegen deren Konzentrationen in allgemeinen zwischen 0,5 und 20 Gew.-%, vorzugsweise zwischen 2 und 14 Gew.-%, bezogen auf die Gesamtmenge an Aminoplastbildnern und Carbonylverbindungen bzw. Thiocarbonylverbindungen.

Phenoplastbildner können in Mengen von 0,5 bis 100 Gew.-%, bezogen auf die Biomassen eingesetzt werden.

Die praktische Durchführung des erfindungsgemäßen Verfahrens erfolgt im allgemeinen in der Weise, daß man eine wässrige Biomassen-Dispersion gegebenenfalls in Gegenwart von Zusatzstoffen mit Carbonylverbindungen Thiocarbonylverbindungen und/oder Carbonylverbindungen, die mit niedermolekularen, nicht kondensierten N-Alkylolverbindungen im Dissoziationsgleichgewicht stehen, versetzt, gegebenenfalls einen Katalysator hinzufügt und die Kondensation einleitet, dann in der zweiten Reaktionsphase mit einer wässrigen Lösung von Aminoplastbildnern (auch teilalkylolierte bzw. teilmethylolierte Produkte) oder Phenoplastbildnern bzw. mit einer wäßrigen Lösung von noch löslichen Aminoplastbildnern (z.B. Polyalkylol- bzw. Polymethylol-Verbindungen) versetzt, gegebenenfalls Zusatzstoffe, Katalysatoren und Kettenabbrecher hinzufügt und die Kondensation vornimmt und gegebenenfalls anschließend noch Nachbehandlungsreaktionen vornimmt. Es ist jedoch auch möglich, wäßrige Dispersionen oder Lösungen von Carbonylverbindungen, Thiocarbonylverbindungen und/oder Carbonylverbindungen, die mit niedermolekularen, nicht kondensierten N-Alkylolverbindungen im Dissoziationsgleichgewicht stehen, vorzulegen, diese dann mit der wäßrigen Biomassen-Dispersion sowie gegebenenfalls mit Zusatzstoffen und/oder Katalysatoren zu versetzen und die Kondensation einzuleiten und dann in der zweiten Reaktionsphase so zu verfahren, wie es zuvor beschrieben ist.

Bevorzugt wird die erfindungsgemäße Umsetzung so durchgeführt, daß man eine wäßrige Lösung oder Dispersion der aminoplastbildenden bzw. phenoplastbildenden Verbindungen sauer eingestellt — etwa auf pH-Werte zwischen 1 und 4 bringt —, und diese Mischung auf die Biomassen einwirken läßt. Hierbei laufen die Reaktionen sehr rasch ab. Es können dabei bezogen auf ein $NH_2$- bzw. NH-Äquivalent aller Reaktionspartner 0,2 bis 1,4 Äquivalente an Carbonylverbindungen zur Einwirkung gelangen.

Bevorzugt ist auch die Verfahrensweise, bei der man Aminoplastbildner und Carbonylverbindung in Wasser oder niederen Alkoholen löst und diese reaktiven Lösungen, in denen sich Gleichgewichte zwischen Ausgangsverbindungen und N-Alkylolierungsprodukten bzw. N-Methylolierungs-produkten einstellen, bei Temperaturen zwischen 20 °C und 100 °C unter intensivem Rühren in die vorgelegten wäßrigen Biomassen-Dispersionen eintropft. Hierbei kann man auch so vorgehen, daß man Lösungen teilalkylolierter, insbesondere teilmethylolierter Verbindungen oder bereits teilweise vorkondensierte, noch lösliche Polyalkylol-, insbesondere Polymethylol-Verbindungen in einem Guß zu den dispergierten Biomassen gibt und die Kondensation durch Wärme, im basischen oder sauren Bereich unter Entfernung des Wassers bei Normaldruck oder unter vermindertem Druck zu Ende führt.

Oft ist es auch vorteilhaft, in einer ersten Phase zu einer wässrigen Biomassen-Dispersion die zur Aminoplastbildung befähigten Verbindungen zu geben, letztere dann im pH-Bereich zwischen 7,5 und 10 durch Zugabe der entsprechenden Carbonylverbindungen zu alkylolieren bzw. zu methylolieren und die bereits vorgebildeten N-Alkylolverbindungen bzw. N-Methylolverbindungen, — gegebenenfalls auch ihre Äther —, auf die Biomassen in Gegenwart unlöslicher Aminoplastbildner (Zusatzstoffe) zur Einwirkung zu bringen.

Die Isolierung der Reaktionsprodukte erfolgt bei dem erfindungsgemäßen Verfahren nach üblichen Methoden. Im allgemeinen geht man so vor, daß man nach beendeter Umsetzung das feste Reaktionsprodukt abfiltriert, wäscht und trocknet.

Häufig ist es von Vorteil, die erfindungsgemäßen Mischkondensate nach ihrer Herstellung mit Ammoniak, primären oder sekundären Aminen zu behandeln bzw. zu begasen, oder mit wäßrigen Ammoniak-Hydrazin-Lösungen, Hydrazinhydrat, Methylhydrazin oder wäßrigen Cyanid-Lösungen nachzuwaschen, um Spuren an Formaldehyd oder anderen Aldehyden oder Ketonen quantitativ zu entfernen. Bei der Einwirkung von Ammoniak bzw. primären Aminen werden zum Beispiel in den mit Formaldehyd

kondensierten Produkten noch enthaltene kleine Mengen an Formaldehyd in Hexamethylentetramin bzw. Hexahydrotriazine überführt. Oft empfiehlt es sich, eine Nachbehandlung mit 25 %iger wäßriger Ammoniak-Lösung vorzunehmen. — Alle diese Reinigungsoperationen können technisch z.B. durch Begasung im Wirbelbett oder gekoppelt mit der Sprühtrocknung der Produkte durchgeführt werden.

Sind in den erfindungsgemäßen Produkten nach ihrer Herstellung noch Katalysator-Reste vorhanden, so ist es häufig zweckmäßig, letztere auszuwaschen oder durch Umsetzung mit geeigneten Reagenzien chemisch zu desaktivieren. Wurden Säuren oder Basen als Katalysatoren verwendet, so empfiehlt es sich, diese durch Zugabe von Basen bzw. Säuren zu neutralisieren.

Sind in den erfindungsgemäßen Produkten nach ihrer Herstellung noch löslich organische Substanzen enthalten, so können diese durch Waschen der Produkte mit organischen Lösungsmitteln, wie Aceton, entfernt werden.

Werden bei dem erfindungsgemäßen Verfahren Biomassen eingesetzt, die Chlorophyll a und b enthalten, so werden diese Chlorophyll-Typen bei der Durchführung des Verfahrens z.B. bei der Kondensation unter Erzeugung von Polymethylenharnstoffen (bei pH = 2 und Temperaturen zwischen 60 und 70 °C) meist in kondensierter Form in den anfallenden Pulvern fixiert. Durch Extraktion mit Aceton können diese Chlorophyll-Typen teilweise isoliert werden.

Das erfindungsgemäße Verfahren kann in üblichen Kesseln, Schnecken und Reaktionsgefäßen auch in technischem Maßstab durchgeführt werden. Zweckmäßigerweise arbeitet man bei Biomassen, die pathogene Erreger enthalten, in geschlossenen Reaktoren, um die Verbreitung von Bakterien durch Aerosolbildung zu vermeiden.

Bei der Herstellung der erfindungsgemäßen Produkte kann man auch so vorgehen, daß man im Anschluß an die Mischkondensation auf den erfindungsgemäßen Verfahrensprodukten in Form ihrer in Wasser unslöslichen Dispersionen nahezu beliebige Mengen an Polymethylenharnstoffen, Polyalkylidenharnstoffen und schwer- oder unlöslichen Verbindungen, d.h. hochvernetzte Aminoplastkondensate erzeugt, die mit den Verfahrensprodukten infolge ihrer Unlöslichkeit nicht in covalente Verknüpfung zur Biomasse treten. Derartige Mischungen, in denen der nicht covalent gebundenen Anteil an Aminoplastkondensaten bzw. Phenoplastkondensaten praktisch beliebig sein kann, stellen insbesondere für den Fall der Beladung mit Polymethylenthioharnstoffen, vernetzten Polymethylenmelaminpulvern, Harnstoff-Hydrazodicarbonamid-Formaldehydkondensaten und Dicyandiamid-, Oxamid-Kondensaten außerordentlich interessante Flammschutzmittel für die verschiedensten Polyurethanschaumkunststoffe, bevorzugt für Polyurethanschaumstoffe dar.

Schwer- oder unlösliche Polymethylen- oder Polyalkylidenharnstoffe, die sich auf den erfindungsgemäßen Produkten niederschlagen lassen, sind zum Beispiel solche, die sich durch die nachstehend angegebenen idealisierten Konstitutionsformeln veranschaulichen lassen :

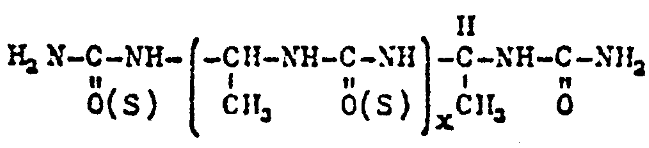

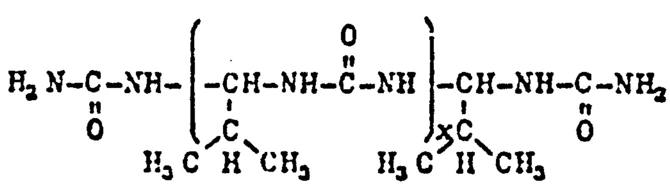

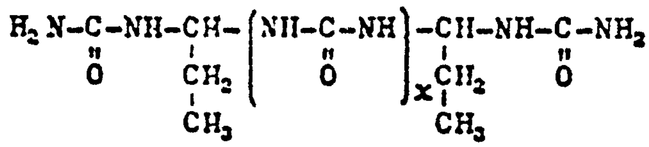

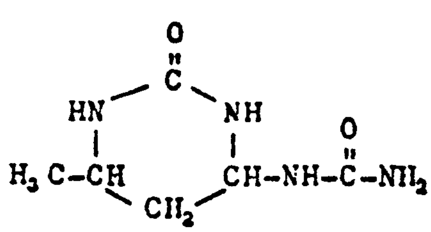

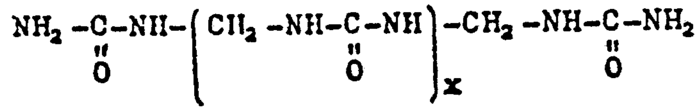

In diesen Formeln kann X jeweils für Zahlen von 0 bis 20 stehen.

Im übrigen lassen sich auf den erfindungsgemäßen Produkten auch andere schwer- oder unlösliche Füllstoffe, Flammschutzmittel, Pigmente, Farbstoffe usw. niederschlagen. So können z.B. im Überschuß vorhandene oder später zugefügte Metallsalze durch Zugabe von Basen in schwerlösliche Metallhydroxide oder Metalloxide überführt werden. Ferner lassen sich in vielen Fällen durch Zugabe entsprechender Säuren Metallsalze in Form von schwerlöslichen Metallphosphaten, Metallsulfaten, Metallcarbonaten oder Metallsilikaten ausfällen. Salze des Wolframs, Vanadins und Molybdäns können in Form von Heteropolysäuren ausgefällt werden. Polykieselsäuren lassen sich auf den erfindungsgemäßen Produkten erzeugen, indem man Natrium- oder Kaliumwasserglas-Lösungen zugibt und mit Kohlendioxid begast. Man kann auch Schwefel auf den unlöslichen Mischkondensaten niederschlagen, indem man Natrium- oder Ammoniumpolysulfide zugibt und stark ansäuert.

Von Interesse ist auch die nachträgliche Beladung der erfindungsgemäßen Mischkondensate mit dem schwerlöslichen Melaminphosphat, dem schwerlöslichen Harnstoffoxalat, oder Harnstoffnitrat, dem schwerlöslichen Ammoniummagnesiumphosphat. Auch die Zugabe von Tonerdehydraten, Aluminiumoxiden, Titandioxid und Calciumcarbonat, von Quarzmehl sowie die Zugabe linearer oder vernetzter Polymethylenharnstoffe, von pulvrigen Melamin-Formaldehyd-Kondensaten, Harnstoff-Hydrazodicarbonamid-Kondensaten und hochmolekularen Polyammoniumpolyphosphaten der Konstitution

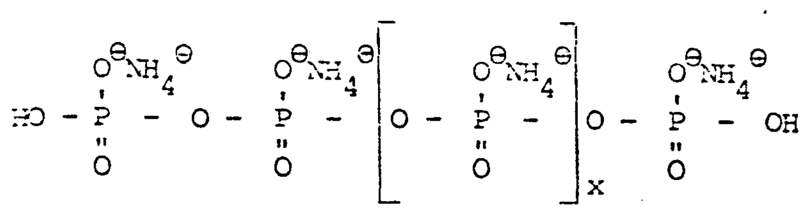

x = 10 – 200

ist von Bedeutung. Die dabei entstehenden Produkte eignen sich hervorragend als Flammschutzmittel für Kunststoffe, insbesondere für Polyurethankunststoffe.

Weiterhin kommen bei dem erfindungsgemäßen Verfahren insbesondere dann, wenn Azulminsäuren mitverwendet werden, als bevorzugte Zusatzstoffe auch Zucker, wie Rohrzucker und andere keine freien Aldehydgruppen aufweisenden Zucker, oder auch aus Formaldehyd hergestellte Formose-Zuckergemische in Frage. Diese verschiedensten Zuckerarten können in Kanälen und Poren der enthaltenen starren Azulminsäurekörper fixiert werden. Darüber hinaus können die verschiedenen Zucker auch in Form ihrer meist schwer löslichen Calcium-Komplexe auf den Misch-kondensaten aufziehen.

Ferner ist es stets dann, wenn die erfindungsgemäßen Produkte Azulminsäuren enthalten, möglich, die Produkte nach ihrer Herstellung gleichzeitig mit Ammoniak und Kohlendioxid zu begasen. Dabei dringen Ammoniak und kohlendioxid als kleine Moleküle in beträchtlichem Maße in das enthaltene Azulminsäuregerüst ein. Man erhält z.B. bei der Begasung mit Ammoniak und Kohlendioxid im Wirbelbett die instabilen Ammoniumcarbaminate, Ammoniumbicarbonate und, — sofern Ammoniak und Kohlendioxid in Anwesenheit von Wasser eingeleitet werden —, carbaminsaures Ammonium der Formel

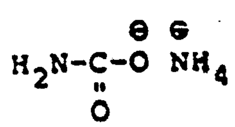

in den Kanälen der Azulminsäure-Mischkondensate fixiert. Das carbaminsaure Ammonium weist in dieser Form bei Raumtemperatur eine verminderte Zersetzlichkeit auf.

Derartige Produkte eignen sich als Düngemittel zur langfristigen Versorgung von Pflanzen mit Stickstoff und, je nach Beladung-, mit anderen Makro- und/oder Mikronährstoffen.

In den Azulminsäuren, die bei dem erfindungsgemäßen Verfahren mit verwendet werden können, läßt sich gewünschtenfalls vor oder während der Umsetzung die Zahl der Fehlerstellen erhöhen. Ferner kann auch nach beendeter Mischkondensation in den nach dem erfindungsgemäßen Verfahren hergestellten Azulminsäure enthaltenden Produkten die Zahl der Fehlerstellen in den Azulminsäuren erhöht werden. Hierzu eignen sich alle diejenigen Verfahren, die bereits im Zusammenhang mit der Beschreibung der Herstellung der modifizierten, also fehlerstellenhaltigen Azulminsäuren aufgeführt wurden. — Soll die Fehlerstellenerzeugung nach erfolgter Mischkondensation vorgenommen werden, so verfährt man in der Weise, daß man die Reaktionsprodukte in wäßrigem Medium, gegebenenfalls bei erhöhten Temperaturen mit den zur Fehlerstellenerzeugung eingesetzten Reagenzien verrührt. Die Produkte werden durch Filtration isoliert.

Bei der Durchführung des erfindungsgemäßen Verfahrens lassen sich insbesondere bei Verwendung hochreaktiver Carbonylverbindungen, wie Formaldehyd oder Crotonaldehyd, auch solche Biomassen, die zur Bildung resistenter Sporen neigen, vollständig desaktivieren. Nötigenfalls können bei besonders stark resistenten Sporen die bekannten Verfahren der Pasteurisierung angewendet werden, indem beispielsweise in den Verfahrensprodukten enthaltende Aldehyd-Spuren durch Ammoniak völlig desaktiviert werden, dann Peptone, Dextrine zugegeben werden, bei 37 °C die Sporen erneut zur Belebung und Zellteilung angeregt werden und anschließend z.B. mit kleinen Mengen an Monomethylolharnstoff und Formaldehyd die erfindungsgemäße Mischkondensation bei 70-80 °C gegebenenfalls mehrfach wiederholt wird.

Der Nachweis dafür, daß bei dem erfindungsgemäßen Verfahren rasch und quantitativ eine Sterilisation der eingesetzten Biomassen erfolgt, kann in bekannter Weise in sterilen Agar und Peptone enthaltenden Petrischalen geführt werden, wobei diese primär zur Sterilisierung in üblicher Weise bei 120 °C etwa 40 Minuten einer Dampfatmosphäre ausgesetzt werden. Messungen an derartig sterilisierten, mit den Verfahrensprodukten angeimpften Nährböden zeigen unter standardisierten Prüfbedingungen in gesättigter steriler Wasserathmosphäre sowohl nach 24 Stunden und als auch nach 48 Stunden bei erfindungsgemäß sterilisierten Bakterien-Biomassen keine Keimbildung, ebenso nicht nach 72 und mehr Stunden. Gleiches gilt für erfindungsgemäß kondensierte und sterilisierte bzw. pasteurisierte Pilzmassen und Hefemassen. Die Keimteste sind in allen Fällen negativ. Entsprechende Prüfungen an anaeroben oder aeroben Mikroorganismen-Kondensaten unter optimalen Anzucht- und Temperaturbedingungen deuten die völlige Sterilisierung der Biomassen bei der Durchführung des erfindungsgemäßen Verfahrens an.

Sterilisationen der eingesetzten Biomassen lassen sich bei dem erfindungsgemäßen Verfahren besonders vorteilhaft auch dadurch erreichen, daß man die Kondensation im Temperaturbereich von 10 °C bis 140 °C unter stark vermindertem Druck vornimmt. Hierdurch können Sterilisationen, Plasmolyse, Zellwandsprengung von Mikroorganismen, wie Bakterien, Algen, Hefen u.s.w., außerordentlich beschleunigt werden.

Um bei der Durchführung des erfindungsgemäßen Verfahrens eine maximale Zellabtötung, Enzymdesaktivierung und Geruchsverbesserung bei beliebigen Biomassen zu erreichen, gleichzeitig aber wertvolle Zellinhaltsstoffe hydrolytisch nicht in wasserlösliche Produkte zu spalten (= Proteinabbau, Polysaccharidabbau) hat es sich als besonders vorteilhaft erwiesen, die Biomassen primär mit einen 1 bis 2-molaren Überschuß an Aldehyden, wie Formaldehyd, Isobutyraldehyd, Crotonaldehyd oder Glyoxal, bei Temperaturen zwischen 20 °C und 100 °C, vorzugsweise zwischen 40 °C und 70 °C, im pH-Bereich zwischen 6 und 7,5, also praktisch neutral, in 30 Minuten bis zu 2 Stunden zu kondensieren und anschließend nach Zusatz eines Aminoplastbildners, wie Harnstoff, Melamin oder bevorzugt Harnstoff-Azulminsäure oder Melamin-Azulminsäure, in Gegenwart von Phosphorsäure oder Schwefelsäure bei pH-Werten zwischen 1 und 4 und bei Temperaturen zwischen 20 °C und 70 °C im Verlaufe von 1 bis 4 Stunden die Mischkondensation zu Ende zu führen. Dabei werden völlig klebfreie, leicht filtrierbare, pulvrige Mischkondensate höchster Lagerstabilität bei vollständiger Enzymdesaktivierung erhalten. Derartige Mischkondensate sind vollständig keimfrei.

Im übrigen lassen sich bei dem erfindungsgemäßen Verfahren auch mit Hilfe erdiger Materialien normalen Bakteriengehaltes, Algen- und Pilzgehaltes, vollständige Sterilisierungen errreichen. Verwendet man zum Beispiel beim erfindungsgemäßen Verfahren als Zusatzmittel für 1 Liter etwa 2 Gew.-% Feststoff enthaltende Jungzellen-Nährhefe-Suspensionen zusätzlich

a) 100 $cm^3$ Flußwasser (= 1 $cm^3$ enthält 10 000 bis eine Billion Bakterien)

b) 100 $cm^3$ normales Leitungswasser (= 1 $cm^3$ enthält ca-1 000 Bakterienkeime)

c) 20 g feuchte, mit Torf gemischte Gartenerde (= 1 g enthält Millionen von Mikroorganismen wie Bakterien, Kleinalgen und Pilze),

so stellt man nach Durchführung der erfindungsgemäßen Kondensation und nach Bestimmung des Sterilitätsgrades nach erfolgter Mischkondensation mit 60 g Harnstoff und 30 g Formaldehyd völlige Keimfreiheit in biologischen Testen fest. Hierbei ist Phosphorsäure oder Schwefelsäure der bevorzugte Kondensationskatalysator, da der Katalysator durch Zugabe von Calciumoxyd, Calciumcarbonat oder

Kalkmilch praktisch quantitativ gefällt werden oder gewünschtenfalls Phosphorsäure auch als Aluminiumphosphat mit frisch gefälltem Aluminiumhydroxyd niedergeschlagen werden kann, so daß keine Abwasserbelastung durch Phosphat-haltige Filtrate erfolgt.

Wenn bei dem erfindungsgemäßen Verfahren eine besonders schnelle Enzymdesaktivierung erreicht werden soll, so geht man zweckmäßigerweise so vor, daß man in der ersten Reaktionsphase 0,5 - 2 Gew.-% an wasserunlöslichen Aldehyden, wie Isobutyraldehyd und insbesondere Crotonaldehyd, vor der Zugabe von Harnstoff und Formaldehyd zusetzt.

Bei der Durchführung des erfindungsgemäßen Verfahrens können die verschiedensten Biomassen gewünschtenfalls vor der Kondensation mit kleinen Mengen an Oxidationsmitteln, wie Salpetersäure, Chlorlauge, Calciumhypochlorit, Wasserstoffperoxid, Chromsäure oder Kaliumpermanganat vorbehandelt werden. Dadurch werden Geruchsträger zum Teil zerstört, und die Farbe der Filtrate wird verbessert.

Im übrigen ist es oft vorteilhaft, schwerlösliches Harnstoffnitrat auf den Biomassen zu erzeugen und dann mit Formaldehyd und Azulminsäuren die erfindungsgemäße Kondensation durchzuführen. Ebenso kann primär schwerlösliches Ammoniummagnesiumphosphat auf den Biomassen als Trägersubstanz erzeugt werden und dann die erfindungsgemäße Kondensation durchgeführt werden.

Bei dem erfindungsgemäßen Verfahren können selbstverständlich auch Mischungen von mehreren Aminoplastbildnern, Phenoplastbildnern und/oder Mischungen von mehreren Carbonylverbindungen zur Kondensation verwendet werden. So können z.B. solche Aldehyd-Gemische eingesetzt werden, die aus Formaldehyd und zahlreichen anderen Aldehyden bzw. Ketonen unter den Bedingungen der Aldolkondensation, der Formose-Synthese oder der Acyloinkondensation gegebenenfalls in situ entstehen (vgl. Reaktionsgleichungen auf Seiten 26 und 27). Derartige Hydroxyaldehyde reagieren im Maße ihrer Bildung, insbesondere im schwach bis stark alkalischen Bereich mit Aminoplastbildnern, wie z.B. Harnstoff, leicht unter Addition zu N-Alkylolverbindungen, die dann mischkondensiert werden.

In einer besonderen Variante des erfindungsgemäßen Verfahrens arbeitet man in der Weise, daß man spezielle Aldehyde, bevorzugt Formaldehyd in alkalischem Milieu auf andere Aldehyde einwirken läßt, und zwar in Gegenwart der zur Aminoplastbildung eingesetzten Stoffe. Obwohl sich dabei N-Alkylolverbindungen bzw. N-Methylolverbindungen bilden, so sind diese doch nicht die thermostabilsten Derivate und es erfolgt Bildung der thermostabileren Hydroxyaldehyde. Letztere führen im Zuge der erfindungsgemäßen Umsetzung zu Hydroxylgruppen enthaltenden Aminoplastkondensaten, die den sprühgetrockneten Produkten eine gewünschte krümelige Struktur verleihen. Über diese Variante des erfindungsgemäßen Verfahrens kann vor allem auch die Quellarbeit der erfindungsgemäßen Produkte beeinflußt werden. Außerdem kann diese Reaktion in Gegenwart von auf Azulminsäure niedergeschlagenem Calciumhydroxid oder Bleihydroxid dazu benutzt werden, um karamelisierte Zucker zu synthetisieren (Butlerow-Löw-Formose-Reaktion), die an den Aminoplastkondensationen teilnehmen und für eine Krümelstruktur der erfindungsgemäßen Produkte sorgen.

Bei der Durchführung der erfindungsgemäßen Mischkondensation können Kalium-Ionen und Cyanid-Ionen, ihre bekannte katalytische Aktivität ausüben und z.B. Acyloinkondensationen bewirken (vgl. Reaktionsgleichung (e) auf Seite 27). Die dabei entstehenden Produkte können im Zuge der Aminoplastkondensationen, insbesondere bei basisch katalysierten Reaktionen, an der Kondensation teilnehmen und ferner bei Angebot von Formaldehyd durch Aldolkondensationen in methylolierte Ketone übergehen, welche ebenfalls Kondensationspartner darstellen.

Eine weitere Variante des erfindungsgemäßen Verfahrens besteht darin, daß man als Aminoplastbildner solche Azulminsäuren einsetzt, die vor der eigentlichen Umsetzung partiell mit Carbonylverbindungen, wie Formaldehyd, Glyoxal oder Glyoxalsulfat, kondensiert und dadurch gegen Blausäuresäureabspaltung stabilisiert wurden. Die damit erhaltenen erfindungsgemäßen Produkte zeichnen sich durch eine besonders hohe thermische Beständigkeit aus.

Ferner besteht eine vorteilhafte Variante des erfindungsgemäßen Verfahrens darin, daß man Harnstoff, Melamin oder Dicyandiamid in wäßriger Lösung vorlegt, dann Azulminsäure und Biomasse dispergiert, danach mit Formaldehyd kondensiert und durch Zugabe von Phosphorsäure bzw. Oxalsäure aus nicht umgesetztem Melamin oder Harnstoff schwerlösliches Melaminphosphat oder Harnstoffoxalat erzeugt. Hierbei laufen Fehlerstellenerzeugung in der Azulminsäure und Mischkondensation gleichzeitig ab. Die dabei entstehenden Produkte stellen hochwertige reaktive Füllstoffe und Flammschutzmittel für Kunststoffe, insbesondere Polyurethankunststoffe, dar.

Die erfindungsgemäße Kondensation der Biomassen in Gegenwart von Azulminsäuren kann auch unter oxidativen Bedingungen mit kleinen Mengen an Oxidationsmitteln, wie Wasserstoffperoxid, Chlorlauge, Chlorkalk oder Kaliumpermanganat, vorgenommen werden.

In einer weiteren Variante kann das erfindungsgemäße Verfahren auch in Form einer Eintopfreaktion durchgeführt werden. Dabei geht man so vor, daß man Biomassen mit größeren Mengen an Aminoplastbildnern, wie Harnstoff, mischt und gegebenenfalls in Gegenwart anderer löslicher Aminoplastbildner sowie gegebenenfalls in Gegenwart von Mono- und Polyalkoholen und kleinen Mengen an Phenoplastbildnern, wie Phenol oder o-Kresol, bei etwa 70 °C vorbehandelt, dann Aldehyde, wie Crotonaldehyd, zugibt und anschließend bevorzugt im pH-Bereich 1 bis 2,5 mit Formaldehyd kondensiert.

Eine andere Variante des erfindungsgemäßen Verfahrens besteht darin, daß man bei der Mischkondensation auf 100 Gewichtsteile Biomasse 100 bis 500 Gewichtsteile an etwa 30 %-igen wäßrigen Natrium-oder Kaliumsilikat-Lösungen zugibt, wobei es in der Regel zweckmäßig ist, bei etwa 40 °C bis 100 °C mit Carbonylverbindung und Aminoplastbildner zu kondensieren, oder die Mischkondensate nachträglich mit den oben genannten Mengen an Alkalisilikaten umzusetzten.

Nach beendeter Reaktion kann überschüssiges gelöstes Natrium- bzw. Kaliumsilikat durch einfaches Begasen der jeweiligen Dispersionen mit Kohlendioxid gefällt werden, oder in besonders vorteilhafter Weise durch Zugabe von Phosphorsäure oder Calciumchlorid in Mischung mit Kalium- bzw. Natriumphosphaten oder Calciumsilikaten ausgefällt werden. Die so entstehenden Produkte sind interessante Kombinationsfüllstoffe mit reaktiven Gruppen.

Eine weitere interessante Variante des erfindungsgemäßen Verfahrens besteht in der Mitverwendung von solchen Aminoplastbildnern, die besonders gute Komplexbildner für Schwermetalle sind. Hierzu gehören z.B. Thioharnstoff und N-Methylolgruppen enthaltende Polymethylenthioharnstoffe der Konstitution

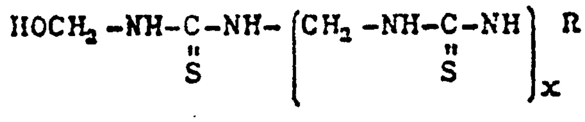

R = H, $CH_2OH$
x = 0 bis 14,
ferner ringförmige Äthylenthioharnstoffkondensate wie

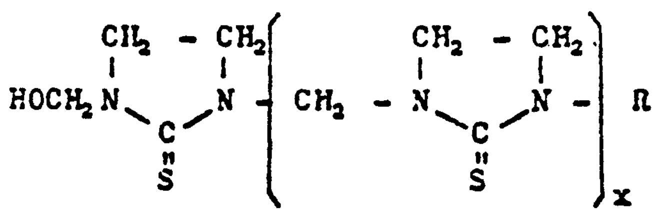

R = H, $CH_2OH$
X = 0 bis 14
Weiterhin Aminoplastbildner mit ionischen Gruppen, z.B. die Verbindung der Konstitution

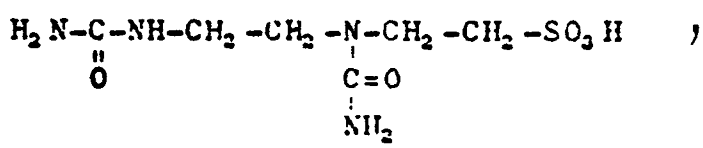

und außerdem Phosphonsäuren, z.B. diejenige der Konstitution

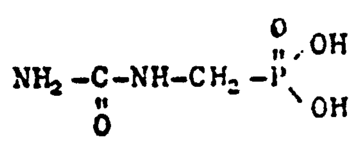

Bei der Verwendung derartiger Komplexbildner können insbesondere in Biomassen aus Kläranlagen für industrielle und kommunale Abwässer enthaltene schädliche Schwermetall-Ionen so fest gebunden werden, daß bei der Verwendung der erfindungsgemäßen Biomassenkondensationsprodukte als Langzeitdüngemittel keine Pflanzenschädigungen durch Schwermetalle, wie Quecksilber, Blei, Chrom, Cadmium und Zink, auftreten.

Wenn man bei der Durchführung des erfindungsgemäßen Verfahrens eine Proteinhydrolyse von Glykoproteiden, Nucleinsäuren und anderen Zellinhaltsstoffen maximal vermeiden will, so erweist es sich als vorteilhaft, die Biomassen zunächst im strengen Neutralbereich bei pH 7 mit Harnstoff und Formaldehyd bzw. Dimethylolharnstoff oder Monomethylolharnstoff in einer Verdünnung von etwa 60 g Harnstoff pro Liter 8-10 % Feststoff enthaltender wäßriger Biomasse bei 60-80 °C in Gegenwart von 80-100 g Azulminsäuren zu kondensieren, anschließend auf 35-20 °C herunterzukühlen und dann spontan durch Ansäuren (pH = 1,5-2,5) die Weiterkondensation gebildeter Methylolierungsprodukte durchzuführen. Hierdurch kann der hydrolysierte und wasserlösliche Anteil von Zellinhaltsstoffen insbesondere bei Mitverwendung der verschiedensten Azulminsäuren auf ein Minimum herabgedrängt werden. In einzelnen Fällen kann es von Vorteil sein, hierbei 2-10 Gew.-% Melamin oder Dicyandiamid, bezogen auf eingesetzten Harnstoff, mitzuverwenden.

Da N-Alkylolverbindungen und insbesondere N-Methylolverbindungen im sauren pH-Bereich leicht Verätherungsreaktionen eingehen, kann man die erfindungsgemäßen Verfahrensprodukte bei ihrer Herstellung durch Mitverwendung von z.B. 10-60 Gew.-% an polyfunktionellen Hydroxylverbindungen, z.B. Polyalkoholen wie Äthylenglykol, Glyzerin, Formose-Zuckergemischen, Glukose, Poly- und Oligosaccariden, Stärke, etc., über Verätherungsreaktionen modifizieren.

Die erfindungsgemäßen Produkte lassen sich formelmäßig nicht eindeutig definieren. Sie sind jedoch durch die Ausgangskomponenten und das Verfahren zu ihrer Herstellung genau charakterisiert. — Ein gemeinsames Merkmal der erfindungsgemäßen Stoffe besteht darin, daß sie Molekülsegmente « A » enthalten, die durch Kondensation von Carbonylverbindungen bzw. Thiocarbonylverbindungen und Aminoplastbildnern bzw. Phenoplastbildnern mit reaktiven Gruppen der Biomassen entstehen.

Das segment « A » kann dabei unter anderem für folgende Reste stehen :

— Aralkyliden — polythioharnstoff — Reste, Polyalkyliden-polythioharnstoff-Reste und insbesondere Polymethylen-polythioharnstoff-Reste, welche sich durch folgende idealisierte Formel veranschaulichen lassen

$$H_2N-\underset{\overset{\|}{O(S)}}{C}-NH-\left[\underset{R}{CH}-NH-\underset{\overset{\|}{O(S)}}{C}-NH\right]_x-\underset{R}{CH}- \qquad (A-1)$$

in welcher

X für ganze Zahlen von 0 bis 18 steht und

R vorzugsweise für Wasserstoff, Methyl, Äthyl, Propyl, Isopropyl sowie für Reste der Formeln

$$HO-CH_2-\underset{CH_3}{\overset{CH_3}{C}}- \qquad CH_3-CH=CH-$$

$$HO-CH_2-, \quad HO-CH_2-\underset{OH}{CH}-, \quad HO-CH_2-\underset{OH}{CH}-\underset{OH}{CH}-,$$

$$HO-CH_2-\underset{OH}{CH}-\underset{OH}{CH}-\underset{OH}{CH}-,$$

$$HO-CH_2-\underset{OH}{CH}-\underset{OH}{CH}-\underset{OH}{CH}-\underset{OH}{CH}-$$

steht sowie für isomere Hydroxyaldehydreste, wie sie in den Formose-Zucker gemischen vorliegen, außerdem für Oligosaccharide und ferner für die Reste der Formeln

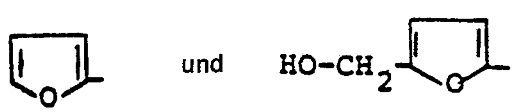

steht ;

— Biuretsulfone der Konstitution

$$H_2N-\underset{\overset{\|}{O}}{C}-NH-SO_2-NH-\underset{R}{CH}-$$

bzw. 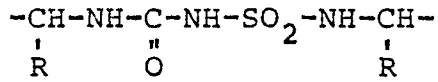

worin R die in der Formel (A – 1) angegebene Bedeutung hat,

— Hydrouracil-Reste, die aus 1 Mol an ungesättigter Carbonsäure, z.B. Crotonsäure, 1 Mol Harnstoff und Formaldehyd entstehen, wobei der Rest der Konstitution

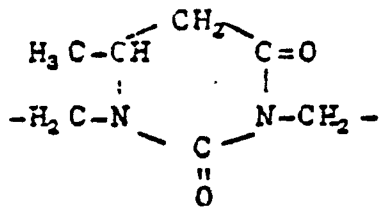

beispielhaft genannt sei ;

— Reste von Umsetzungsprodukten der Maleinsäureureide mit Aldehyden, bevorzugt Formaldehyd, wobei der Rest der Konstitution

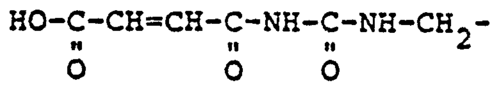

beispielhaft genannt sei ;

— Methylengruppen enthaltende Lactam- und Azalactam-Reste, z.B. Reste der Konstitution

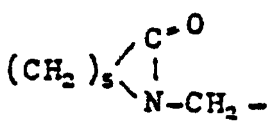 bzw. 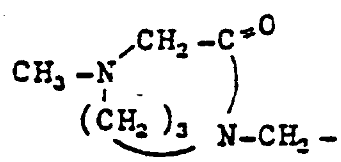

— Methylengruppen enthaltende Reste cyclischer Verbindungen der Konstitution

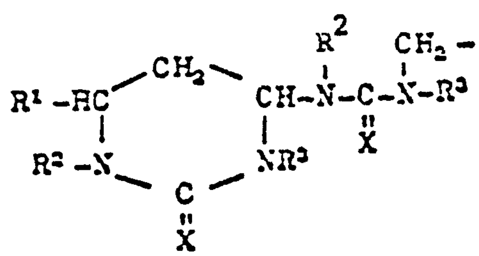

worin

X für Sauerstoff oder Schwefel steht und

$R^1$, $R^2$ und $R^3$ gleich oder verschieden sind und für Wasserstoff, Methyl, Äthyl oder Methylengruppen stehen ;

— Methylengruppen enthaltende Reste des Äthylendiharnstoffs wie z.B. der Konstitution

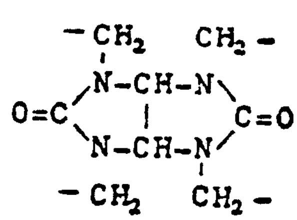

— Methylengruppen enthaltende Reste des Äthylenharnstoffs, Vinylidenharnstoffs und Dihydroxyäthylenharnstoffs wie Reste der Formeln

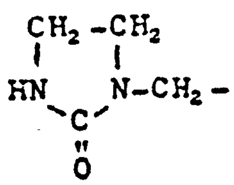 bzw. 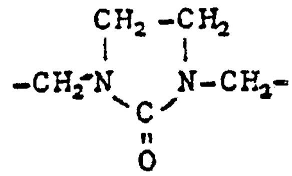 bzw.

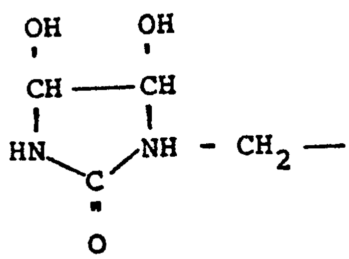 bzw. 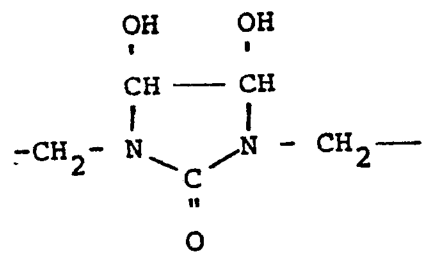

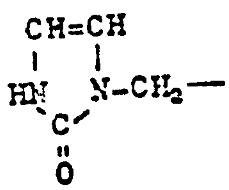 und 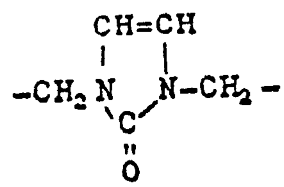 ;

— Methylengruppen enthaltende Reste des Harnstoffs, wie z.B. Reste der Konstitution

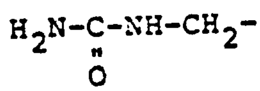 bzw. 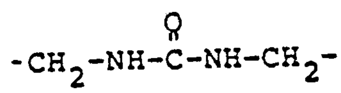

— Methylen- bzw. Alkyliden-Reste des 2-Oxo-4-methyl-6-ureido-hexahydropyrimidins der Konstitution

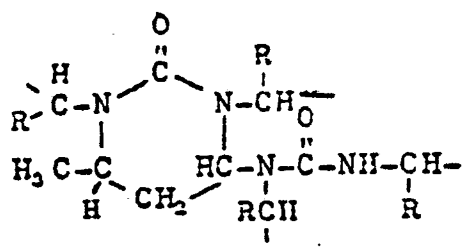

wobei R die obengenannte Bedeutung hat ;

— Methylen- bzw. Alkyliden-Reste mit Melamin-Strukturen wie

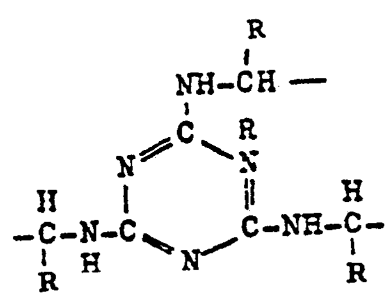

wobei R die obengenannte Bedeutung hat ;

— Reste der Formel

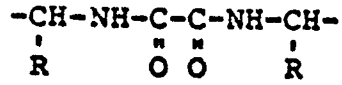

worin

R die oben angegebene Bedeutung hat ;

— Methylen- bzw. Alkyliden-Reste mit Sulfonamid-Strukturen wie z.B.

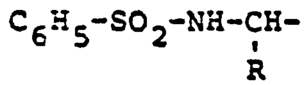

bzw.

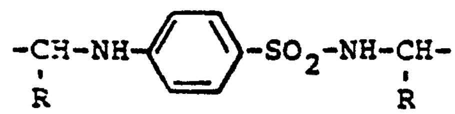

worin

R die oben angegebene Bedeutung hat ;

— Methylen- bzw. Alkyliden-Reste mit Dicyandiamid-, Guanidin-, Formaldehyd- bzw. Isobutyraldehyd-Strukturen ;

— durch Methylen- bzw. Alkyliden-Reste substituierte aromatische und aliphatische Amine, Polyamine, Hydrazine, Hydrazide, Hydrazodicarbonamide, Dicarbonsäure-dihydrazide, Hydrazincarbonsäure-äthylester und Phosphoramid-Reste.

Die erfindungsgemäßen Stoffe sind keimfrei, lagerbeständig und in den meisten Fällen geruchlos. Sie können für eine Vielzahl von Zwecken verwendet werden.

So können erfindungsgemäße Produkte Verwendung finden als Mehrkomponentenfüllstoffe mit reaktiven Gruppen in Kunststoffen verschiedenster Art.

Bei der Herstellung von gegebenenfalls zelligen Kunststoffen unter Verwendung erfindungsgemäßer Mischkondensate als Füllstoffe können selbstverständlich auch andere gebräuchliche Füllstoffe, Flammschutzmittel und Zusatzstoffe der verschiedensten Art mitverwendet werden. Bevorzugt in Betracht kommen hierbei wasserlösliche oder wasserdispergierbare anorganische Salze, Doppelsalze oder komplexe Verbindungen, beispielsweise Ammoniumsulfat, ammoniumsulfathaltige Endlaugen der Caprolactamherstellung, Calciumsulfat, $(NH_4)_2SO_4.H_2O.Ca(H_2PO_4)_2$, Ammoniumphosphat, $CaNaPO_4.Ca_2SiO_4$, $5\,CaO.P_2O_5.SiO_2$, $Ca_4P_2O_9$, $(CaMg)O.Al_2O_3.4\,SiO_2$, $Al_2O_3.2\,SiO_2$. $2\,H_2O$, $KCl.MgSO_4.3\,H_2O$, $K_2SO_4.MgSO_4.6\,H_2O$, $Si_2O_6AlK$, Natriumnitrat, Ammoniumnitrat, sekundäres Natriumammoniumphosphat und ähnliche Stoffe.

Zahlreiche erfindungsgemäße Stoffe lassen sich als Flammschutzmittel bzw. Alterungsschutzmittel zur Verhinderung des oxidativen Abbaus in den verschiedensten Vinylpolymerisaten, Polyamidkunststoffen, Kautschuken und Epoxidharzen verwenden. Insbesondere eignen sich hierzu diejenigen erfindungsgemäßen Stoffe, die kondensierte Azulminsäuren und gleichzeitig Phosphorsäure, phosphorige Säure, Polymethylenharnstoffe, Polymethylenmelamine, Calciumphosphate, Calciumcarbonat, Titandioxid, Tonerdehydrate, Aluminiumphosphate, Aluminiumsilikate, Aluminiumoxidhydrat, Wasserglas, Melaminphosphat, Bariumphosphate, Ammonium-magnesium-phosphate und/oder Harnstoffoxalat enthalten.

Darüber hinaus können erfindungsgemäße Stoffe als Trägermaterialien für zahlreiche Katalysatoren eingesetzt werden, wobei Mischkatalysatoren entstehen, die sich vielseitig verwenden lassen.

Im übrigen lassen sich erfindungsgemäße Stoffe als Futterzusatzmittel, als Nährmedium für Bakterien als Bodenverbesserungsmittel und als Düngemittel verwenden. Sie eignen sich insbesondere zur langfristigen Versorgung von Pflanzen mit Stickstoff und, — je nach den enthaltenden Komponenten —, auch zur Versorgung von Pflanzen mit anderen Makro- und/oder Mikronährstoffen. Hierbei sind als Düngemittel vor allem diejenigen Produkte geeignet, die aus schwermetallfreien Biomassen der Arzneimittel-, Genußmittel- und Lebensmittelindustrie bestehen. Ferner auch solche Produkte, die aus Biomassen aus biologisch oder vollbiologisch arbeitenden Kläranlagen für industrielle und kommunale

Abwässer hergestellt werden, sofern die betreffenden Abwässer vor der biologischen oder vollbiologischen Reinigung einer Sulfidfällung, z.B. mittels Ammoniumsulfid, Natriumsulfid, Schwefelwasserstoff u.s.w., unterzogen werden und die entstehenden Biomassen-Mischkondensate somit nur winzige Mengen an Schwermetallen enthalten.

Das erfindungsgemäße Verfahren ist insbesondere bei Verwendung hochreaktiver Carbonylverbindungen, wie Formaldehyd, zur problemlosen Aufarbeitung von Biomassen der verschiedensten Art geeignet. Die entstehenden Produkte sind keimfrei, leicht filtrierbar, lagerfähig und meistens frei von Geruchsträgern.

Bei der Durchführung des erfindungsgemäßen Verfahrens werden somit alle in den Biomassen vorkommenden Enzyme, die zum Beispiel Oxydationen, Reduktionen, hydrolytische Reaktionen, Acylierungen und Kondensationen, Aminierungen, Decarboxylierungen, Einführungen von Hydroxylgruppen in Steroide, Hydroxylierungen und Weiteroxydationen, oxydative Ringöffnungen, Dehydrierungsreaktionen, Hydrierungsreaktionen olefinischer Doppelbindungen, Hydrolyse von Amidgruppen, von Harnstoffgruppen und Hydantoin-Gruppen, hydrolytische Eliminierung von Substituenten wie $NH_2$- durch OH-Gruppen, fermentative Wasseradditionen an Doppelbindungen, hydrolytische Ringöffnungsreaktionen, Phosphorylierungesreaktionen, Umgruppierungen und Abbaureaktionen an Nucleotiden, Acylolinkondensationen, Desaminierungsreaktionen, Transaminierungsreaktionen, Amidierungsreaktionen oder Decarboxylierungsreaktionen mit anschließender gekoppelter β-Oxydation bewirken können, vollständig desaktiviert.

Ferner gelingt es mit Hilfe des erfindungsgemäßen Verfahrens auch solche Biomassen, die bei einer Vielzahl von technischen Fermentationsprozessen oder bei der vollbiologischen Reinigung von Abwässern anfallen, und die sehr schwer oder gar nicht filtrierbar sind, schon bei niedrigen Temperaturen innerhalb kurzer Zeiten in leicht filtrierbare Produkte zu überführen, die nach dem Trocknen völlig klebfreie Pulver darstellen und keine kolloidalen Lösungen bilden.

Das erfindungsgemäße Verfahren kann mit besonderem Vorteil auch angewendet werden, um mit Pflanzenschutzmittel-Resten verseuchte Biomassen, wie z.B. Belebtschlamm aus biologischen Kläranlagen oder Antibiotika enthaltende Biomassen der Penicillinherstellung völlig zu desaktivieren und in weiter verwendbare Produkte zu überführen. — Die Desaktivierung von noch vorhandenen Pflanzenschutzmittel-Resten in Biomassen aus der biologischen Reinigung industrieller und kommunaler Abwässer ist zum Beispiel dadurch möglich, daß diese Wirkstoffe mitkondensieren oder hydrolysiert werden und oft über entstehende funktionelle Gruppen in Kondensationsreaktionen mit Carbonylverbindungen bzw. N-Methylolverbindungen eintreten. So kondensieren zum Beispiel Carbamate an ihren NH-Gruppen z.B. mit Formaldehyd und/oder N-Methylolverbindungen ; ebenso 3-Amino-1,2,4-triazol an $NH_2$- und NH-Gruppen ; Dithiocarbamate hydrolysieren und werden desaktiviert ; Phosphorsäureester der verschiedensten Art werden generell hydrolysiert und z.B. durch Formaldehydkondensation im Phenolteil desaktiviert. Quecksilber und andere Metalle enthaltende Wirkstoffe werden bei Mitverwendung von Azulminsäuren als Aminoplastbildner durch komplexierung desaktiviert, Harnstoffgruppen aufweisende Wirkstoffe werden durch Kondensation ihrer NH-Gruppen z.B. mit Formaldehyd oder N-Methylolverbindungen desaktiviert. Wirkstoffe der Thiophosphorsäure-Reihe werden hydrolysiert, Phosphorsäure-Gruppen enthaltende Wirkstoffe werden hydrolysiert, Sulfamidgruppen enthaltende Wirkstoffe werden hydrolysierend kondensiert. Phenol- und Nitrophenol-Derivate als Wirkstoffe werden bei nicht substituierter ortho- und para-Stellung z.B. mit Formaldehyd oder N-Methylolverbindungen kondensiert, ebenso Benzthiazolyl-harnstoffe an ihren NH-Gruppen.

2,4-Dichlorphenoxyessigsäure und ähnliche Wirkstoffe werden bei Mitverwendung von Azulminsäuren salzartig fixiert und desaktiviert. Imidazolidin- und Uracilgruppen enthaltende Wirkstoffe werden an freien NH-Gruppen kondensiert und desaktiviert. N-Trichlormethyl-thiophthalimid wird hydrolysierend kondensiert. Der Wirkstoff 4-Amino-6-tert.-butyl-3-(methylthio)-1,2,4-triazin-5(4 H)-on wird durch Kondensation der hochreaktiven $NH_2$-Gruppe z.B. mit Formaldehyd oder N-Methylolverbindungen desaktiviert. 3-Dimethyl-3-(2-benzthiazolyl)-harnstoff wird durch Kondensation an der reaktiven NH-Gruppe desaktiviert, desgleichen NH-Gruppen enthaltende Thiadiazol-Derivate und Benzimidazol-Derivate.

Das erfindungsgemäße Verfahren eignet sich vor allem dann, wenn Thioharnstoff oder andere stark komplexierend wirkende Verbindungen als Aminoplastbildner verwendet werden, um in den Biomassen enthaltene Schwermetallionen, wie z.B. Ionen des Bleis, Kupfers, Quecksilbers, Cadmiums oder des Zinks, so fest zu binden, daß beim Einsatz derartiger Produkte als Düngemittel keine Pflanzenschädigungen auftreten.

Wird das erfindungsgemäße Verfahren unter Mitverwendung von Azulminsäuren (Rohazulminsäuren, modifizierten Azulminsäuren und/oder stabilisierten Azulminsäuren) durchgeführt, so können normalerweise sehr leicht wasserlösliche Zellinhaltsstoffe der Biomassen wie Polysaccharide, wasserdispergierbare oder lösliche Glykolipide, Lipoproteide, abgebaute Proteine, Nucleinbasen, abgebaute aber nicht kondensierte Nucleinsäuren vollständig auf Azulminsäuren adsorbiert werden, so daß die Abwässer nicht mit den für die Humifizierung und für die Pflanzenernährung wertvollen Nährstoffen belastet werden.

Weiterhin kann das erfindungsgemäße Verfahren besonders vorteilhaft zur Aufarbeitung solcher Biomassen angewendet werden, in denen die Bildung unangenehmer Geruchsträger durch Zersetzungsreaktionen bereits weit fortgeschritten ist und in denen insbesondere Enzyme von Mikroorganismen zur

Bildung der Geruchsträger beitragen.

Derartige Biomassen können sich auf verdorbenen Nährstoffen bilden, ferner auf Ricinusölrückständen, Hefemassen, Baumwollsaatmehl, Sojamehl, Fischmehl, Tiermehl, Knochenmehl, Algenmehl, Ricinussamenmehl, Stärkemehl, Dextrinen, Peptonen, Lignin, feuchten Cellulosepulvern, feuchtem homogenisiertem Nadelholz und homogenisiertem Blattmaterial, feucht gelagerten Gelatinen, Pepton-Agarkombinationen, Fleisch-Extraktpulver, etc. Solche Mischungen von Naturstoffen mit Biomassen können erfindungsgemäß völlig sterilisiert oder pasteurisiert werden und ihre Geruchsträger weitgehend durch Kondensationsreaktionen eliminiert werden. Die anfallenden Produkte weisen gegenüber den Ausgangsstoffen einen erhöhten Stickstoffgehalt auf und lassen sich deshalb besonders gut als Stickstoff-Düngemittel mit Langzeitwirkung einsetzen.

Das erfindungsgemäße Verfahren eignet sich auch besonders gut zur Aufarbeitung

— von Biomassen in Gegenwart von größeren Anteilen an extracellulären Kolloiden,

— ebenso von Biomassen, in denen der Zelltod bereits weit fortgeschritten ist, d.h. Biomassen, die eine große Menge an Zellinhaltsstoffen außerhalb der Zellmembranen in wässriger Lösung enthalten,

— ferner von Biomassen der cellulären und extracellulären Blutbestandteile,

— sowie schleimartige, völlig unfiltrierbare Biomassen.

Dabei werden diese Biomassen, schwer filtrierbare Schwebstoffe, Faul- und Bioschlämme der verschiedensten Art und Biomassen mit hohen Anteilen an Escherichia coli unter sterilisierenden Bedingungen als Pulver ausgefällt.

Mit Hilfe des erfindungsgemäßen Verfahrens können auch extrem große Mengen an Bakterienzellen abgetötet und in ihren wichtigsten Zellinhaltsstoffen kondensiert werden. Größenordnungsmäßig sind bei Bakterien mit 1 $\mu$ Größe bei dichtester Kugelpackung in einem Würfel von 1 $cm^3$ ca. $10^{12}$ Bakterien und pro Liter $10^{15}$ Bakterien enthalten. Beim Einsatz von 10 Litern einer wäßrigen, etwa 10 %igen Biomasse (= Trockengewicht ca 1 kg.) gelingt es daher etwa z.B. mit 6-10 Mol Harnstoff und 6-10 Mol Formaldehyd $10^{15}$ Bakterien und ihr Enzymspektrum abzutöten. Durch Zerstörung der Bakterienzellwände und durch Hydrolyse von enthaltener Poly-β-hydroxybuttersäure tritt hierbei der Geruch von β-Hydroxybuttersäure auf. Durch kurzzeitiges Waschen mit 2 %iger Ammoniaklösung und durch Neutralisation mit Calciumsalzen kann ihr Geruch weitgehend beseitigt werden.

Weiterhin kann das erfindungsgemäße Verfahren mit besonderem Vorteil zur Aufarbeitung von industriell genutzten Mikroorganismen herangezogen werden. Derartige Biomassen, wie sie beispielsweise in großen Mengen bei der Penicillin-Herstellung anfallen, lassen sich wegen ihres hohen Gehaltes an zellgebundenem Wasser auf mechanischem Wege nicht wirksam entwässern. Auch bei einer mühseligen Trocknung zu krümeligen Filterkuchen ist eine Unterbringung dieser Biomassen auf Deponien schwierig, da sie sich durch sogenannte Autolyse wieder verflüssigen und sich zudem unter Geruchsentwicklung leicht zersetzen. Mit Hilfe des erfindungsgemäßen Verfahrens lassen sich derartige Biomassen jedoch in einfacher Weise denaturieren. Außerdem können die dabei anfallenden Produkte, die frei von Schwermetallen sind und einen hohen Stickstoffgehalt besitzen, nicht nur umweltfreundlich beseitigt werden, sondern sogar in vielfältiger Weise sinnvoll verwertet werden.

Überall dort, wo primär keine Kontinuität in der analytischen Zusammensetzung von Biomassen industrieller und kommunaler Abwässer-Kläranlagen gegeben ist, kann das erfindungsgemäße Verfahren besonders vorteilhaft angewendet werden, denn die fehlende Gleichartigkeit in der Zusammensetzung wird dadurch ausgeglichen, daß zum Beispiel Schadstoffe, wie Schwermetalle oder Pflanzenschutzmittel, vor allem bei Verwendung von Azulminsäuren als zusätzliche Aminoplastkomponente so fest gebunden werden, daß keine schädigenden Wirkungen mehr zu befürchten sind. Im übrigen können nicht kondensierte bzw. nicht desaktivierte organische Stoffe wie chlorierte Kohlenwasserstoffe oder Nitrobenzol, die nur schwer abgebaut werden und dem Belebtschlamm eventuell anhaften, aus den erfindungsgemäßen Produkten leicht durch organische Lösungsmittel extrahiert werden.

Mit Hilfe des erfindungsgemäßen Verfahrens können bevorzugt auch Biomassen enthaltende Produkte der Kartoffelstärke-Industrie, z.B. mit verschiedenen bakteriellen und hefeartigen Mikroorganismen befallene lösliche Proteinanteile in Mutterlaugen der Stärkeaufbereitung, ohne kostspieliges Eindampfen der Mutterlaugen aufgearbeitet werden. Gleiches gilt für entsprechende Produkte der Cellulose-Industrie, wie z.B. für neutralisierte und von Bakterien, Pilzen, Algen oder Hefen befallene Sulfitablaugen der Zellstoff-Industrie.

Das erfindungsgemäße Verfahren ermöglicht es, den organischen Anteil von wachsenden Zellen, der oft aus 40 bis 60 % Protein und größenordnungsmäßig aus etwa 20 % Zellwandpolymeren (Kohlehydratanteile), Nucleinsäuren und Lipiden besteht, weitgehend in pulverförmige, in ihrem Stickstoffgehalt stark angereicherte Stoffe umzuwandeln. Im Ruhestand befindliche Zellen, bei denen die Proteingehalte niedriger liegen (= oft 30 % Proteinanteile und Nucleinsäureanteile von 5 bis 8 %), verhalten sich bei ihrer Verwendung und ihrem Einsatz zur erfindungsgemäßen Mischkondensation gleichartig. Schwankungen im Lipoidgehalt, d.h. im Neutralfett, in den Wachsen, Phospholipiden, Steroiden und in den Einzelbausteinen der Zellwandpolymeren in ruhenden und einseitig ernährten Zellen wirken sich nicht störend auf das erfindungsgemäße Verfahren aus. In einseitig ernährten Zellen stärker auftretende Reservekörper wie β-Hydroxybuttersäure und Neutralfette bei Hefen und Pilzen werden auf den Mischkondensaten leicht durch Adsorption fixiert, ebenso stärkeähnliche Produkte, die z.B. bei Algen in größerem Maße gebildet werden können. Auch extracelluläre Schleime wie z.B. Dextrane aus bei

Fermentationen zugeführten Kohlehydraten können pulvrig an den Verfahrensprodukten adsorbiert werden.

Auch Mikroorganismen der biologischen Schlämme aus Kläranlagen, sie sich im Zustand einer Nachgärung befinden, werden zu völlig geruchlosen Verfahrensprodukten kondensiert.

Das erfindungsgemäße Verfahren bietet auch die interessante Möglichkeit, Hydrolysate von Zellschlämmen und Biomassen in Wasser anzureichern und durch Einengen in farbloser Form zu isolieren. Hierbei erfolgt die erfindungsgemäße Kondensation unter hydrolysierenden Bedingungen, Abtrennung der Mutterlauge und Entfernung der darin vorhandenen Schwermetallsalze mit Hilfe handelsüblicher Ionenaustauscher. Anschließend wird die Hydrolysat enthaltende wäßrige Lösung eingedampft. Derartige Hydrolysate können zum Beispiel als Nährboden für eine Vielzahl von Mikroorganismen bei technischen Fermentationsprozessen verwendet werden. Die anfallenden Hydrolysate enthalten zuckerartige Bestandteile, Ammoniumsalze der Phosphorsäure und kondensierte, wasserlösliche Aminosäuren.

Das erfindungsgemäße Verfahren überführt ferner Biomassen aus der Gruppe der Brauereihefen, die sich in Enzymatischer Zersetzung befinden, in lagerstabile, geruchslose Produkte und standardisiert damit in Zersetzung befindliche Biosysteme, ferner mit Mikroorganismen befallene Treberrückstände, Überschußschlämme der Tierkörperverwertung, mikrobiell infiziertes Tiermehl und Verhefungsprodukte aus Abfällen von Massentierhaltungen. Damit wird ein Weg aufgezeigt, bisher schwierig oder gar nicht zu bewältigende Beseitigungsprobleme in einfacher Weise zu lösen. Die gleichen Vorteile bieten sich an bei in Verrottung befindlichen Belebtschlämmen, an mit Bakterien und Pilzen, etc., befallenen Sojaproteinen, bei Naß- und Faulschlämmen zur Erzielung hygienischer Unbedenklichkeit und zur Beseitigung von Geruchsträgern.

Außerdem ermöglicht das erfindungsgemäße Verfahren eine problemlose Aufarbeitung von Biomassen anaerober Faulung (= Intensivfaulung), von Biomassen aus aeroben Stabilisierungsprozessen, Müll-Klärschlamm-Kompostierungsprodukte z.B. aus Verfahren der thermophilen Faulung (= aerobthermophile Verfahren), bei der die freiwerdende Reaktionswärme des mikrobiellen Stoffwechsels zur Erwärmung des Schlammes ausgenutzt wird (= Prozeßtemperaturen ca. 70 °C), ferner von Produkten der aeroben Klärschlammkompostierung nach dem System der Schnellrotte (= Prozeßdauer 10-15 Tage), wobei eine Nachrotte von etwa 6 Wochen vermieden wird. Auch mikrobiell befallene Faserschlämme der Zellstoff- und Papierindustrie, Schlämme der Nahrungs- und Genußmittelindustrie, z.B. Schlämme aus Molkereien, Schlachthöfen, in Zersetzung befindliche Rückstände aus Fermentationsprozessen, geruchlich nicht einwandfreie Hefen, bereits getrocknete und in Deponien befindliche Bioschlämme, z.B. nach dem Porteous-Verfahren getrocknete Biosysteme, können erfindungsgemäß geruchlich stabilisiert werden.

Nach dem erfindungsgemäßen Verfahren können durch die beschriebenen Kondensationsreaktionen Entwässerungsverfahren unter großtechnisch leicht realisierbaren oder beispielsweise in Kläranlagen bereits praktizierten Verfahrensbedingungen wesentlich verbessert werden; d.h. man erhält Klärschlämme oder andere Biomassen mit bedeutend erhöhtem Feststoffgehalt.

Im einzelnen wird das erfindungsgemäße Verfahren durch die nachfolgenden Beispiele veranschaulicht.

### Vergleichsbeispiele

### Beispiel 1

Kondensation von Bakterien-Biomassen mit bestimmten Aldehyden bzw. Ketonen nach konventionellen Methoden

Bei den nachstehend unter (a) bis (d) beschriebenen Umsetzungen wird jeweils ein schwarz-brauner Bakterienbelebtschlamm aus einer vollbiologisch arbeitenden Anlage der Abwasserreinigung industrieller und kommunaler Abwässer mit Formaldehyd kondensiert. Die verwendete Biomasse besteht aus Pseudomonas-Arten, Flagellaten, Algen und anderen Mikroorganismen sowie extrazellulären Bestandteilen. Die eingesetzte Biomasse enthält ca. 85 g an Trockengewicht, Proteingehalt in der Trockensubstanz etwa 42 Gew.-%.

a) 1 000 g der obengenannten Bakterien-Biomasse werden mit 100 g einer 30 %igen wäßrigen Formaldehyd-Lösung (1 Mol) im Verlaufe von 6 Stunden in Gegenwart von 0,5 g Kaliumcarbonat als Methylolierungskatalysator bei 60 °C unter kondensierenden Bedingungen umgesetzt.

b) 1 000 g der obengenannten Bakterien-Biomasse werden bei pH = 7,5 mit 100 g einer 30 %igen wäßrigen Formaldehyd-Lösung (1 Mol) im Verlaufe von 6 Stunden bei 100 °C kondensiert. Hierbei liegen Bedingungen vor, unter denen sich N-Methylolierungsprodukte von Proteinen bzw. N-Methylolierungsprodukte von Nucleinsäuren und Desoxyribonucleinsäuren in ihrem Adenin-, Guanin-, Thymin-, Cytosin-Anteil bilden und neben N-Methylolierungsprodukten auch die Bildung von $>N-CH_2-N<$ und $>N-CH_2-O-CH_2-N<$-Segmenten nicht ausgeschlossen werden kann.

c) 1 000 g der obengenannten Bakterien-Biomasse werden mit 100 g einer 30 %igen wäßrigen Formaldehyd-Lösung (1 Mol) in Gegenwart von 25 g 85 %iger Phosphorsäure im Verlaufe von 8 Stunden

bei 80 °C kondensiert. Hierbei liegen Bedingungen vor, unter denen an Proteinen, wie DNS, RNS und Bakterienzellenwänden unter gleichzeitigen hydrolytischen Abbaureaktionen bevorzugt die Bildung von verzweigten und vernetzten $>N-CH_2-N<$ Segmenten und $>N-CH_2-O-CH_2-N<$ Segmenten erfolgt.

d) 1 000 g der obengenannten Bakterien-Biomasse werden mit 25 g einer 30 %igen wäßrigen Formaldehyd-Lösung (0,25 Mol) in Gegenwart von 18,4 g konzentrierter Schwefelsäure im Verlaufe von 6 Stunden bei 100 °C kondensiert. Hierbei liegen Bedingungen vor, unter denen wegen der relativ niedrigen Formaldehyd-Konzentration überwiegend Vernetzungsreaktionen zwischen Proteinen und Oligopeptiden und ähnlichen Stoffen unter Bildung von N,N-Methylenbindungen zwischen Peptidbindungsarten $(-NH-\underset{\underset{O}{\|}}{C}-)$, Amidgruppen, $NH_2$-Gruppen der Purinbasen bzw. Nucleotide und Nucleoside einsetzen.

Die Biomassen-Kondensate, die nach den unter (a) bis (d) beschriebenen Methoden hergestellt werden, erweisen sich als unfiltrierbar. Die Biomassen-Kondensate können zwar durch Zentrifugierung isoliert werden, jedoch werden leimartige, stark klebrige Kondensate erhalten, die selbst nach schonender Trocknung bei 80 °C klebrig sind und nicht in pulvrige Form überführt werden können.

Setzt man Formaldehyd nach den unter (a) bis (d) beschriebenen Methoden mit

— Biomassen aus der Reihe der Essigbakterien (Acetobacter),
— Biomassen aus der Reihe der Bakterien der Ordnung der Eubacteriales (Escherichia coli),
— Biomassen mit eiweißzersetzenden Heubazillen (Bakterium subtilis),
— Biomassen mit Bakterien aus der Reihe der Chlamydobacteriales (Fadenbakterien),
— Biomassen mit Mikroorganismen aus der Reihe der Actinomycetales

um, so entstehen ebenfalls schleimartige Kondensationsprodukte, die nicht filtrierbar sind.

Verwendet man bei den vorstehend beschriebenen Umsetzungen nicht Formaldehyd, sondern

— Acetaldehyd,
— Glyoxal,
— Glutardialdehyd,
— Isobutyraldehyd,
— Chloralhydrat,
— Crotonaldehyd,
— Acrolein,
— Furfurol,
— Salicylaldehyd,
— Methyläthylketon,
— Cyclohexanon,

so werden wiederum jeweils nur unfiltrierbare Biomassen-Kondensate erhalten.

Beispiel 2

Kondensation von Pilz-Biomassen mit bestimmten Aldehyden bzw. Ketonen nach konventionellen Methoden

Jeweils 1 000 g einer

a) wäßrigen weiß-gelblichen, in Zersetzung befindlichen Bäckereihefekultur, enthaltend etwa 70 Gew.-Teile an Trockensubstanz,

b) weiß-gelblichen Biomassen-Dispersion einer kolloidalen käuflichen Jungzellen-Nährhefe-Kultur mit 20 Gew.-Teilen Trockensubstanz an Hefezellen,

c) einer bräunlichen, obergärigen Bierhefedispersion mit einem Trockengewicht von 50 Gew.-Teilen werden nach den im Beispiel 1 unter (a) bis (d) angegebenen Methoden in dem dort jeweils genannten Mengenverhältnis mit wäßriger Formaldehyd-Lösung umgesetzt.

In gleicher Weise werden die obengenannten Pilz-Biomassen nach den im Beispiel 1 unter (a) bis (d) angegebenen Methoden in dem dort jeweils genannten Mengenverhältnis mit

— Glyoxal,
— Isobutyraldehyd,
— Crotonaldehyd,
— Acrolein,
— Cyclohexanon

umgesetzt.

In allen Fällen erhält man unfiltrierbare Biomassen-Kondensate.

Beispiel 3

Kondensation von pflanzlichen und mikrobiellen Biomassen mit Formaldehyd nach konventionellen Methoden

a) jeweils 1 000 g eines wäßrigen, in mikrobiologischer Zersetzung befindlichen Gras-Homogenisates, — (Trokkensubstanz der Pflanzenzellen ca. 14 Gew.-%, Proteingehalt des Homogenisates ca. 12 Gew.-%, bezogen auf Trockensubstanz) —, dessen mikrobiologische Zersetzung aerob durch Fermentation unter Zusatz von einem g Gartenerde durch 48-stündige Erwärmung auf 35 °C eingeleiter wurde, werden nach den im Beispiel 1 unter (a) bis (d) angegebenen Methoden in dem dort jeweils genannten Mengenverhältnis mit wäßriger Formaldehyd-Lösung umgesetzt.

Man erhält jeweils eine äußerst schwierig filtrierbare Kondensat-Dispersion.

b) Führt man die in diesem Beispiel unter (a) beschriebene Kondensation an einem gleichen Pflanzenzellhomogenisat jedoch nach Fermentation mit mikrobiell aktiver Gartenerde unter anaeroben Bedingungen durch (Fermentationsdauer wie unter (a) angegeben), so wird nach der Umsetzung mit Formaldehyd jeweils eine äußerst schwierig zu filtrierende, klebrig antrocknende Kondensat-Dispersion erhalten.

Beispiel 4

Kondensation von Biomassen aus der Gruppe der Cyanophyceae, Blaualgen, Grünalgen, Flagellaten, Braunalgen, Diatomen, Mycomyceten und Amöben mit Formaldehyd nach konventioneller Methode

1 000 g eines Belebtschlammes mit einem Feststoffgehalt von 2 bis 3 Gewichtsprozent eines Gemisches von Biomassen aus der Reihe der Blaualgen, Grünalgen, die zusätzlich Flagellaten, die verschiedensten Algenarten und schleimige extrazelluläre Bestandteile enthalten, werden bei pH = 7,5 mit 100 g einer 30 %igen wäßrigen Formaldehyd-Lösung (1 Mol) im Verlaufe von 6 Stunden bei 100 °C kondensiert. Man erhält ein Biomassen-Kondensat, das sich noch schwieriger filtrieren läßt als die zugrunde-liegenden unbehandelten Biomassen.

Beispiel 5

Kondensation von cellulären Blutbestandteilen mit Formaldehyd

a) 500 g frisches Rinderblut, dessen Biomassen-Anteil hauptsächlich aus Erythrozyten (ca. 44 % des Zellvolumens) und Granulocyten, Lymphozyten, Thrombozyten (= ca. 1 % des Gesamtzellvolumens) besteht und dessen extracelluläre Bestandteile aus Blutplasma (6-8 % Proteine) und Blutserum bestehen, werden mit 500 g destilliertem Wasser verdünnt und anschließend mit 100 g einer 30 %igen wäßrigen Formaldehyd-Lösung (1 Mol) in Gegenwart von 25 g 85 %iger Phosphorsäure im Verlaufe von 4 Stunden bei 40 °C kondensiert.

b) In analoger Weise werden 500 g frisches Rinderblut der oben angegebenen Zusammensetzung nach dem Verdünnen mit 500 g destilliertem Wasser mit 25 g einer 30 %igen wäßrigem Formaldehyd-Lösung (0,25 Mol) in Gegenwart von 18,4 g konzentrierter Schwefelsäure im Verlaufe von 4 Stunden bei 40 °C kondensiert.

Man erhält in beiden Fällen durch gleichzeitige Bildung von schleimartigen Oligopeptiden und abgebauten Blutbestandteilen schwer filtrierbare, verklebende Kondensate.

Beispiel 6

1 000 g eines bei Normaldruck unfiltrierbaren Bakterienbelebtschlammes (Biomassen-Mycel) aus einer vollbiologisch arbeitenden Anlage zur Reinigung industrieller und kommunaler Abwässer (Zusammensetzung der Biomasse wie im Beispiel 1 angegeben ; Trockengewicht der Biomasse etwa 85 g) werden 10 Stunden bei 5 atü auf eine Drucknutsche gepreßt. Dadurch erhält man eine Biomasse mit einem Trokkengewicht von etwa 11,6 Gewichtsprozent. Die verwendeten Filter verstopfen schon nach kurzer Zeit.

a) 1 000 g des oben angegebenen unfiltrierbaren wäßrigen Biomassen-Mycels werden nach der in der DT-OS 25 23 483 beschriebenen Verfahrensweise mit 1 Mol einer alkalisch eingestellten Methylolharnstoff-Lösung, die zuvor in alkalischem Medium in 20 Minuten bei einer Temperatur von 30 °C in ein höhermolekulares Oligokondensat überführt wurde, in 200 g Wasser eine halbe Stunde bei einem pH-Wert von 3 kondensiert, wobei der pH-Wert durch Zugabe von Salzsäure eingestellt wurde. Man erhält ein schleimartiges, praktisch nicht filtrierbares Reaktionsgemisch.

b) 1 000 g des oben angegebenen unfiltrierbaren wäßrigen Biomassen-Mycels werden nach der in der DT-OS 25 23 483 beschriebenen Verfahrensweise mit 1 Mol einer alkalisch eingestellten Methylolharnstoff-Lösung, die zuvor in alkalischem Medium in 20 Minuten bei einer Temperatur von 80 °C in ein höhermolekulares Oligokondensat überführt wurde, in 200 g Wasser eine halbe Stunde bei einem pH-Wert von 5 kondensiert, wobei der PH-Wert durch Zugabe von Schwefelsäure eingestellt wurde. Man erhält ein schleimartiges, praktisch nicht filtrierbares Reaktionsgemisch.

Verlängert man bei den unter (a) und (b) angegebenen Kondensationen die Reaktionsdauer auf 4 Stunden, so erhält man ebenfalls nicht filtrierbare Biomassen-Dispersionen mit schleimartigem Charakter. Ebenso werden extrem unangenehme Geruchsträger nicht gebunden.

Erfindungsgemäße Beispiele

Beispiel 7

a) 1 000 g eines unter Normaldruck unfiltrierbaren bzw. in Drucknutschen extrem schwer filtrierbaren Bakterienbelebtschlammes mit einem Feststoffgehalt von etwa 8,5 Gew.-% aus einer vollbiologisch arbeitenden Kläranlage für kommunale und industrielle Abwässer werden in einem mit Rückflußkühler, Thermometer und Rührer bestückten Schliff-Rührbecherglas zunächst mit 0,2 Mol Formaldehyd 20 Minuten bei 60 °C kondensiert und dann mit 90 g (1 Mol) an einer frisch bereiteten Monomethylolharnstoff-Lösung (hergestellt durch Auflösen von 90 g Monomethylolharnstoff in 200 g Wasser) versetzt und 20 Minuten auf 80 °C erhitzt. Man läßt anschließend auf 50 °C abkühlen und katalysiert die Biomassen-Mischkondensation durch Zugabe von 18,4 g konzentrierter Schwefelsäure bei einem pH-Wert von ca. 1,9. Nach 1 bis 2 Stunden ist die Mischkondensation und Polymethylenharnstoffbildung beendet. Filtrationsproben zeigen, daß sich bereits nach 10 Minuten Kondensationsdauer ausgezeichnet filtrierbare, nicht-klebende Biomassen-Mischkondensat-Pulver bilden. Zur Aufarbeitung wird der Ansatz mit 14 g Calciumhydroxid neutralisiert, wobei sich schwerlösliches Calciumsulfat auf dem Biomassen-Mischkondensat niederschlägt. Man erhält ein hervorragend filtrierbares Biomassen-Mischkondensat, das nach dem Trocknen bei 80 °C unter vermindertem Druck in Form eines grauen Pulvers anfällt. Die Ausbeute beträgt 160 g an Biomassen-Mischkondensat. Es enthält 14,2 Gew.-% an Stickstoff und bedingt durch die kondensierten bzw. vernetzten Nucleinsäuren und phosphorhaltigen Zellinhaltsstoffe etwa 1,5 Gew.-% an Phosphor. Somit wird durch diese Umsetzung der Stickstoffgehalt der eingesetzten Biomasse um etwa 8 Gew.-% erhöht. Durch Behandlung des feuchten oder getrockneten, pulvrigen Biomassen-Mischkondensates mit einer 2 %igen wäßrigen Ammoniak-Lösung werden Spuren an Formaldehyd in Hexamethylentetramin umgewandelt und Spuren an Hydroxybuttersäuren entfernt. Man erhält ein praktisch geruchloses Pulver.

Biologische Prüfungen auf Sterilität der so erhaltenen Pulver wie auch der Filtrate zeigen, daß die Verfahrensprodukte wie auch die Mutterlaugen völlig frei von Bakterien, sporenbildenden Mikroorganismen und pathogenen Krankheitserregern sind.

Durch Einengung der Mutterlaugen unter vermindertem Druck werden etwa 18 g an wasserlöslichen Zellinhaltsstoffen, die aus Polysacchariden, Harnstoff-Formaldehydkondensaten und löslichen Zellreservestoffen bestehen, in gegenüber der Ausgangsfarbe der Biomassen stark aufgehellter Form isoliert. Bezogen auf eingesetzten Harnstoff gelangen bei der Ausführungsform dieses Beispiels ca. 93 Gew.-% Harnstoff und 93 Gew.-% Formaldehyd unter Bildung pulvriger Biomasse-Mischkondensate zur Reaktion.

Parallelversuche und analytische Untersuchungen an Biomassen-Mischkondensaten, die nach der in diesem Beispiel beschriebenen Verfahrensweise erhalten werden, zeigen aufgrund des Phosphorsäuregehaltes der Verfahrensprodukte, daß in 132 g des pulvrigen Biomassen-Polymethylenharnstoff-Mischkondensates etwa 27,6 g an kondensierten Ribonucleinsäuren, Desoxyribonucleinsäuren und phosphorhaltigen Zellinhaltsstoffen vorliegen (= ca. 21 Gew.-%).

b) Man verfährt in der unter (a) beschriebenen Weise, verwendet jedoch anstelle von Monomethylolharnstoff jetzt 1 Mol Monomethylolthioharnstoff.

Man erhält ein graues, pulvriges, leicht filtrierbares Biomassen-Mischkondensat in einer Ausbeute von 145 g.

N-Gehalt : 12,8 %
S-Gehalt : 17,2 %

Beispiel 8

Man verfährt nach der im Beispiels 7, Variante (a) beschriebenen Weise, setzt dabei die gleiche Art und Menge an Biomasse ein und führt zunächst eine Vorkondensation der Biomasse mit 100 g einer 30 %igen wäßrigen Formaldehyd-Lösung (1 Mol) in Gegenwart von 100 g 85 Gew.-%iger Phosphorsäure unter hydrolysierenden Bedingungen in einem Zeitraum von 4 Stunden bei 100 °C durch. Anschließend gibt man nach erfolgter Abkühlung auf 80 °C eine Lösung von 60 g Harnstoff (1 Mol) und 20 g wäßriger Formaldehyd-Lösung hinzu. Die kondensierende Pfropfung von Polymethylenharnstoffen an Biomassen setzt dabei sofort unter leichter Erwärmung ein. Nach 1-stündiger Kondensationsdauer erhält man ein pulvriges, durch Filtration leicht zu isolierendes Biomassen-Mischkondensat. Das Kondensat wird von nicht-gebundener Phosphorsäure durch mehrfaches Verrühren mit 2 %iger wäßriger Ammoniaklösung befreit und bei 80 °C im Vakuum getrocknet. Ausbeute : 136 g N-Gehalt = 18,5. Das Verfahrensprodukt besteht aus etwa 52 Gew.-% an kondensierter und sterilisierter Biomasse, etwa 2,9 Gew.-% Phosphor und etwa 45 Gew.-% an Harnstoff-Formaldehyd-Kondensaten (Polymethylenpolyharnstoffen).

In einer besonders vorteilhaften Variante dieses Verfahrens wird das Auswaschen der noch enthaltenden Phosphorsäure aus der Biomasse erspart, indem man nach Durchführung der Kondensation die freie Phosphorsäure durch Zugabe von Calciumhydroxid bzw. frisch hergestelltem Aluminiumhydroxid in schwerlösliches Calciumphosphat bzw. Aluminiumphosphat überführt. Durch Verwendung von überschüssigen Mengen an Calciumhydroxid werden wasserlösliche Oligosaccharide als schwerlösliche Calciumoxid-Oligosaccharid-Komplexe gefällt.

Beispiel 9

Man verfährt genau wie im Beispiel 7, Variante (a), beschrieben, führt jedoch die Kondensation bei einem Druck von 18 Torr bei einer Temperatur zwischen 55 und 60 °C durch, wobei kleine Mengen an Wasser abdestillieren. Durch diese Verfahrensweise werden Bakterienzellwände und cytoplastisches Material wesentlich schneller zerstört und angegriffen als bei der zuvor beschriebenen Methode. Bereits nach 45-minütiger Reaktionsdauer wird das Biomassen-Polymethylenharnstoff-Mischkondensat durch einfache Filtration in Pulverform isoliert.

Beispiel 10

Man verfährt genau wie im Beispiel 7, Variante (a) beschrieben, verwendet aber anstelle von 1 Mol Monomethylolharnstoff jetzt ein Gemisch aus 0,7 Mol Monomethylolharnstoff und 0,3 Mol Monomethylolthioharnstoff. — Nach dem Aufarbeiten erhält man 144 g eines Polymethylenharnstoff-Polymethylenthioharnstoff-Biomassen-Mischkondensates, das sich ausgezeichnet filtrieren läßt.

Dieses Mischkondensat bindet schädliche Schwermetall-Ionen wie die des Quecksilbers und des Bleis, und Metallionen, z.B. Ionen des Cadmiums, Nickels, Chroms und des Zinks, sehr fest und kann daher als Ionenaustauscher und als Schwermetall-Ionenfänger für Quecksilber und Blei enthaltende Abwässer verwendet werden. So beträgt z.B. in einem Abwasser, das einen Quecksilbergehalt von 50 ppm und einen Bleigehalt von 2 100 ppm aufweist, nach der Behandlung mit dem obengenannten Mischkondensat die Konzentration an Quecksilber nur noch 0,5 ppm und die an Blei nur noch 0,8 ppm.

Beispiel 11

Man verfährt genau wie in Beispiel 7, Variante (a) beschrieben, verwendet aber die gleiche Art und Menge an Biomasse aus einer vollbiologisch arbeitenden Kläranlage und ersetzt lediglich die wäßrige Monomethylolharnstoff-Lösung durch :

a) 1 Mol Dimethylolharnstoff, gelöst in 400 g Wasser,
b) 1 Mol Dimethylolthioharnstoff, gelöst in 400 g Wasser,
c) 1 Mol Trimethylolmelamin, gelöst in 400 g heißem Wasser,
d) 1 Mol Hexamethylolmelamin, gelöst in 600 g siedendheißem Wasser,
e) 1 Mol eines methylolierten Dicyandiamids aus 1 Mol Dicyandiamid und 2 Mol Formaldehyd,
f) 1 Mol Monomethyloloxamid in 200 g Wasser,
g) 1 Mol Monomethylol-äthylenharnstoff der Konstitution

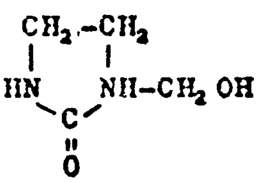

h) 1 Mol Monomethylol-äthylenthioharnstoff der Konstitution

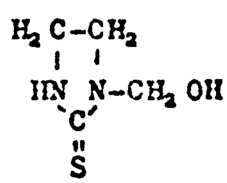

i) 1 Mol Tetramethylolacetylendiharnstoff der Konstitution

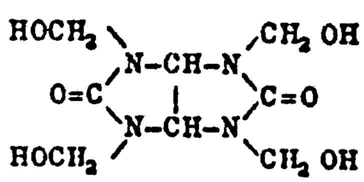

j) 0,5 Mol 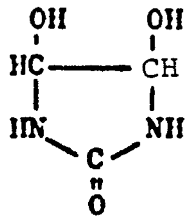 und 0,5 Mol Harnstoff.

Man erhält im Falle der Umsetzungen (a) bis (j) jeweils ein Biomassen-Mischkondensat, in dem von 85 g an eingesetzter Biomasse (Feststoffanteil) etwa 82-88 Gew.-% durch Mischkondensation mit den genannten Aminoplastbildnern und durch Eigenkondensation der genannten Aminoplastbildner gut filtrierbare, pulvrige Kondensate ergeben. Die restlichen 18-12 Gew.-% der eingesetzten Biomasse (Feststoffanteil) können als wasserlösliche Zellinhaltsstoffe durch Eindampfen der Mutterlaugen isoliert werden. Alle Verfahrensprodukte enthalten durch die Neutralisation der Schwefelsäure mit Calciumhydroxid etwa 22-27 g Calciumsulfat. Die Ausbeuten an Biomassen-Mischkondensaten betragen bei den Umsetzungen (a) bis (j) :

a) 175 g f) 158 g
b) 196 g g) 187 g
c) 255 g h) 202 g
d) 280 g i) 242 g
e) 198 g j) 149 g

Die Kondensat-Mischungen, die bei den Umsetzungen (a) bis (j) erhalten werden, stellen graue, lagerbeständige Pulver dar, in denen alle Enzyme der Biomassen durch Kondensation vollständig desaktiviert sind. Störende Geruchsbildungen sind selbst nach beliebig langen Lagerzeiten nicht zu beobachten.

Bei der Durchführung der obigen Mischkondensationen läßt sich die bei den Umsetzungen (a) bis (j) jeweils zur Acidifizierung verwendete Schwefelsäure auch durch jeweils 25 g an wäßriger 85 %iger Phosphorsäure ersetzen. Hierbei erhält man nach der Neutralisation der Ansätze mit Calciumoxid oder Calciumhydroxid eine Fällung von schwerlöslichem Calciumphosphat in einer Menge von 22 bis 28 g gleichmäßig auf die Biomassen-Mischkondensate verteilt.

## Beispiel 12

Man verfährt genau wie in Beispiel 7, Variante (a) beschrieben, verwendet aber nur jeweils 100 g der dort beschriebenen Biomasse, 1,9 g Schwefelsäure und jeweils eine der folgenden zur Aminoplastbildung befähigten Mischungen :

a) 0,01 Mol eines mit 4 Mol Formaldehyd methylolierten Hexamethylendiharnstoffs der Konstitution

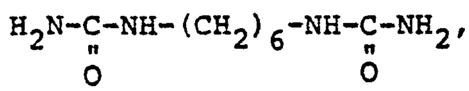

und 0,09 Mol Monomethylolharnstoff.

b) 0,1 Mol des methylolierten Diurethans der Konstitution

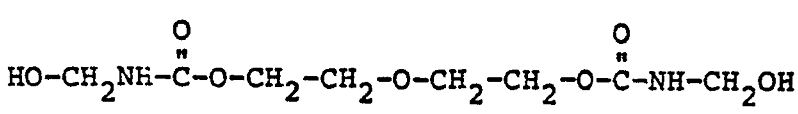

und 0,01 Mol Monomethylolharnstoff.

d) 0,01 Mol Tetramethylolhydrazodicarbonamid der Konstitution

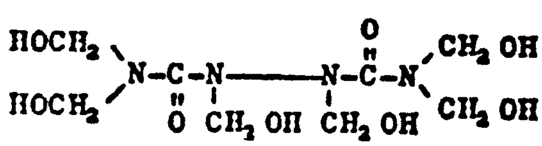

und 0,09 Mol Dimethylolharnstoff

d) 0,01 Mol eines mit 4 Mol Formaldehyd permethylolierten Adipinsäurediamids und 0,09 Mol Monomethylolharnstoff.

e) 0,01 Mol eines mit 4 Mol Formaldehyd permethylolierten Sulfonsäurediamids der Konstitution

$$H_2NO_2S.(CH_2)_4-SO_2NH_2$$

und 0,09 Mol Monomethylolharnstoff.

f) 0,01 Mol eines mit 4 Mol Formaldehyd methylolierten Phosphorsäuretriamids der Konstitution

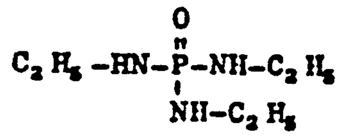

und 0,09 Mol Monomethylolharnstoff.

g) 0,01 Mol Calciumcyanamid, 0,03 Mol Formaldehyd und 0,09 Mol Monomethylolharnstoff.

h) 0,01 Mol eines permethylolierten, höhermolekularen, Urethangruppen enthaltenden α, ω-Diharnstoffes und 0,09 Mol Monomethylolharnstoff in 100 g Wasser. — Der benötigte α, ω-Diharnstoff wird hergestellt durch Umsetzung von 1 Mol eines α, ω-Dihydroxypolyäthylenoxids vom Durchschnittsmolekulargewicht 1 500 mit 2 Mol Hexamethylendiisocyanat, anschließende Umsetzung des NCO-Prepolymeren mit überschüssigem, wäßrigen Ammoniak und Methylolierung mit 4 Mol Formaldehyd unter Verwendung von 0,5 g Kaliumcarbonat als Methylolierungskatalysator.

i) 0,025 Mol Trimethylolmelamin und 0,05 Mol Monomethylolharnstoff.

Man erhält im Falle der Umsetzungen (a) bis (i) leicht filtrierbare Biomassen-Mischkondensate mit den genannten Aminoplast-bildnern. Bei den Produkten handelt es sich um graue, nahezu geruchlose Pulver.

Die Ausbeuten an Biomassen-Mischkondensaten betragen bei den Umsetzungen (a) bis (i) :

a) 18,5 g f) 15,8 g
b) 17,1 g g) 16,4 g
c) 17,5 g h) 32,3 g
d) 16,1 g i) 19,5 g
e) 16,9 g

## Beispiel 13

Die erfindungsgemäße Umsetzung der im Beispiel 7 beschriebenen Biomasse läßt sich in vereinfachter Weise dadurch erreichen, daß man die Bildung des Monomethylolharnstoffs in der Biomasse in situ aus 1 Mol Harnstoff und 1 Mol Formaldehyd (30 %ige Formalin-Lösung) durchführt, und zwar

a) durch basische Katalyse der Methylolierung mit 0,3 g Kaliumcarbonat,
b) durch basische Katalyse der Methylolierung mit 0,4 g Calciumhydroxid,
c) durch basische Katalyse der Methylolierung mit 0,5 g Triäthylenamin,
d) durch thermische Kondensation ohne zusätzlichen Katalysator.

Man verfährt bei den Umsetzungen (a) bis (d) genau nach der im Beispiel 7, Variante (a) beschriebenen Methode, mischt aber alle Reaktionskomponenten ohne vorhergehende N-Methylolierung der Aminoplastbildner. Anschließend erfolgt die Temperaturführung und sauer katalysierte Kondensation genau wie im Beispiel 7, Variante (a) offenbart.

Die Ausbeuten an Biomassen-Mischkondensat betragen bei den Umsetzungen (a) bis (d) :

a) 161 g N-Gehalt : 14,3 % c) 159 g N-Gehalt : 14,5 %
b) 160 g N-Gehalt : 14,1 % d) 158 g N-Gehalt : 14,2 %

## Beispiel 14

Man verfährt genau wie in Beispiel 7, Variante (a) beschrieben unter Verwendung eines Bakterienbelebtschlammes der vollbiologischen Abwasserreinigung — das heißt mit einer Biomasse, welche verschiedenartige mikrobielle Zusammensetzungen enthält —, führt die Kondensation jedoch bei nur 35 °C durch und setzt vor der Durchführung der Mischkondensation jeweils 100 g von einem der folgenden pigmentartigen, schwerlöslichen, pulvrigen Trägerstoffe zu :

a) 100 g eines Polymethylenharnstoffes und 400 g Wasser,
b) 100 g eines Äthyliden-polyharnstoffes und 400 g Wasser,
c) 100 g eines Polyisobutylidenpolyharnstoffes und 400 g Wasser,
d) 100 g eines schwerlöslichen Kondensates aus 2 Mol Harnstoff und 1 Mol Crotonaldehyd und 400 g Wasser,

e) 20 g Quarzsand und 80 g eines Polymethylenpolyharnstoffes und 400 g Wasser,

f) 20 g Tonerdehydrat (= Aluminiumoxidhydrat) und 80 g eines Isobutylidenpolyharnstoffes und 400 g Wasser,

g) 30 g Calciumphosphat und 70 g eines Polyisobutylidenpolyharnstoffes und 400 g Wasser,

h) 30 g Aluminiumsilikat und 70 g eines Polyisobutylidenharnstoffes und 400 g Wasser.

Die saure Kondensation wird genau wie in Beispiel 7 mit Schwefelsäure als Katalysator bei Raumtemperatur durchgeführt.

Man erhält im Falle der Umsetzungen (a) bis (h) ausgezeichnet filtrierbare Biomassen-Mischkondensate, die mit den zugesetzten Trägerstoffen innig vermischt sind.

Die Ausbeuten betragen bei den Umsetzungen (a) bis (h) :

a) 262 g Stickstoffgehalt : 22,6 %
b) 259 g Stickstoffgehalt : 21,4 %
c) 264 g Stickstoffgehalt : 18,4 %
d) 265 g Stickstoffgehalt : 22,7 %
e) 263 g
f) 260 g
g) 258 g
h) 266 g

## Beispiel 15

Man verfährt mengenmäßig genau nach den im Beispiel 7, Variante (a) enthaltenden Angaben, führt die Kondensation jedoch bei Raumtemperatur im Verlaufe von 8 Stunden durch. Anschließend wird das durch Filtration isolierte Biomassen-Mischkondensat bei 110 °C getrocknet. Hierbei tritt eine vollständige Sterilisation des Verfahrensproduktes ein. In der Mutterlauge ist nur eine sehr geringe Menge an Zellinhaltsstoffen vorhanden.

Man erhält ein Biomassen-Mischkondensat in einer Ausbeute von 175 g ; Stickstoffgehalt : 18,4 %.

## Beispiel 16

Man verfährt genau wie in Beispiel 7, Variante (a) beschrieben, führt die Mischkondensation jedoch bei 75 °C aus. Nach erfolgter Kondensation und Neutralisation des Reaktionsgemisches mit Ammoniak und Abkühlung auf 35 °C werden 2 g Dextrinpulver und 3 g einer Fleischextrakt-Nährstofflösung zugegeben. Man rührt anschließend 48 Stunden bei 35 °C, um Sporen bildende Mikroorganismen wieder zur Keimbildung und Zellteilung anzuregen. Anschließend wird unter Zusatz von 20 g einer 30 %igen Formalin-Lösung 2 Stunden- auf 80 °C erhitzt. — Nach Filtration und Trocknung erhält man 165 g an Mischkondensat mit einem Stickstoff-Gehalt von 18,5 %.

Die gekoppelte Pasteurisierung und Sterilisation durch die verwendeten Reagenzien führt zu einem völlig keimfreien Mischkondensat.

## Beispiel 17

a) 1 000 g eines aus einer vollbiologisch arbeitenden Kläranlage für industrielle und kommunale Abwässer stammenden, etwa 8,5 % Feststoff enthaltenden Bakterienbelebtschlammes, der aus verschiedensten Mikroorganismen besteht und mit Spuren an den Pflanzenschutzmitteln (Herbiziden)

N-Methyl-isopropyl-carbamat (0,5 g),
4-Amino-6-tert.-butyl-3-methylthio-4,5-dihydro-1,2,4-triazin-5-on (0,5 g),
N-(3-Benzthiazolyl)-N,N'-dimethylharnstoff (0,5 g),

verunreinigt ist, werden in einem Schliffbecherglas unter intensivem Rühren zunächst mit 100 g einer 30 %igen Formalin-Lösung (1 Mol) und 25 g 85 %iger Phosphorsäure auf 80 °C erwärmt. Hierbei werden Bakterienzellwände gesprengt und die enthaltenden Pflanzenschutzmittel durch Reaktion ihrer $NH_2$- bzw. NH-Funktionen mit Formaldehyd unter N-Methylolierung ($>N-CH_2-O-CH_2-N<$) bzw. Methylenverknüpfung ($>N-CH_2-N<$) desaktiviert und hydrolysiert. — Nach dieser Primärreaktion werden Proben entnommen und zentrifugiert. Durch Titration des Formaldehyds in den Filtraten wird analytisch ermittelt, daß 0,05 Mol an Formaldehyd verbraucht worden sind. — Anschließend werden zu dem Reaktionsgemisch eine Lösung von 60 g Harnstoff (1 Mol) in 100 g Wasser sowie 10 g einer 30 %igen Formalin-Lösung (0,1 Mol) gegeben. Man läßt 15 Minuten bei 70 °C kondensieren, kühlt dann innerhalb von 30 Minuten auf 45 °C ab und erhält ein leicht filtrierbares, pulvriges Biomassen-Mischkondensat. Dieses Biomassen-Mischkondensat wird mit Calciumhydroxid neutralisiert, wobei sich schwerlösliches Calciumphosphat in feinst verteilter Form in der Biomassen-Kondensat-Dispersion niederschlägt. Man filtriert das pulvrige Produkt ab, wäscht mit einer 2 %igen wäßrigen Ammoniak-Lösung, trocknet das Produkt anschließend bei 70 °C unter vermindertem Druck und erhält so ein nahezu geruchloses Pulver in einer Ausbeute von 176 g ; der Stickstoffgehalt beträgt 13,4 %.

Das Verfahrensprodukt enthält, — bezogen auf die Mischung von kondensierten Proteinen, Enzymen, Nucleinsäuren und anderen Zellinhaltsstoffen —, etwa 39 Gew.-% an Polymethylenharnstoffen

der idealisierten Konstitution

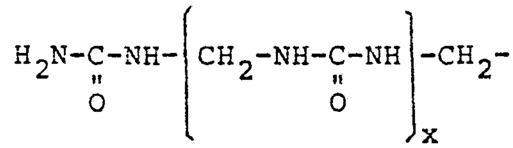

(=K)

wobei x unbekannt ist und der an funktionelle Gruppen der Biomasse ankondensierte Anteil von (K) infolge der Unlöslichkeit des Biomassen-Mischkondensates zur Zeit analytisch nicht ermittelt werden kann.

Biologische Prüfungen auf Sterilität der pulvrigen Biokondensate wie auch der Filtrate zeigen, daß die pulvrigen Verfahrensprodukte und die Filtrate frei von Bakterien und pathogenen Krankheitserregern sind.

b) Man verfährt genau wie unter (a) beschrieben, ersetzt aber die als Katalysator verwendete Phosphorsäure durch 18,4 g an konzentrierter Schwefelsäure. Nach Durchführung der Kondensation wird mit Calciumhydroxid neutralisiert. Man isoliert ein Calciumsulfat enthaltendes Biomassen-Mischkondensat. Ausbeute : 171 g.

Beispiel 18

a) Man verwendet wie in Beispiel 17 beschrieben eine Menge von 865 g des dort genannten Bakterienbelebtschlammes, welcher etwa 73,8 g Trockensubstanz enthält, erhitzt diese Biomasse in Gegenwart von 0,1 Mol Formaldehyd unter intensivem Rühren 30 Minuten lang auf 70 °C, fügt dann 63 g Melamin hinzu und versetzt nach weiteren 5 Minuten mit 150 g einer 30 %igen wäßrigen Formalin-Lösung (1,5 Mol) sowie mit 0,2 g Kaliumcarbonat als Methylolierungskatalysator. Hierbei erzeugt man in der Biomasse innerhalb einer halben Stunde im wesentlichen das Trimethylolmelamin der Konstitution

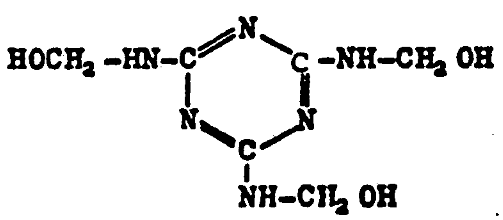

Anschließend kühlt man auf 45 °C ab. Nachdem die Innentemperatur der noch unfiltrierbaren Biomassen-Dispersion 45 °C erreicht hat, fügt man 18 g konzentrierte Schwefelsäure hinzu. Die Biomassen-Trimethylolmelamin-Kondensation setzt bei einem pH-Wert von ca. 2,5 sofort ein. Man rührt 2 Stunden nach und neutralisiert mit 24 g Calciumhydroxid. Das Biomassen-Mischkondensat wird abfiltriert und fällt nach der Trocknung bei 70 °C im Vakuum in einer Ausbeute von 184 g an. Stickstoffgehalt des Biomassen-Mischkondensates : 28,1 %. Der Stickstoffgehalt des Biomassen-Mischkondensates ist also von ca. 7,4 Gew.-%, — bezogen auf Trockengewicht des Ausgangsproduktes —, auf 28,1 % angehoben worden, d.h. um einen Wert von $\Delta = +20{,}7\ \%$ erhöht. Der Phosphorgehalt des Mischkondensates beträgt 2 %, der Calcium-sulfat-Anteil ca. 12,8 Gew.-%.

Nach der Durchführung der erfindungsgemäßen Mischkondensation wird in einem Parallelversuch der wäßrige Überstand über dem Biomassen-Mischkondensat durch Zentrifugierung als fast farblose bzw. nur leicht gelbstichige Flüssigkeit isoliert. Beim Eindampfen dieser wäßrigen Phase bei einem Druck von 14 Torr verbleiben etwa 7 g eines Rückstandes, der im wesentlichen aus Zellinhaltsstoffen, wie Polysacchariden und Oligopeptiden bzw. Glykoproteiden unbekannter Konstitution besteht und sich als wertvoller Nährstoff für die verschiedensten mikrobiellen System eignet. Bei dieser Variante des erfindungsgemäßen Verfahrens wird somit nur eine relativ geringe Menge an Zellinhaltsstoffen in die Mutterlauge überführt.

b) Man verfährt genau wie unter (a) beschrieben, mit dem Unterschied, daß die eingesetzte Trimethylolmelaminmenge verringert wird, indem man 31,5 g Melamin (0,25 Mol) und 75 g einer 30 %igen Formalinlösung verwendet (0,75 Mol). Ansonsten werden die Kondensationsbedingungen eingehalten, die unter (a) beschrieben sind. Man gewinnt ein ausgezeichnet filtrierbares Biomassen-Mischkondensat.

Ausbeute : 122 g ; N-Gehalt : 23,3 %
P-Gehalt : 2,8 %

Das erhaltene Produkt bindet Ionen von Schwermetallen, wie Blei, Quecksilber und Kupfer, ferner

Ionen von Metallen wie Zink, Cadmium und Chrom.

c) Man verfährt genau wie unter (b) beschrieben, setzt jedoch lediglich 15,75 g (0,125 Mol) Melamin und 37,5 g einer wäßrigen 30 %igen Formalin-Lösung (0,375 Mol entsprechend 11,2 g Formaldehyd) ein. — Man erhält ein pulvriges Biomassen-Mischkondensat in einer Ausbeute von 100 g.

Bei dieser Ausführungsform des erfindungsgemäßen Verfahrens werden etwa 21 g an dreidimensional vernetzten Polymethylenmelaminen der idealisierten Konstitution

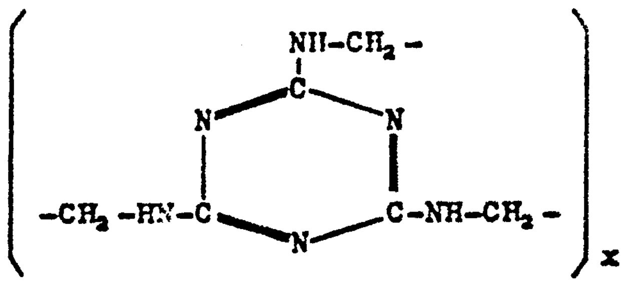

(=M)

mit unbekannter Größe von x erzeugt. Sie wandeln etwa 74 g der verwendeten Ausgangsbiomasse durch Mischkondensation ihrer reaktiven Zentren in pulvrige Produkte um. Infolge der Unlöslichkeit der Biomassen-Mischkondensate ist der Anteil von (M), der an Biomassen fixiert ist, nicht bestimmbar. Das Filtrat des Ansatzes zeigt eine bemerkenswert helle Farbe. Aus der Mutterlauge werden etwa 9 Gew.-%, — bezogen auf eingesetzte Biomasse —, an hellen Polysaccharid- und Oligopeptid-Kondensaten, d.h. nicht vernetzten Zellinhaltsstoffen, durch Einengen der Mutterlaugen isoliert.

Stickstoffgehalt des Biomassen-Trimethylolmelamin-Mischkondensates : 15,5 %.

d) Verwendet man gemäß Ausführungsform (c) lediglich 0,03 Mol Melamin (3,78 g) und zur Trimethylolmelaminbildung 0,09 Mol Formaldehyd (9 g 30 %iger Formaldehydlösung) und verfährt wie unter (a) beschrieben, so wird ebenfalls ein flockbares, filtrierbares Biomassen-Mischkondensat erhalten. Ausbeute : 79 g ; Stickstoffgehalt : 8,4 % ; P-Gehalt : 3,9 %.

e) Man verfährt wie unter (a) beschrieben, verwendet aber eine Mischung von Aminoplastbildnern folgender Art : 6 g Harnstoff (0,1 Mol), 11,4 g Azulminsäure, — hergestellt gemäß DT-PS 662 338 —, 1,26 g Melamin (0,01 Mol) und 18,4 g konzentrierte Schwefelsäure als Katalysator. Man kondensiert, neutralisiert anschließend mit Calciumoxid und arbeitet, wie in Beispiel 16 beschrieben, auf. Ausbeute : 121 g ; Stickstoffgehalt : ca. 11,7 Gew.-%.

Durch die Anwesenheit der mitkondensierenden Azulminsäure werden Ionen von Metallen wie Quecksilber, Blei, Cadmium, Zink, Kupfer, Nickel, Eisen, Chrom und Mangan im Biomassen-Mischkondensat fest gebunden. Selbst ein Biomassen-Mischkondensat, das einen Gehalt von 43 ppm Quecksilber, 480 ppm Zink, 320 ppm Kupfer, 2 % Eisen, 216 ppm Nickel, 230 ppm Chrom, 0,21 % Blei und 440 ppm Mangan aufweist, wirkt sich im Pflanzenkeimtest nicht negativ aus.

f) Man verfährt wie unter (e) beschrieben, setzt jedoch eine nach bekannten technischen Verfahren hergestellte bereits bei 110 °C getrocknete Biomasse ein, in der weitgehender Zelltod eingetreten ist. Ausbeute : 119 g an pulverförmiger Substanz.

g) Man verfährt wie unter (e) beschrieben, setzt aber die in (f) verwendete, jedoch 2 Monate auf einer Deponie gelagerte, mit Pilzen und anderen Mikroorganismen befallene Biomasse ein. Man erhält ein pulvriges Biomassen-Mischkondensat in einer Ausbeute von 112 g. Das Produkt ist in der Lage, Metallionen sehr fest zu binden.

h) Beim Zusatz von 6 g Thioharnstoff zu dem unter (a) angegebenen Reaktionsgemisch wird ebenfalls ein Biomassen-Mischkondensat erhalten, das in der Lage ist, Ionen der Metalle Quecksilber, Blei, Kupfer, Chrom, Zink und Cadmium zu binden.

## Beispiel 19

Eintopfverfahren, bei dem N-Methylolverbindungen der Biomasse und des Melamins nicht in isolierter Form eingesetzt, sondern in situ nach primärer Vorkondensation der Biomasse mit Aldehyd erzeugt werden und anschließend sauer kondensiert wird.

1 100 g eines aus einer vollbiologisch arbeitenden Kläranlage für industrielle und kommunale Abwässer stammenden, etwa 11,2 Gew.-% Feststoff enthaltenden Bakterienbelebtschlammes, der aus verschiedensten Mikroorganismen besteht, werden zunächst zur Verbesserung der Rührbarkeit mit 200 g Wasser versetzt. Hierauf werden 100 g einer 30 %igen wäßrigen Formaldehyd-Lösung (1 Mol) zugegeben. Anschließend wird 2 Stunden bei pH = 7,5 in Abwesenheit von Melamin bei 96 °C kondensiert. Danach gibt man 63 g (0,5 Mol) pulvriges Melamin und zusätzlich 50 g 30 %iger Formalinlösung (0,5 Mol) hinzu und kondensiert eine Stunde bei 96 °C. Man läßt auf 45 °C abkühlen, versetzt das Reaktionsgemisch mit 1,84 g konzentrierter Schwefelsäure und hält 4 Stunden unter intensivem Rühren auf dieser Temperatur. Man neutralisiert mit Calciumhydroxid und erhält ein sehr leicht filtrierbares Biomassen-Mischkondensat. Ausbeute des getrockneten Produktes : 195 g ; Stickstoffgehalt des Mischkondensates : 27,3 % ; Calcium-

sulfat-Anteil ca. 2,5 Gew.-%.

Im biologischen Test erweist sich das Produkt als völlig keimfrei. Durch Eindunsten der wäßrigen Mutterlauge werden etwa 10 g an polysaccharid- und peptidartigen Zellinhaltsstoffen isoliert, ebenso etwa 0,9 g an Hydroxybuttersäuren als Bestandteile von zerstörten Bakterienzellwänden.

Beispiel 20

a) Man verfährt wie in Beispiel 17, Variante (a) beschrieben, erhöht jedoch die Biomassen-Menge auf 2 000 g und setzt neben 200 g 30 %iger Formalinlösung (2 Mol) auch 200 g an 85 %iger Phosphorsäure ein. Man erwärmt zwei Stunden auf 80 °C, wobei eine Vorkondensation unter hydrolyisierenden, proteinabbauenden Bedingungen durchgeführt wird. Anschließend fügt man 40 g 30 %ige Formaldehydlösung und zuletzt 120 g Harnstoff (2 Mol) zu, wobei die Mischkondensation sofort einsetzt. Man läßt im Verlaufe von 2 Stunden auf 45 °C erkalten und neutralisiert dann mit Calciumhydroxid. Nach erfolgter Filtration und Trocknung erhält man ein Biomassen-Mischkondensat in einer Ausbeute von 525 g. Dieses Kondensat besitzt infolge der Bildung von unlöslichem Calciumhydrogenphosphat bei der Neutralisation der großen menge an Phosphorsäure, die als Katalysator verwendet wurde, einen Calciumphosphat-Anteil von etwa 46,6 Gew.-% und einen Stickstoffgehalt von 13,8 Gew.-%.

b) Verwendet man eine Menge von nur 20 g an 85 %iger Phosphorsäure als Katalysator und kondensiert genau wie unter (a) beschrieben, indem man die primäre Formaldehydkondensation 8 Stunden bei 98 °C bei einem pH-Wert von 3,4 durchführt, also unter Bedingungen arbeitet, unter denen Proteine, Zellwände und Zellinhaltsstoffe hydrolysieren, so erhält man nach dem Filtrieren und Trocknen ein Biomassen-Mischkondensat in einer Ausbeute von 341 g. Der Calciumhydrogenphosphat-Gehalt beträgt etwa 7,2 Gew.-% ; der Stickstoffgehalt beträgt 17,8 Gew.-%.

Beispiel 21

Durchführung des erfindungsgemäßen Verfahrens bei Raumtemperatur.

971 g eines etwa 7,4 Gew.-% Trockensubstanz enthaltenden Bakterienbelebtschlammes, dessen mikrobielle Bestandteile noch in voller Aktivität sind, werden mit 100 g Wasser und 100 g einer 30 %igen Formalinlösung (1 Mol Formaldehyd) bei Raumtemperatur ohne Zusatz eines basischen Methylolierungskatalysators 90 Minuten vorkondensiert. Anschließend werden 60 g Harnstoff (1 Mol) in der Dispersion gelöst, zusätzlich 10 g Formalinlösung (30 %ig) zugegeben, und nach 10 Minuten werden 18,4 g Schwefelsäure als saurer Kondensationskatalysator zugesetzt. Man kondensiert 4 Stunden bei Raumtemperatur unter sauren Bedingungen (pH-Wert = 2,3). Anschließend wird das Biomassenmischkondensat filtriert, durch Waschen mit Wasser von Schwefelsäure befreit und bei 80 °C unter einem Druck von 14 Torr getrocknet. Man erhält 133 g eines pulvrigen Biomassen-Mischkondensates, das einen Stickstoffgehalt von 24,7 % besitzt.

Beispiel 22

Die erfindungsgemäße Biomassen-Mischkondensation kann auch so durchgeführt werden, daß man in der ersten Stufe eine relativ große Menge an Aldehyd zugibt, wobei vorwiegend Methylolierungsprodukte von Proteinen sowie N-Methylolierungsprodukte von Nucleinbasen innerhalb der Makromoleküle der Nucleinsäuren erhalten werden, die in der zweiten Stufe mit den jeweiligen Aminoplastbildnern unter sauren Bedingungen kondensiert werden.

1 093 g eines aus einer biologisch arbeitenden Kläranlage für industrielle und kommunale Abwässer stammenden, etwa 11,2 Gew.-% Feststoff enthaltenden Bakterienbelebtschlammes, dessen mikrobielle Bestandteile noch eine hohe Aktivität aufweisen, werden mit 150 g Wasser und 200 g einer 30 %igen wäßrigen Formaldehyd-Lösung (2 Mol) 2 Stunden bei 98 °C umgesetzt. Anschließend wird auf Raumtemperatur abgekühlt. Zu diesem Zeitpunkt ist die methylolierte Biomasse frei von phatogenen Krankheitserregern, jedoch läßt sich eine Isolierung der N-Methylolierungsprodukte durch Filtration nicht durchführen. Man fügt eine Lösung von 120 g Harnstoff (2 Mol) in 200 g Wasser zu und versetzt mit 18,4 g konzentrierter Schwefelsäure. Es wird 6 Stunden bei Raumtemperatur bei pH = 2,3 kondensiert und dann mit Calciumhydroxid neutralisiert. Man erhält ein pulvriges, gut filtrierbares Biomassen-Mischkondensat. Ausbeute nach der Trocknung bei 80 °C : 257 g ; Stickstoffgehalt des Kondensates : 22,7 % ; Calciumsulfat-Gehalt : 9,4 %.

Biologische Prüfungen zeigen, daß dieses Biomassen-Mischkondensat steril und frei von phatogenen Krankheitserregern ist.

Beispiel 23

1 000 g einer aus einer biologisch arbeitenden Kläranlage stammenden, etwa 8,4 Gewichtsprozent Trockensubstanz enthaltenden Biomasse, die sich im Zustand reger Zellteilung befindet, werden mit 18,4 g 85 %iger Phosphorsäure versetzt und 2 Stunden auf 96 °C erhitzt. Hierbei wird Kohlendioxid in einer Menge von 2,2 g durch ablaufende Zersetzungsvorgänge entbunden. Das entweichende Kohlendio-

xid wird in einer mit Natronlauge beschickten Vorlage aufgefangen und nach der Bariumcarbonat-Methode titrimetrisch bestimmt. Nachdem die Kohlendioxid-Entwicklung innerhalb von 3 Stunden auf den Nullwert abgesunken ist, verfährt man wie folgt :

Zu 1 000 g der vorgenannten Biomasse fügt man 100 g an schwarzer Rohazulminsäure, — hergestellt gemäß DT-PS 662 338 —, und 100 g einer 30 %igen wäßrigen Formalin-Lösung (1 Mol) hinzu. Man kondensiert 8 Stunden bei 90 °C, wobei 1,36 g (0,03 Mol) Kohlendioxid entbunden werden. Diese Kohlendioxid-Entwicklung ist auf die Bildung von $F_2$-Fehlerstellen durch Decarboxylierung von $F_1$-Fehlerstellen in der Azulminsäure zurückzuführen. Es werden etwa 0,87 Gew.-% an $F_2$-Fehlerstellen der Konstitution

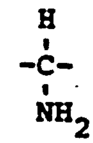

und etwa 9 Gew.-% an $F_1$-Fehlerstellen der Konstitution

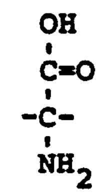

in der Azulminsäure erzeugt und freie Aminogruppen trotz der Unlöslichkeit der Azulminsäure mit dem eingesetzten Formaldehyd praktisch quantitativ unter Aminalbildung kondensiert.

Anschließend wird die Biomassen-Azulminsäure-Kondensat-Mischung auf 80 °C abgekühlt und mit 60 g (1 Mol) Harnstoff versetzt. Nach 10 Minuten werden in einem Guß 100 g einer 30 %igen Formalinlösung (1 Mol) zugegeben und anschließend werden 18,4 g 85 %ige Phosphorsäure hinzugefügt. Man kondensiert 2 Stunden bei 80 °C und erhält ein ausgezeichnet filtrierbares Biomasse-Azulminsäure-Harnstoff-Formaldehyd-Kondensat. Ausbeute nach dem Waschen mit 2 %iger wäßriger Ammoniaklösung und nach Trocknung bei 80 °C unter einem Druck von 15 Torr : 288 g ; N-Gehalt : 24,2 % ; Phosphorgehalt : 1,3 %.

Beim Eindampfen der Mutterlauge werden nur 8 Gew.-% an Polysaccharid- und anderen Zellinhaltsstoffen isoliert.

Im übrigen ist die kondensierte Azulminsäure bei der Umsetzung vollständig gegenüber Blausäure-Abspaltung stabilisiert worden.

Die in der Ausgangsbiomasse vorhandenen Metallmengen von 0,0043 % Quecksilber, 0,0005 % Cadmium, 0,042 % Zink, 0,032 % Kupfer, 1,8 % Eisen, 0,022 % Nickel, 0,03 % Chrom und 0,19 % Blei sind in dem erhaltenen Biomassen-Mischkondensat fest gebunden. Dies zeigt sich daran, daß der Metallgehalt in der Mutterlauge (= Abwasser) des obigen Biomassen-Kondensat-Ansatzes nach der erfindungsgemäßen Umsetzung um den Faktor $10^2$ bis $10^3$ gesunken ist. — Im einzelnen wurden folgenden Metallkonzentrationen im Abwasser gefunden :

0,25 mg Zink pro Liter
0,13 mg Blei pro Liter
weniger als 0,1 . $10^{-6}$ g Chrom pro Liter
weniger als 0,1 . $10^{-6}$ g Kupfer pro Liter
weniger als 0,1 . $10^{-6}$ g Quecksilber pro Liter

In 100 g der eingesetzten Biomassen waren etwa 43 mg Quecksilber enthalten ; davon gelangen nach der erfindungsgemäßen Umsetzung nur 0,0001 mg in das Abwasser.

Beispiel 24

Man verwendet 1 000 g einer aus einer vollbiologisch arbeitenden Kläranlage stammenden, etwa 8,4 Gew.-% an Trockensubstanz enthaltenden Biomasse, die aus den verschiedensten Mikroorganismen besteht. Diese Biomasse ist durch 3-tägige Lagerung bei Raumtemperatur ohne weitere Nährstoffzufuhr bereits in starker Zersetzung begriffen, wobei sich übelriechende Geruchsträger gebildet haben. Man rührt in diese wäßrige Biomassen-Aufschlämmung 100 g einer mit Formaldehyd kondensierten und gegenüber Blausäure-Rückspaltung stabilisierten Azulminsäure ein. Danach fügt man 100 g 30 %ige Formalin-Lösung hinzu, erhitzt eine halbe Stunde auf 80 °C, gibt anschließend 60 g (1 Mol) Harnstoff und nach weiteren 10 Minuten 25 g 85 %ige Phosphorsäure hinzu. Es wird 0,5 Stunden bei 80 °C kondensiert, dann auf 50 °C abgekühlt und filtriert.

Man erhält ein pulvriges, schwarz-graues Biomassen-Mischkondensat ; Ausbeute nach dem Trocknen bei 80 °C unter einem Druck von 16 Torr : 291 g ; Stickstoffgehalt 23,9 % ; Phosphorgehalt : 1,35 %.

Das Produkt erweist sich im biologischen Test als völlig keimfrei. Es ist nahezu geruchlos. Völlige Geruchlosigkeit wird durch Nachwaschen mit wenig Aceton oder Methanol erreicht.

## Beispiel 25

1 000 g eines aus einer biologisch arbeitenden Kläranlage für industrielle und kommunale Abwässer stammenden, etwa 8,4 Gew.-% Feststoff enthaltenden Bakterienbelebtschlammes, der sich in Zellteilung befindet, wird für eine anionisch katalysierte Blausäurepolymerisation eingesetzt.

Dazu gibt man zu der gerührten Biomasse 163 g Blausäure, 440 ml Wasser, 25 ml einer 25 %igen wäßrigen Ammoniak-Lösung und 5 g Natriumcyanat hinzu. Diese Mischung wird 7 Stunden bei 70-90 °C gerührt, wobei die Blausäure zu Azulminsäure polymerisiert. Danach zieht man noch enthaltenen Ammoniak und Cyanwasserstoff im Wasserstrahlvakuum ab. Hierbei schäumt die Polymerisat-Biomassen-Dispersion sehr stark auf.

Nach der Entfernung monomerer Blausäure werden 120 g 30 %ige Formaldehyd-Lösung zugesetzt, und es wird eine Stunde bei 100 °C gerührt. Anschließend wird auf 40 °C abgekühlt. Nunmehr werden 120 g Harnstoff (2 Mol) zugegeben. Man läßt 30 Minuten bei 40 °C nachrühren und fügt 10 ml 96 %ige Schwefelsäure zu (pH-Wert = 4). Hierauf werden 200 g 30 %ige Formalinlösung (2 Mol) zugesetzt. Die Polymethylenharnstoffbildung und Mischkondensation setzt sehr rasch ein. Man rührt 6 Stunden bei 40 °C und neutralisiert anschließend mit 13 g Calciumhydroxid. Das erhaltene Biomassen-Azulminsäure-Harnstoff-Formaldehyd-Mischkondensat ist pulvrig und wird durch einfache Filtration isoliert. Das Mischkondensat wird mit 1 000 ml Wasser und anschließend mit 200 ml einer 2 %igen wäßrigen Ammoniak-Lösung gewaschen. Ausbeute nach 20-stündigem Trocknen bei 50 °C 315 g ; Stickstoffgehalt : 28,5 Gew.-%. Das Mischkondensat bindet adsorptiv ca. 10 Gew.-% an Wasser.

Die Metallanalyse des Verfahrensproduktes nach der Methode der Atomadsorption ergibt folgende Werte :

| | | | |
|---|---|---|---|
| 0,0001 % | Quecksilber | 0,007 % | Kupfer |
| 0,04 % | Zink | 0,24 % | Natrium |
| 0,03 % | Blei | 1,05 % | Calcium |
| 0,02 % | Chrom | 2,48 % | Eisen |

Der Metallgehalt in der Mutterlauge (= Abwasser) beträgt pro Liter :

| | | | |
|---|---|---|---|
| 0,75 g | Natrium | 235 mg | Eisen |
| 1,02 g | Calcium | 1,0 µg | Chrom |
| 0,3 mg | Zink | 0,1 µg | Kupfer |
| 2,8 mg | Blei | 0,1 µg | Quecksilber |

## Beispiel 26

Man verfährt genau wie in Beispiel 25 beschrieben, erzeugt die Azulminsäure durch Polymerisation der Blausäure in Gegenwart der gleichen Art und Menge an Biomasse, kondensiert aber anschließend die Biomassen-Azulminsäure-Dispersion mit Trimethylolmelamin als Aminoplastbildner. Hierzu verfährt man wie folgt :

1 000 g der in Beispiel 25 beschriebenen, etwa 8,4 % Feststoff enthaltenden wäßrigen Biomasse werden mit 163 ml (= 110 g) Blausäure, 440 ml Wasser, 25 ml einer 25 %igen wäßrigen Ammoniak-Lösung und 5 g Natriumcyanat vermengt. Diese Mischung wird 7 Stunden bei 70-90 °C gerührt, wobei die Blausäure zu Azulminsäuren polymerisiert. Bei 20-30 °C entfernt man restliche Blausäure und Ammoniak im Wasserstrahlvakuum und sorgt durch langsame Vakuumeinstellung dafür, daß die zu diesem Zeitpunkt noch nicht filtrierbare Azulminsäure-Biomassen-Dispersion nicht überschäumt. Hierauf werden dem Ansatz 120 g 30 %ige Formaldehyd-Lösung beigefügt. Es wird eine Stunde bei 100 °C kondensiert. Anschließend werden 126 g Melamin (1 Mol) zugesetzt. Man läßt 30 Minuten bei 100 °C rühren und setzt nun 300 g 30 %ige Formaldehyd-Lösung zu. Nach einer halben Stunde läßt man auf 45 °C abkühlen. Hierauf erfolgt Zugabe von 8 ml 96 %iger Schwefelsäure (pH = 4). Man kondensiert vier Stunden bei 45 °C unter intensivem Rühren. Anschließend wird mit 10 g Calciumhydroxid auf pH = 7 eingestellt. Man erhält ein gut filtrierbares Biomassen-Azulminsäure-Trimethylolmelamin-Kondensat. Man wäscht mit 1 000 ml Wasser und 200 ml 2 %iger wäßriger Ammoniak-Lösung. Ausbeute nach 20-stündiger Trocknung bei 60 °C im Vakuumtrockenschrank : 430 g.

Stickstoffgehalt des Mischkondensates : 32,5 %.

Metallgehalt des Biomassen-Azulminsäure-Trimethylolmelamin-Mischkondensates :

| | | | |
|---|---|---|---|
| $<$ 0,0001 % | Quecksilber | 0,007 % | Kupfer |
| 0,04 % | Zink | 0,15 % | Natrium |
| 0,04 % | Blei | 0,58 % | Calcium |
| 0,01 % | Chrom | 2,31 % | Eisen |

Gehalt an Metallen pro Liter Abwasser :

| | | | |
|---|---|---|---|
| 0,50 g | Natrium | 2,2 mg | Eisen |
| 0,95 g | Calcium | 48,0 μg | Quecksilber |
| 0,3 mg | Zink | < 1,0 μg | Chrom |
| 2,8 mg | Blei | < 0,1 μg | Kupfer |

Das hochgetrocknete Verfahrensprodukt bindet bei offener Lagerung bei normaler Luftfeuchtigkeit etwa 8-10 Gew.-% an Wasser adsorptiv, wobei es seine pulvrige Form beibehält.

Beispiel 27

Man verwendet 1 000 g einer aus einer vollbiologisch arbeitenden Kläranlage stammenden, etwa 8,4 Gew.-% Trockensubstanz enthaltenden Biomasse, die aus verschiedensten Mikroorganismen besteht. Die Biomasse ist durch 5-tägige Lagerung bei 25 °C ohne die Zufuhr von Nährstoffen in mikrobieller und enzymatischer Zersetzung begriffen, wobei sich neben lebenden Pseudomonas-Arten zahlreiche Mikroorganismen im Zustand des Zelltodes befinden. Durch enzymatische katalysierte Zersetzungsreaktionen, Decarboxylierungen von Aminosäuren und anderen Zellinhaltsstoffen werden hierbei übelriechende Geruchsträger entwickelt. Zu dieser Biomasse fügt man 100 g 30 %ige Formalinlösung (1 Mol) hinzu, rührt gut durch und kondensiert eine halbe Stunde bei 95 °C. Anschließend versetzt man bei 74 °C mit 60 g Harnstoff (1 Mol) und leitet die Polymethylenharnstoffbildung bzw. kondensierende Anpfropfung von Polymethylenharnstoffen an reaktive Zentren der Biomassen durch Zugabe von 18,4 g 85 %iger Phosphorsäure ein. Man hält zwei Stunden bei 75 °C, filtriert und wäscht das gut filtrierbare Pulver mit 2 %iger wäßriger Ammoniak-Lösung. Man erhält nach dem Trocknen bei 50 °C unter einem Druck von 18 Torr ein Biomassen-Mischkondensat in einer Ausbeute von 132 g in Form eines nahezu geruchlosen Pulvers.

N-Gehalt des Biomassen-Mischkondensates : 18,6 %

Phosphor-Gehalt : 2,9 %.

In Parallelversuchen wird festgestellt, daß als Kondensationskatalysatoren Schwefelsäure, Oxalsäure, Ameisensäure, Salzsäure, p-Toluolsulfonsäure, phosphorige Säure, Trichloressigsäure, Dichloressigsäure, Fluorwasserstoffsäure zur Herstellung der Biomassen-Mischkondensate ebenfalls brauchbar sind. Phosphorsäure, Schwefelsäure und phosphorige Säure sind die bevorzugt verwendeten Säuren.

Beispiel 28

Man verfährt genau wie in Beispiel 27 beschrieben, fügt jedoch vor der Formaldehydzugabe 1,2 g Natriumsulfid, 0,5 g Natriumhydrogensulfid und 0,4 g an Ammoniumpolysulfiden als Methylolierungskatalysator und Schwefelüberträger zu, um Schwermetalle wie Quecksilber, Blei, Kupfer, Cadmium, etc. in unlösliche Sulfide zu überführen. Bei der anschließenden sauren Kondensation, die nach den im Beispiel 27 enthaltenen Angaben durchgeführt wird, entstehen kleine Mengen an Schwefelwasserstoff, der teilweise mit Formaldehyd zu Polythioformaldehyd reagiert. Unter den sauren Kondensationsbedingungen geht der Polythioformaldehyd Kondensationsreaktionen ein, wobei Schwefelwasserstoff abgespalten wird. Ausbeute des Schwefelwasserstoff-freien Biomassen-Mischkondensates : 133 g. N-Gehalt des Biomassen-Mischkondensates : 18,1 % Phosphor-Gehalt : 2,7 %.

Beispiel 29

Man verfährt genau wie in Beispiel 17, Variante (a) beschrieben, verwendet aber zur Neutralisation des etwa 25 g 85 %ige Phosphorsäure enthaltenden Ansatzes etwa 40 g Calciumhydroxid. Durch die Calciumhydroxidzugabe werden zuckerartige, in der Mutterlauge gelöste Zellinhaltsstoffe wie Oligosaccharide und Glykoproteide Mitgefällt, indem sich Komplexe etwa der Zusammensetzung 3 CaO. 2 Saccharose-Moleküle bilden. Durch diese Maßnahme wird die Mutterlauge um ca. 8 g ärmer an hydrolysierten, wasserlöslichen Zellinhaltsstoffen. Anschließend wird überschüssiges Calciumhydroxid durch Begasen des Reaktionsgemisches mit Kohlendioxid in Calciumcarbonat umgewandelt.

Ausbeute : 185 g

Stickstoffgehalt : 11,9 %

Calciumhydrogenphosphat-Anteil : ca. 13,8 Gew.-%

Calciumcarbonat-Anteil : ca. 14 Gew.-%.

In der Mutterlauge des Biomassen-Kondensates sind weniger als 0,01 ppm an Quecksilber vorhanden. Diese Konzentration an Quecksilber kommt nach A. Stock überall in der belebten Natur vor und spielt in diesen Spurenkonzentrationen vermutlich eine positive, biologisch wichtige Rolle.

Beispiel 30

Man verfährt genau wie in Beispiel 17, Variante (a) beschrieben, setzt jedoch vor der Zugabe des Harnstoffes jeweils als Kettenabbrecher für entstehende Polymethylenharnstoffsegmente einen der nachfolgend genannten Stoffe ein :

a) 0,1 Mol N-Methylolcaprolactam der Formel

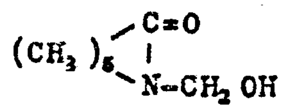

b) 0,1 Mol eines Azalactams der Formel

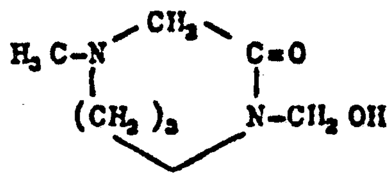

c) 0,1 Mol Benzolsulfonsäureamid,
d) 0,1 Mol Acetamid,
e) 0,1 Mol Thioacetamid,
f) 0,1 Mol Glyzerin,
g) 0,1 Mol Trimethylolpropan.

Ansonsten führt man die Kondensation nach den im Beispiel 17, Variante (a) enthaltenen Angaben durch.

Ausbeuten an Biomassen-Mischkondensat :

a) 179 g e) 179 g
b) 178 g f) 177 g
c) 181 g g) 178 g
d) 180 g

Durch den Zusatz der Kettenabbrecher werden feinteiligere Biomassen-Mischkondensate erhalten als dies ohne derartige Zusätze der Fall ist. Bei den obigen Umsetzungen werden Biomassen-Mischkondensate erhalten, deren Teilchengrößen zwischen 150 µm und 200 µm liegen. Führt man die Reaktion ohne Kettenabbrecher durch, so liegen die Teilchengrößen der entstehenden Produkte zwischen 350 µm und 550 µm.

Beispiel 31

Man verfährt genau wie in Beispiel 17, Variante (b) beschrieben, dispergiert aber vor der Formaldehydzugabe in der Biomasse jeweils einen der folgenden schwer- bzw. unlöslichen Trägerstoffe bzw. eines der folgenden Trägerstoff-Gemische :

a) 20 g eines Lignin-Cellulose-Homogenisates,
b) etwa 20 g Kieselsäure. Diese wird dadurch erzeugt, daß man 126 g einer ca. 32 %igen Wasserglaslösung zusetzt, bei 70 °C mit der Biomasse mischt und Kohlendioxid einleitet, wodurch unlösliche Polykieselsäuren in situ in der Biomasse dispergiert werden.
c) 20 g Quarzpulver,
d) 10 g Tonerdehydrat und 10 g Antimontrioxid,
e) 20 g mikrobiell infiziertes und geruchlich nicht einwandfreies Sojamehl,
f) 20 g mikrobiell infiziertes und in Zersetzung befindliches Fischmehl.

Ansonsten wird die Biomassen-Mischkondensation nach den in Beispiel 17, Variante (b) enthaltenen Angaben durchgeführt.

Ausbeute an gut filtrierbaren und geruchlosen Biomassen-Mischkondensaten :

a) 196 g d) 198 g
b) 197 g e) 194 g
c) 198 g f) 192 g

Beispiel 32

Man verfährt genau wie in Beispiel 17, Variante (b) beschrieben, verwendet die gleiche Art und Menge (1 000 g) an einer aus einer biologisch arbeitenden Kläranlage stammenden, 8,4 Gew.-% Trockensubstanz (Proteingehalt ca. 45 Gew.-% bezogen auf Trockensubstanz) enthaltenden Biomasse, setzt aber als Carbonyl-Komponente jeweils eine der nachstehend genannten Verbindungen bzw. eines

der folgenden Verbindungs-Gemische ein :

a) 1 Mol Acetaldehyd,
b) 1 Mol Isobutyraldehyd,
c) 0,5 Mol Crotonaldehyd, 0,2 Mol Isobutyraldehyd und 0,2 Mol Formaldehyd,
d) 0,5 Mol Glyoxal und 0,5 Mol Formaldehyd,
e) 0,5 Mol Crotonaldehyd und 0,5 Mol Formaldehyd,
f) 0,5 Mol Acrolein und 0,5 Mol Formaldehyd,
g) 1,5 Mol Formaldehyd und 0,5 Mol Cyclohexanon,
h) 2,5 Mol Formaldehyd und 0,5 Mol Methyläthylketon,
i) 0,5 Mol Glutardialdehyd und 0,5 Mol Formaldehyd,
j) 0,6 Mol Formaldehyd und 0,5 Mol Salicylaldehyd,
k) 0,5 Mol Formaldehyd und 0,5 Mol Furfurol,
l) 0,5 Mol Formaldehyd und 0,5 Mol Chloralhydrat.

Zunächst wird in Abwesenheit von Harnstoff und ohne Säure 1 Stunde bei 70 °C kondensiert. Anschließend wird das Reaktionsgemisch in den Fällen (a) bis (l) jeweils mit einem Mol Harnstoff versetzt und die Temperatur auf 35 °C herabgesetzt. Zu diesem Zeitpunkt fügt man bei 35 °C zu jedem Ansatz 25 g 85 %ige Schwefelsäure hinzu. Man läßt acht Stunden reagieren, kondensiert anschließend zwei Stunden bei Raumtemperatur nach und erhält nach der Neutralisation mit Calciumoxid gut filtrierbare Biomassen-Aminoplast-Mischkondensate, die nach ihrer Trocknung bei 50 °C unter einem Druck von 18 Torr in feinpulvriger Form in folgenden Ausbeuten erhalten werden :

a) 155 g g) 172 g
b) 158 g h) 169 g
c) 163 g i) 158 g
d) 159 g j) 164 g
e) 164 g k) 169 g
f) 164 g l) 171 g

Alle Biomassen-Aminoplast-Mischkondensate enthalten durch die Neutralisation der Schwefelsäure durch Calciumoxid zwischen 11 bis 14 Gew.-% an mit den Biomischkondensaten innig vermischtem Calciumsulfat.

Bei den vorgenannten Umsetzungen reagieren Acetaldehyd, Crotonaldehyd, Isobutyraldehyd, Chloralhydrat infolge der relativ großen Verdünnung und infolge der geringeren Reaktivität vorgenannter Carbonylverbindungen gegenüber Formaldehyd zwischen 60-70 % der Theorie, bezogen auf die eingesetzten Aldehydmengen.

## Beispiel 33

Man verfährt wie in Beispiel 17, Variante (b) beschrieben und setzt als Carbonyl-Komponente jeweils einen der folgenden polymeren Thioaldehyde ein :

a) Polymeren Thioformaldehyd (1,2 Mol)
b) Trimeren Thioformaldehyd (Trithian) (1,5 Mol)
c) Polymeren Thioacetaldehyd (1,3 Mol).

Man verwendet zur Aufspaltung der Thioaldehyde 20 g konzentrierter Schwefelsäure und verwendet jeweils 1 Mol Harnstoff als Aminoplastbildner. Die Mischkondensation verläuft unter Schwefelwasserstoffentbindung etwa bei 95 °C. Man kondensiert 12 Stunden und arbeitet wie in Beispiel 17, Variante (b) beschrieben auf, indem man die verwendete Schwefelsäure mit Calciumhydroxid neutralisiert.

Ausbeuten :

a) 158 g c) 142 g
b) 147 g

## Beispiel 34

Man verfährt wie in Beispiel 17, Variante (b) beschrieben, verwendet aber andere Biomassen, und zwar jeweils eine der nachstehend genannten :

a) 1 800 g einer wäßrigen Biomasse-Dispersion, enthaltend lebende Zellen des Bacteriums Alcaligenes eutrophus (Stamm $H_{16}$, ATCC 176 999), die zur Gewinnung von Einzellerproteinen verwendet werden (Gehalt an Trokkensubstanz : ca. 2 Gew.-%).

b) 1 800 g einer wäßrigen Biomasse-Dispersion, enthaltend Zellen des Bacterienstammes Serratia mascescens (Gehalt an Trockensubstanz : ca. 2 Gew.-%).

c) 900 g einer wäßrigen Biomasse-Dispersion, enthaltend Bakterienzellen von Streptomyces-Arten (ca. 2 Gew.-% Trockensubstanz), die zur Herstellung des Antibioticums Streptomycin verwendet werden.

d) 1 000 g einer wäßrigen Biomasse-Dispersion, bestehend aus fadenartigen Bacterien, enthaltend Zellen des Bacteriums Mikromonospora, die zur Herstellung des Breitband-Antibioticums Sisomicin verwendet werden (ca. 5 Gew.-% Trockensubstanz).

e) 1 000 g einer wäßrigen Biomasse-Dispersion, bestehend aus Pilzen der Penicillin-Herstellung, enthaltend Zellen des Pilzes Penicillium chrysogenum (Penicillium notatum) (enthaltend ca. 5 Gew.-%

Trockensubstanz).

f) 1 000 g einer wäßrigen Biomasse-Dispersion, bestehend aus Zellen des Pilzes Aspergillus niger (Trockengewicht ca. 5 Gew.-%).

g) 1 000 g einer wäßrigen Biomasse-Dispersion, bestehend aus pilzlichen Organismen aus der Reihe hochaktiver Hefen der alkoholischen Gärung, enthaltend zusätzlich pflanzliche Stoffe, ferner Saccharomyces cerevisiae, Saccharomyces uvarum und andere Mikroorganismen, wie Botrytis cinerea, Milchsäure- und Essigbakterien (Trokkengewicht : Summe an pflanzlichem ànd mikrobiellem Material ca. 5 Gew.-%).

Die vorgenannten wäßrigen Biomassen-Dispersionen werden jeweils zunächst mit 15 g Formaldehyd bei 70 °C vorkondensiert (Methylolierung und Zellwandsprengung) anschließend werden 60 g (1 Mol) Harnstoff und 100 g 30 %ige Formalinlösung (1 Mol) zugesetzt, und es wird 20 Minuten bei 70 °C kondensiert. Für die Kondensation mit Harnstoff werden 4 g konzentrierte 96 %ige Schwefelsäure verwendet, so daß ein pH-Wert von etwa 2,2 erreicht wird. Die Mischkondensation setzt im Falle der Umsetzungen (a) bis (g) sofort ein. Man rührt 0,5 Stunden bei 70 °C, kühlt anschließend auf Raumtemperatur ab und läßt 4 Stunden bei Raumtemperatur nachrühren. Man erhält ausgezeichnet filtrierbare, pulvrige Biomassen-Mischkondensate, die durch Waschen mit 2 %iger wäßriger Ammoniak-Lösung von Spuren an Schwefelsäure befreit werden.

Ausbeuten nach dem Trocknen bei 50 °C im Vakuumtrockenschrank :

a) 96 g, N-Gehalt : 27,6 % e) 102 g, N-Gehalt : 21,2 %
b) 85 g, N-Gehalt : 27,9 % f) 114 g, N-Gehalt : 24,4 %
c) 82 g, N-Gehalt : 26,7 % g) 116 g, N-Gehalt : 24,8 %
d) 106 g, N-Gehalt : 25,6 %

Durch Einengen der Mutterlaugen werden bei den Ansätzen (a) bis (f) Zellinhaltsstoffe wie Polysaccharide, Glykoproteide und Oligopeptide noch unbekannter Art isoliert, die eine weiß-gelbliche Farbe besitzen und wertvolle Nährstoffe für andere Bakterienkulturen darstellen.

Ausbeuten an Zellinhaltsstoffen :

a) 7 g e) 8,9 g
b) 5 g f) 7,5 g
c) 4,8 g g) 8,3 g
d) 12 g

## Beispiel 35

Man verfährt wie in Beispiel 17, Variante (b) beschrieben, jedoch mit 1 000 g einer etwa 4,8 % Trockensubstanz enthaltenden wäßrigen Biomasse, bestehend aus Flagellaten, Euglena, Escherichia coli, Amöben (Wechseltierchen), Pantoffeltierchen, Fadenalgen (Spirogyra), und extracellulären schleimigen Massen und Zellen abgestorbener Pflanzenteile. Ansonsten verfährt man genau nach den im Beispiel 17, Variante (b) enthaltenden Angaben und kondensiert bei pH = 2,8.

Ausbeute an Biomassen-Mischkondensat : 163 g.

Stickstoffgehalt : 16,4 %.

Im biologischen Test erweist sich das feinpulvrige Produkt als völlig keimfrei und frei von pathogenen Krankheitserregern.

## Beispiel 36

1 000 g einer wäßrigen, oberflächlich wachsenden und unter Bildung von Geruchsträgern befindlichen Pilzmyceldispersion verschiedenster Schimmelpilze, wachsend auf einer Mischung von Cellulose-Pulver, Kartoffelstärke-Konzentraten und Peptone enthaltenden Nährlösungen, enthaltend ferner celluloseabbauende Asco- und Deuteromyceten, ferner Lignin-abbauende Pilze wie Phellinus igniarius und Basidiomyceten, mit einem Trockengewicht von ca. 8 Gew.-%, bezogen auf celluläre und extracelluläre Bestandteile, werden

a) genau wie im Beispiel 17, Variante (a) beschrieben zunächst mit Formaldehyd und dann mit Harnstoff und Formaldehyd kondensiert,

b) genau wie im Beispiel 17, Variante (a) beschrieben zunächst mit Formaldehyd und dann nach der im Beispiel 19 beschriebenen Methode mit Trimethylolmelamin kondensiert.

Ausbeuten :

a) 161 g an Polymethylenharnstoff-Biomassen-Mischkondensat.
b) 153 g an Polymethylen-melamin-Biomassen-Mischkondensat.

## Beispiel 37

Man kondensiert primär 30 Minuten mit 0,1 Mol Formaldehyd bei pH = 8 und 80 °C, anschließend mit 0,4 Mol Monomethylolharnstoff und 0,1 Mol Trimethylolmelamin bei 60 °C eine Stunde lang mit jeweils einem der folgenden wäßrigen Biomassen-Homogenisate, die extracelluläre Bestandteile enthalten :

a) 200 g eines frischen, wäßrigen, vermahlenen Zellhomogenisates aus Leguminosenwurzeln, die

durch Infektion unter natürlichen Wachstumsbedingungen Stickstoff assimilierenden Bakterien (= Rhizobium, Bacterium radiciola) enthalten. Trockengewicht : ca. 15 g an Pflanzenzellen und Rhizobium.

b) 200 g eines frischen, wäßrigen Zellhomogenisates von Wurzeln der Erle, die symbiontisch lebende Actinomyceten enthalten.

c) 200 g eines frischen, wäßrigen Grashomogenisates, das einen Proteingehalt von ca. 12 Gew.-%, bezogen auf das eingesetzte Trockengewicht von ca. 10 g aufweist.

d) 200 g eines durch Zusatz von einem g mikrobiell aktiver Gartenerde in anaerober Fäulnis befindlichen wäßrigen Grashomogenisates von Mischgräsern (= 7-tägige Lagerung unter Luftabschluß bei 37 °C), auf dem sich zahlreiche Mikroorganismen und schleimartige, extracelluläre Bestandteile gebildet haben ; Trockengewicht : 12 g.

e) 200 g eines wäßrigen in aerober Fäulnis bei 35 °C befindlichen Zellhomogenisates von Mischgräsern und Unkräutern, in denen sich bei 7-tägiger Lagerung unter Luftzutritt zahlreiche Mikroorganismen als Biomassen gebildet haben, deren Bildung hauptsächlich durch Zusatz von einem g mikrobiell aktiver Gartenerde eingeleitet wurde, wobei in diesen Biomassen auch Sulfatreduzierende, Nitrit- und Nitratbakterien enthalten sind ; Trockengewicht ca. 16 g.

Alle vorgenannten Biomassen-Zellhomogenisate sind durch Bildung extracellulärer Schleimstoffe primär unfiltrierbar. Nach Durchführung der Kondensation unter den im Beispiel 17, Variante (b) angegebenen Bedingungen und nach dem Ansäuern mit Schwefelsäure auf pH 2,2 wird die Kondensation zu Ende geführt. Man erhält leicht filtrierbare Aminoplast-Biomassen-Mischkondensate, die nach der Entfernung des Katalysators durch Waschen mit Wasser und nach Trocknung bei 60 °C unter vermindertem Druck in folgenden Ausbeuten anfallen :

a) 59 g d) 53 g
b) 57 g e) 57 g
c) 54 g

Bei der obigen Umsetzung (c) sind pflanzliche Zellinhaltsstoffe wie Chlorophyll (a) und Chlorophyll (b) weitgehend mischkondensiert worden. Nichtkondensierte kleinere Anteile derartiger Zellinhaltsstoffe können mit Aceton extrahiert werden.

## Beispiel 38

Man kondensiert genau wie in Beispiel 37 beschrieben, verwendet aber jeweils eines der folgenden wäßrigen Biomassen-Systeme :

a) 1 000 g einer mikrobiell infizierten, etwa 87 Gew.-% Wasser und etwa 12 Gew.-% Trockensubstanz (hauptsächlich bestehend aus Casein, Milchzucker und Milchfett) enthaltenden Milch, die Milchsäurebakterien und andere Bakteriensorten enthält.

b) 100 g einer bakteriell infizierten Molke, die nach der Ausscheidung von Milchfett und Casein noch Milchzucker, Salze und etwa 2 Gew.-% an löslichem Eiweiß enthält.

Die unter (a) und (b) genannten Biomassen-Systeme werden genau wie in Beispiel 37 beschrieben mischkondensiert.

Ausbeuten an Aminoplast-Biomasse-Mischkondensaten, enthaltend vernetzte extracelluläre Stoffe :

a) 58 g
b) 51 g

Die pulvrigen Aminoplast-Biomassen-Mischkondensate, die extracelluläre vernetzte Bestandteile enthalten, erweisen sich im biologischen Test als völlig keimfrei.

## Beispiel 39

Man verfährt genau wie in Beispiel 37 beschrieben, verwendet aber jeweils eines der folgenden wäßrigen Biomassen-Homogenisate :

a) 60 g eines wäßrigen Homogenisates von pflanzlichem Plankton (= Algen, und zwar überwiegend Phaeophyceen und Rhodophyceen (Desmarestia, Delesseria, Bangia, Porphyra und Fucus). Trockengewicht ca. 3 g.

b) 180 g pflanzliches Plankton des Meeres [= Pyrrophyceen, Chrysophyceen, Diatomeen, Peridineen, Coccolithineen und Silicoflagellaten, die in Maschen des Plankton-Netzes nicht erfaßt werden können (Nannoplankton)] ; Trockengewicht : ca. 2 Gew.-%.

c) 12 g In Zersetzung befindliches dispergiertes Fischmehl in 200 ml Wasser.

d) 240 g eines wäßrigen antarktischen Krill-Homogenisates [= homogenisierte tierische Zellen der Krill-Krebsart, die eine Hauptnahrung der Bartenwale darstellen = Walkrebschen, Euphausia superba, (= garnelenartiges Krebschen von 2-4 cm Länge)], das in Zersetzung befindlich ist ; Trockengewicht etwa 18 g.

Man erhält im Falle der Umsetzungen (a) bis (d) jeweils pulvrige, gut filtrierbare Polymethylenpolyharnstoff-polymethylenmelamin-Biomassen-Mischkondensate, die völlig geruchlos sind und in folgenden Ausbeuten anfallen :

a) 56 g, Stickstoffgehalt : 40,8 % c) 63 g, Stickstoffgehalt : 38,5 %

b) 51 g, Stickstoffgehalt : 39,2 % d) 71 g, Stickstoffgehalt : 38,1 %.

Beispiel 40

1 000 g einer aus einer vollbiologisch arbeitenden Kläranlage für industrielle und kommunale Abwässer stammenden, etwa 8,4 Gew.-% Trockensubstanz enthaltenden Biomasse werden zunächst mit 300 g einer 30 %igen wäßrigen Formaldehyd-Lösung 30 Minuten bei 95 °C behandelt. Anschließend werden 15 g in Wasser aufgeschlämmtes Calciumhydroxid langsam zugegeben. Danach werden bei 90 °C im Verlaufe von 4 Stunden etwa 2 Mol an Formaldehyd in karamelisierte Formose, — ein Gemisch von zuckerartigen Hydroxyaldehyden —, umgewandelt. Es wird zusätzlich 1 Mol Harnstoff zugegeben und schließlich mit 34 g Schwefelsäure als Katalysator die Kondensation des Harnstoffs mit Formaldehyd und den entstandenen Hydroxyaldehyden eingeleitet.

Anschließend wird mit Calciumoxid neutralisiert. Man erhält ein filtrierbares Biomassen-Mischkondensat. Zur Vermeidung der Entfernung von löslichen Zellinhaltsstoffen wird jedoch nicht filtriert, sondern das Wasser unter vermindertem Druck entfernt und die Reaktionsmischung zusammen mit den gebildeten karamelisierten Zuckern auf einer Blechschale eingedampft. Man erhält ein Calciumsulfat enthaltendes Biomassen-Mischkondensat, das nach karamelisierten Zuckern riecht. Das Mischkondensat wird in krümeliger Form mit torfbrauner Farbe erhalten :

Ausbeute : 332 g

Stickstoffgehalt : 12,8 %

Beispiel 41

Verwendung von Aminen, Hydrazinen und Hydraziden zur Biomassen-Mischkondensation.

100 g einer aus einer vollbiologisch arbeitenden Kläranlage für kommunale Abwässer stammenden, etwa 7,8 Gew.-% Trockensubstanz enthaltenden Biomasse, in der neben Pseudomonas-Arten zahlreiche andere Mikroorganismen, z.B. Flagellaten, Escherichia coli-Bakterien, Amöben etc. und extracelluläre Stoffwechselprodukte, schleimige Bestandteile und Schwebstoffe pflanzlicher cellulose- und ligninhaltiger Materialien vorliegen, werden zunächst mit 10 g einer 30 %igen wäßrigen Formaldehyd-Lösung (0,1 Mol) bei 95 °C kondensiert, wobei pathogene Krankheitserreger und Bakterienzellwände bereits angegriffen und abgetötet werden. Man läßt die Temperatur auf 30 °C fallen und dosiert im Verlaufe von einer Stunde jeweils eine der folgenden, sehr rasch kondensierenden Verbindungen (Aminoplastbildner bzw. Phenoplastbildner) bzw. Verbindungs-Gemische hinzu :

a) 0,05 Mol Hexamethylendiamin
b) 0,1 Mol Anilin
c) 0,01 Mol Isophorondiamin der Formel

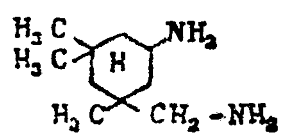

d) 0,05 Mol Anilin und 0,05 Mol Phenol
e) 0,05 Mol Anilin und 0,025 Mol Bisphenol A
f) 0,02 Mol Hydrazinhydrat
g) 0,05 Mol Adipinsäuredihydrazid und 0,02 Mol Thioacetamid
h) 0,05 Mol Hydroxylamin und 0,005 Mol Hexamethylendiamin
i) 0,05 Mol p-Phenylendiamin, 0,01 Mol Salicylaldehyd und 0,01 Mol Terephthaldialdehyd.

Man kondensiert anschließend 4 Stunden bei Raumtemperatur nach und erhält pulvrige, filtrierbare Aminoplast-Biomassen-Mischkondensate in Form von braun-schwarzen Pulvern. Im Falle der Verwendung von Hexamethylendiamin (Umsetzung a) oder Isophorondiamin (Umsetzung b) als zur Aminoplastbildung befähigte Verbindungen werden in der Mischung hochvernetzte Polyhexahydrotriazinkondensate erhalten.

Ausbeute nach erfolgter Trocknung bei 80 °C bei einem Druck von 14 Torr :

a) 14,8 g f) 11,2 g
b) 16,2 g g) 15,8 g
c) 16,9 g h) 14,4 g
d) 16,3 g i) 18,3 g
e) 17,5 g

Beispiel 42

Verwendung von Ammoniak als zur Aminoplastbildung befähigte Verbindung und Benzochinon als

Carbonylkomponente zur Mischkondensation von Biomassen.

Man erhitzt 100 g der im Beispiel 41 genannten Biomasse auf 80 °C, dispergiert dann 10,8 g feingepulvertes Benzochinon und leitet in die gut erührte Dispersion bei 85 °C gasförmigen Ammoniak ein. Es erfolgt hierbei sehr rasch Bildung von Additions- und Polykondensationsprodukten, welche die Farbe der dispergierten Biomasse über braunschwarz bie graphitartif schwarz vertiefen.

Man erhält nach der Filtration und Trocknung 19,4 g Biomassen-Mischkondensat in Form einer pulvrigen Substanz.

Beispiel 43

Verwendung von Hexamethylentetramin und anderen Hexahydrotriazinen als verkappte Carbonylverbindungen und verkappte Aminoplastbildner zur Mischkondensation von Biomassen.

Man erhitzt jeweils 100 g der im Beispiel 41 genannten wäßrigen Biomassen-Dispersion zunächst mit 0,1 Mol Formaldehyd 20 Minuten auf 90 °C und anschließend mit einer der folgenden Verbindungen auf 100 °C :

a) 10 g Hexamethylentetramin
b) 15 g Tri-n-butyl-hexahydrotriazin
c) 18 g 2,4,6-Tris-dimethylaminomethylphenol.

Im Verlaufe von 2 Stunden erfolgt kontinuierliche Abspaltung von Ammoniak (a), bzw. n-Butylamin (b) bzw. von Dimethylamin (c), wobei Ankondensation von N-Methylengruppen bzw. C-Methylengruppen enthaltenden Resten der obengenannten Kondensationspartner an aktive Zentren der Biomassen erfolgt. Nach 6-stündiger Kondensationsdauer erhält man nach Filtration und Trocknung folgende Ausbeuten an Biomassen-Mischkondensaten :

a) 15,6 g c) 17,8 g.
b) 11,8 g

Beispiel 44

Verwendung von Blut zur Mischkondensation mit Carbonylverbindungen und zur Aminoplastbildung befähigten Verbindungen.

a) 450 g frisches Rinderblut werden bei Raumtemperatur zunächst bei pH = 8 mit 0,1 Mol Formaldehyd in Gegenwart von Kaliumcarbonat kondensiert, wobei die im Blut enthaltenen Proteine partiell methyloliert werden. Anschließend wird eine Lösung von 45 g (0,75 Mol) Harnstoff in 75 g (0,75 Mol) einer 30 %igen Formalin-Lösung und 325 g Wasser innig mit dem Blut vermischt und unter intensivem Rühren mit 18,4 etwa 98 %iger Schwefelsäure versetzt. Das Reaktionsgemisch weist einen pH-Wert von 1,6 auf, wobei die Kondensation sofort einsetzt und die Temperatur durch exotherme Reaktionswärme auf ca. 33 °C ansteigt. Bereits nach 15 Minuten zeigt die Kontrollprobe (5 ml), daß durch die fortschreitende Mischkondensation der Biomasse bereits ausgezeichnete Filtrierbarkeit gegeben ist. Man kondensiert vier weitere Stunden bei Raumtemperatur, neutralisiert dann mit Calciumhydroxid und erhält ein ausgezeichnet filtrierbares, bräunliches Blut-Biomassen-Mischkondensat. Das Filtrat ist wasserhell und enthält nach dem Einengen unter vermindertem Druck praktisch keine extracellulären Proteine, sondern lediglich geringe Mengen an Polymethylenharnstoffen und ca. 1,6 g Calciumsulfat.

Ausbeute nach dem Trocknen im Vakuumtrockenschrank bei 50 °C : 170 Gew.-Teile : 15,6 % N Calciumsulfat-Gehalt : 11,2 %.

b) Man verfährt genau wie unter (a) beschrieben, verwendet jedoch eine erhöhte Menge an Harnstoff und Formaldehyd und zwar 90 g Harnstoff (1,5 Mol) und 150 g 30 %ige Formalinlösung. Man erhält ein Polymethylenharnstoffreicheres Biomassen-Mischkondensat, das alle extracellulären wasserlöslichen Proteine des Blutes mischkondensiert enthält. Die völlig farblose Mutterlauge enthält lediglich 10 g an Harnstoff-Formaldehyd-Kondensaten sowie Kochsalz und Calciumsulfat. Ausbeute nach dem Trocknen bei 50 °C im Vakuumtrockenschrank : 232 g, 23,4 % Stickstoffgehalt, Calciumsulfat-Gehalt ca. 10,3 %.

c) Verfährt man gemäß (b) mit frischem Schweineblut, so wird nahezu in identischer Ausbeute ein Biomassen-Mischkondensat von torfbrauner Farbe erhalten. Ausbeute : 233 g, Stickstoffgehalt : 20 %, Calciumsulfat-Gehalt : 10,5 %.

Beispiel 45

1 000 g eines aus einer vollbiologisch arbeitenden Kläranlage für industrielle und kommunale Abwässer stammenden, etwa 8,5 % Trockensubstanz enthaltenden, extrem schwer filtrierbaren Bakterienbelebtschlammes werden in der ersten Stufe mit 0,6 Mol Formaldehyd und 0,4 Mol Isobutyraldehyd 30 Minuten bei 70 °C umgesetzt, wobei Zellwandsprengung und Plasmolyse der Mikroorganismen eintritt und N-Alkylolierungsreaktionen sowie N,N-Aminalbildungen proteinhaltiger und nucleinsäurehaltiger Zellinhaltsstoffe ablaufen. Anschließend wird auf 30 °C abgekühlt und mit 60 g Harnstoff unter Verwendung von Schwefelsäure als Katalysator 4 Stunden kondensiert. Man erhält ein leicht filtrierbares pulvriges Biomassen-Mischkondensat, das neben

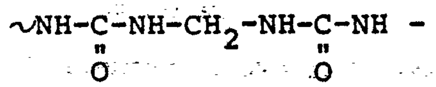 Segmenten

auch hydrolytisch und biolgisch leichter abbaubare

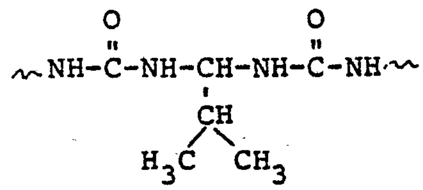

Segmente eingebaut enthält. Ausbeute : 118 g, N-Gehalt : 19,5 %.

Obwohl die Ausgangsbiomasse außerordentlich unangenehme Geruchsträger enthält, erweist sich das Verfahrensprodukt nach erfolgter Behandlung mit 2 %iger wäßriger Ammoniaklösung und intensivem Nachwaschen mit Aceton als völlig geruchlos.

Beispiel 46

Auf jeweils 100 g der gemäß Beispiel 17, Varianten (a) und (b) hergestellten Biomassen-Mischkondensate wird in wäßriger Dispersion zunächst durch Zugabe von überschüssiger, wäßriger Natronlauge jeweils eines der nachstehend genannten Hydroxide niedergeschlagen :

Calciumhydroxid,
Bariumhydroxid,
Magnesiumhydroxid,
Bleihydroxid,
Eisen-II-hydroxid,
Aluminiumhydroxid,

wobei diese Hydroxide aus den zuvor zugefügten Metallhalogeniden entstehen. Anschließend wird die überschüssige Natronlauge durch Zugabe einer äquivalenten Menge an Phosphorsäure neutralisiert. Hierbei werden schwerlösliche Phosphate auf der Biomassen-Mischkondensat-Matrix erzgugt. — Man erhält leicht filtrierbare, Metallhydroxid- und Metallphosphat-haltige Biomassen-Mischkondensate.

Beispiel 47

Behandlung der Oberfläche von Biomassen-Mischkondensaten durch Umsetzung dieser Produkte mit Reagenzien, die Alkylierungs-, Acylierungs-Reaktionen, ringöffnende Additions-reaktionen, Michael-Additionen,

Polymerisationen oder Mischpolymerisationen bewirken und so die Oberfläche der Biomassen-Mischkondensate modifizieren.

Jeweils 100 g des gemäß Beispiel 17 hergestellten Verfahrensproduktes werden in hoch getrocknetem Zustand (100 °C, 12 Torr) mit jeweils einem der nachstehend genannten Stoffe bzw. Stoffgemische versetzt :

a) mit 30 g Essigsäureanhydrid und 50 g Toluol unter Verwendung von 0,4 g Natriumacetat als Katalysator,

b) mit 80 g Styrol, 20 g Acrylnitril (in 100 g Xylol) und 2 g des Radikalbildners Azoisobuttersäuredinitril,

c) mit 100 g Styrol, gelöst in 400 g o-Dichlorbenzol und 2 g des Radikalbildners Azoisobuttersäuredinitril,

d) mit 100 g Methacrylsäuremethylester, gelöst in 400 g o-Dichlorbenzol und 2 g Azoisobuttersäuredinitril,

e) mit 100 g Vinylacetat, gelöst in 400 g Dichlorbenzol und 2 g Azoisobuttersäuredinitril,

f) 50 g Styrol und 50 g Methacrylsäure-β-hydroxypropylester.

Das jeweilige Gemisch wird auf 150 °C aufgeheizt und 6 Stunden bei dieser Temperatur gehalten. Die Aufarbeitung erfolgt jeweils durch Ausfällung der Produkte mit Methanol. Man erhält im Falle der Umsetzungen (a) bis (f) jeweils an ihren Oberflächen modifizierte Biomassen-Mischkondensate, die völlig geruchlos sind.

Ausbeuten :

a) 105 g c) 168 g e) 158 g
b) 175 g d) 162 g f) 172 g.

Beispiel 48

a) 300 g des gemäß Beispiel 17, Variante (a) hergestellten Biomassen-Mischkondensates werden in einem zusätzlich mit einem Rührer ausgestatteten Wirbelbett, in einem Glasrohr vom Querschnitt 2,5 cm im Gegenstrom von unten mit Kohlendioxid und von oben mit Ammoniak begast, wobei man die Dosierung derartig vornimmt, daß das molare $NH_3/CO_2$-Verhältnis 2:1 beträgt und durch ein seitlich angebrachtes Abgangsrohr in der Säule Druckausgleich erfolgt. Man erzeugt hierbei innerhalb der Kanäle und Poren des Biomassen-Mischkondensates im Verlaufe einer Stunde 100 g von carbaminsaurem Ammonium der Formel

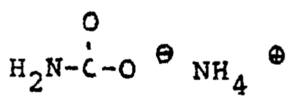

neben Ammoniumbicarbonat und Ammoniumcarbonat. Das hierbei entstehende Gemisch enthält die Ammoniumsalze in wesentlich verbesserter lagerfähiger Form, indem Ammoniak bei der Lagerung bei Raumtemperatur um den Faktor 4 lansamer in den Luftraum von Gebinden abgegeben wird als dies bei den freien Salzen der Fall ist.

Ausbeute : 400 g ; N-Gehalt : 18,8 %.

b) Führt man die Erzeugung von carbaminsaurem Ammonium 4 Stunden durch, so werden von 300 g des feinpulvrigen Biomassen-Mischkondensates etwa 300 g Ammoniumcarbaminate includiert bzw. auf der Biomassen-Mischkondensat-Matrix niederschlagen.

Ausbeute : 600 g, N-Gehalt : 24,6 %.

Mit zunehmendem Wassergehalt der eingesetzten Biomassen-Mischkondensate bilden sich dabei neben dem carbaminsauren Ammonium

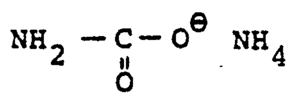

in verstärktem Maße Ammoniumbicarbonat und Ammoniumcarbonat.

**Ansprüche**

1. Verfahren zur Aufarbeitung von Biomassen verschiedenster Art durch Umsetzen der Biomassen in wäßrigem Medium mit Carbonylverbindungen, Thiocarbonylverbindungen und/oder Carbonylverbindungen, die mit niedermolekularen, nicht kondensierten N-Alkylolverbindungen im Dissozationsgleichgewicht stehen, gegebenenfalls in Gegenwart eines Katalysators sowie gegebenenfalls in Gegenwart von Zusatzstoffen, dadurch gekennzeichnet, daß man anschließend die nicht umgesetzten Carbonylverbindungen, Thiocarbonylverbindungen und/oder Carbonylverbindungen, die mit niedermolekularen, nicht kondensierten N-Alkylolverbindungen im Gleichgewicht stehen, in wäßrigem Medium mit Aminoplastbildnern, die gegebenenfalls N-Alkylolgruppen enthalten, bzw. mit Phenoplastbildnern gegebenenfalls in Gegenwart eines Katalysators und gegebenenfalls in Gegenwart von Kettenabbrechern sowie gegebenenfalls in Gegenwart von Zusatzstoffen kondensiert und die so entstehenden modifizierten Biomassen gegebenenfalls anschließend von noch enthaltenen unerwünschten Stoffen befreit, und/oder einer Nachbehandlung unterwirft.

2. Verfahren gemäß Anspruch 1, dadurch gekennzeichnet, daß man die Umsetzung bei Temperaturen zwischen 0 °C und 200 °C durchführt.

3. Verfahren gemäß Anspruch 2, dadurch gekennzeichnet, daß man die Umsetzung bei Raumtemperatur durchführt.

4. Verfahren gemäß Anspruch 1, dadurch gekennzeichnet, daß man die Umsetzung unter vermindertem oder erhöhtem Druck durchführt.

5. Verfahren gemäß Anspruch 1, dadurch gekennzeichnet, daß man Formaldehyd, Crotonaldehyd oder Isobutyraldehyd als Carbonylverbindung einsetzt.

6. Verfahren gemäß Anspruch 1, dadurch gekennzeichnet, daß man Harnstoff, Monomethylolharnstoff, Dimethylolharnstoff, Rohazulminsäuren, modifizierte Azulminsäuren und/oder stabilisierte Azulminsäuren als Aminoplastbildner einsetzt.

7. Verfahren gemäß Anspruch 1, dadurch gekennzeichnet, daß man Schwefelsäure oder Phosphorsäure als Katalysatoren einsetzt.

8. Modifizierte Biomassen, gekennzeichnet durch einen Gehalt an

— mit Carbonylverbindungen, Thiocarbonylverbindungen und/oder Carbonylverbindungen, die mit niedermolekularen, nicht kondensierten N-Alkylolverbindungen im Dissozationsgleichgewicht stehen,

— und mit Aminoplastbildnern, die gegebenenfalls N-Alkylolgruppen enthalten, bzw. mit Phenoplastbildnern kondensierten Biomassen-Zellinhaltsstoffen bzw. anderen in Biomassen vorkommenden reaktionsfähigen Stoffen,

sowie gegebenenfalls durch einen Gehalt an Zusatzstoffen.

9. Verwendung von modifizierten Biomassen gemäß Anspruch 8 als reaktive Füllstoffe und/oder als Flammschutzmittel in Kunststoffen, als Katalysatoren und/oder als Trägermaterialien für Katalysatoren.

**Claims**

1. Process for working up biomasses of the most diverse nature by reacting the biomasses with carbonyl compounds, thiocarbonyl compounds and/or carbonyl compounds which are in dissociation equilibrium with low-molecular, non-condensed N-alkylol compounds, in an aqueous medium, if appropriate in the presence of a catalyst and optionally in the presence of additives, characterised in that the unreacted carbonyl compounds, thiocarbonyl compounds and/or carbonyl compounds which are in equilibrium with low-molecular, non-condensed N-alkylol compounds are then subjected to a condensation reaction with aminoplast-forming agents, which optionally contain N-alkylol groups, or with phenoplast-forming agents, in an aqueous medium, if appropriate in the presence of a catalyst and if appropriate in the presence of chain stoppers, and optionally in the presence of additives, and, if appropriate, the modified biomasses thus formed are then freed from undesired substances still contained therein and/or subjected to an after-treatment.

2. Process according to Claim 1, characterised in that the reaction is carried out at temperatures between 0 °C and 200 °C.

3. Process according to Claim 2, characterised in that the reaction is carried out at room temperature.

4. Process according to Claim 1, characterised in that the reaction is carried out under reduced or increased pressure.

5. Process according to Claim 1, characterised in that formaldehyde, crotonaldehyde or isobutyráldehyde are employed as the carbonyl compound.

6. Process according to Claim 1, characterised in that urea, monomethylolurea, dimethylolurea, crude azulmic acids, modified azulmic acids and/or stabilised azulmic acids are employed as aminoplast-forming agents.

7. Process according to Claim 1, characterised in that sulphuric acid or phosphoric acid are employed as catalysts.

8. Modified biomasses, characterised in that they contain cell contents of biomasses, and other reactivé substances occurring in biomasses, condensed with carbonyl compounds, thiocarbonyl compounds and/or carbonyl compounds which are in dissociation equilibrium with low-molecular, non-condensed N-alkylol compounds, and with aminoplast-forming agents, which optionally contain N-alkylol groups, or with phenoplast-forming agents, and characterised in that they optionally contain additives.

9. Use of modified biomasses according to Claim 8 as reactive fillers and/or as flameproofing agents in plastics, as catalysts and/or as supports for catalysts.

**Revendications**

1. Procédé de traitement de biomasses de nature très diverse par réaction des biomasses en milieu aqueux avec des composés carbonylés, des composés thiocarbonylés et/ou des composés carbonylés qui sont en équilibre de dissociation avec des composés N-alcoylolés non condensés à poids moléculaire inférieur, éventuellement en présence d'un catalyseur de même qu'éventuellement en présence de substances additionnelles, caractérisé en ce qu'ensuite les composés carbonylés, les composés thiocarbonylés et/ou les composés carbonylés qui sont en équilibre avec des composés N-alcoylolés non condensés à poids moléculaire inférieur et qui n'ont pas réagi sont condensés en milieu aqueux avec des formateurs d'aminoplastes qui éventuellement contiennent des groupes N-alcoylol ou avec des formateurs de phénoplastes, éventuellement en présence d'un catalyseur et éventuellement en présence d'agents d'arrêt de chaîne, de même qu'éventuellement en présence de substances additionnelles, et en ce qu'ensuite les biomasses modifiées ainsi obtenues sont débarrassées le cas échéant des substances indésirables qui y sont encore contenues et/ou sont soumises à un post-traitement.

2. Procédé selon la revendication 1, caractérisé en ce qu'on exécute la réaction à des températures entre 0 °C et 200 °C.

3. Procédé selon la revendication 2, caractérisé en ce qu'on exécute la réaction à la température ordinaire.

4. Procédé selon la revendication 1, caractérisé en ce qu'on exécute la réaction sous pression réduite ou plus élevée.

5. Procédé selon la revendication 1, caractérisé en ce qu'on utilise comme composé carbonylé de la formaldéhyde, de la crotonaldéhyde ou de l'isobutyraldéhyde.

6. Procédé selon la revendication 1, caractérisé en ce qu'on utilise comme formateurs d'aminoplastes de l'urée, de la monométhylol-urée, de la diméthylol-urée, des acides azulminiques bruts, des acides azulminiques modifiés et/ou des acides azulminiques stabilisés.

7. Procédé selon la revendication 1, caractérisé en ce qu'on utilise de l'acide sulfurique ou de l'acide phosphorique comme catalyseurs.

8. Biomasses modifiées, caractérisées par une teneur en biomasses-substances du contenu cellulaires, ou en d'autres substances réactives existant dans les biomasses, qui sont condensées :

— avec des composés carbonylés, des composés thiocarbonylés et/ou des composés carbonylés qui sont en équilibre de dissociation avec des composés N-alcoylolés non condensés à poids moléculaire inférieur,

— et avec des formateurs d'aminoplastes qui contiennent éventuellement des groupes N-alcoylol ou avec des formateurs de phénoplastes, de même éventuellement que par une teneur en substances additionnelles.

9. Utilisation de biomasses modifiées selon la revendication 8 comme matières de charge réactives et/ou comme agents ignifugeants dans des matières plastiques, comme catalyseurs et/ou comme matériaux de support pour catalyseurs.